(19) Europäisches Patentamt
European Patent Office
Office européen des brevets

(11) EP 1 106 615 B1

(12) EUROPEAN PATENT SPECIFICATION

(45) Date of publication and mention of the grant of the patent:
**05.03.2003 Bulletin 2003/10**

(51) Int Cl.[7]: **C07D 417/14**

(21) Application number: **00310792.7**

(22) Date of filing: **05.12.2000**

# (54) Substituted 1,4-dihydropyridine compounds as bradykinin antagonists

Substituierte 1,4-Dihydropyridinverbindungen zur Verwendung als Bradykininantagonisten

Composés de 1,4-dihydropyridines substitués ayant une fonction d'antagonistes bradykinines

(84) Designated Contracting States:
**AT BE CH CY DE DK ES FI FR GB GR IE IT LI LU NL PT SE TR**

(30) Priority: **10.12.1999 US 170033 P**

(43) Date of publication of application:
**13.06.2001 Bulletin 2001/24**

(73) Proprietor: **PFIZER INC.**
**New York, N.Y. 10017 (US)**

(72) Inventors:
- **Okumura, Yoshiyuki, c/o Pfizer Global Res. & Dvl. Chita-gun, Aichi-ken 470-2393 (JP)**
- **Kawamura, Mitsuhiro, c/o Pfizer Global Res. & Dvl. Chita-gun, Aichi-ken 470-2393 (JP)**
- **Kawai, Makoto, c/o Pfizer Global Res. & Dvl. Chita-gun, Aichi-ken 470-2393 (JP)**
- **Murase, Noriaki, c/o Pfizer Global Res. & Dvl. Chita-gun, Aichi-ken 470-2393 (JP)**
- **Ikeda, Takafumi, c/o Pfizer Global Res. & Dvl. Chita-gun, Aichi-ken 470-2393 (JP)**

(74) Representative: **Hayles, James Richard et al**
**Pfizer Limited,**
**Patents Department,**
**Ramsgate Road**
**Sandwich Kent CT13 9NJ (GB)**

(56) References cited:
**US-A- 5 859 011**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 106 615 B1

## Description

### Technical Field

This invention relates to novel 1,4-dihydropyridine compounds. These compounds are useful as antagonists of bradykinin, and are thus useful in the treatment of inflammation, asthma, allergic rhinitis, pain or the like in mammalian, especially humans. The present invention also relates to a pharmaceutical composition comprising the above compounds.

### Background Art

Bradykinin ("BK") is generated under normal conditions in mammalia by the action of various plasma enzymes such as kallikrein on high molecular weight kininogens. It is widely distributed in mammals, as are its two receptor subtypes, $B_1$ and $B_2$. The actions of BK at the $B_2$ receptor include mainly contraction of arterial and venous preparations, although it can cause relaxation of peripheral resistance vessels as well.

Many of the more important functions of BK, such as increases in vascular permeability, pain, and vasodilatation, however, are mediated by the $B_2$ receptor. These effects at the $B_2$ receptor are believed to be responsible for BK's role in numerous diseases, such as inflammation, cardiovascular disease, pain, and the common cold. Hence antagonists at the $B_2$ receptor should find considerable therapeutic applications. Most of the efforts in this area thus far have been directed at peptidic analogues of the BK structure, some of which have been studied as analgesics and antiinflammatory agents.

International Publication Number WO 96/06082 discloses a variety of 1,4-dihydropyridine compounds having a piperazinylcarbonylmethy group at the 2-position, as antagonists of bradykinin.

It would be desirable if there were provided a non-peptide antagonist of the $B_2$ receptor, having an improved $B_2$ antagonistic activity and a good metabolic stability against human liver microsomes.

### Brief Disclosure of the Invention

The present invention provides a compound of the following formula:

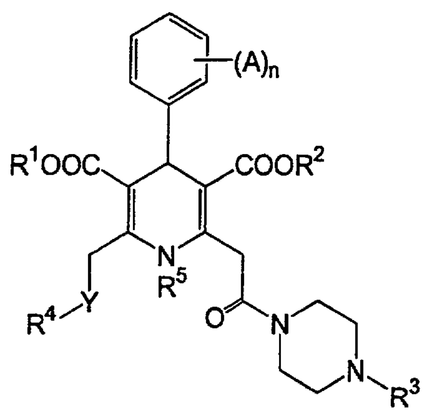

(I)

or the pharmaceutically acceptable salts thereof wherein

**A** is independently halo;

**Y** is $-(CH_2)_m-$, -C(O)- or -S(O)-;

$\mathbf{R^1}$ and $\mathbf{R^2}$ are independently $C_{1-4}$ alkyl;

$\mathbf{R^3}$ is $C_{7-9}$ bicycloalkyl, $C_{5-7}$ azacycloalkyl or $C_{7-9}$ azabicycloalkyl, the $C_{7-9}$ bicycloalkyl, $C_{5-7}$ azacycloalkyl or $C_{7-9}$ azabicycloalkyl being optionally substituted with one, two or three substituents independently selected from oxo, hydroxyl, $C_{1-4}$ alkyl, $C_{1-4}$ alkyloxy, $C_{1-4}$ alkyl-carbonyl, formyl, $C_{1-4}$ alkylenedioxy and phenyl-$C_{1-4}$ alkyl;

$\mathbf{R^4}$ is thiazolyl, imidazolyl or oxazolyl, the thiazolyl, imidazolyl or oxazolyl being optionally substituted with one or two substituents independently selected from $C_{1-4}$ alkyl and halo;

$\mathbf{R^5}$ is hydrogen or $C_{1-4}$ alkyl;

**m** is 0, 1 or 2; and

**n** is 0,1, 2, 3, 4 or 5.

The 1,4-dihydropyridine compounds of this invention have an antagonistic action towards bradykinin and are thus useful in therapeutics, particularly for the treatment of inflammation, rheumatoid arthritis, cystitis, post-traumatic and post ischemic cerebral edema, liver cirrhosis, Alzheimer's disease, cardiovascular disease, pain, common cold, allergies, asthma, pancreatitis, burns, virus infection, head injury, multiple trauma, rhinitis, hepatorenal failure, diabetes, metastasis, pancreatitis, neovascularization, corneal haze, glaucoma, ocular pain, ocular hypertension or the like in mammalian, especially humans.

The 1,4-dihydropyridine compounds of this invention have an antagonistic action towards bradykinin and are thus useful in therapeutics, particularly for the treatment of Amyotrophic lateral sclerosis, Huntington's disease, Parkinson's disease, multiple sclerosis, stroke, head trauma, post-surgical brain edema, brain edema (general), cytotoxic brain edema (such as that associated with brain tumors, stroke, head trauma,etc.), brain edema associated with metabolic diseases (renal failure, pediatric metabolic diseases, etc.), rheumatoid arthritis, osteoarthritis, migraine, neuropathic pain, pruritis, brain tumor, pseudotumor cerebri, glaucoma, hydrocephalus, spinal cord trauma, spinal cord edema, neurodegenerative diseases, respiratory diseases, diuresis, natriuresis calciuresis, COPD (chronic obstructive pulmonary disease), post-traumatic brain injury, itching, sepsis or the like in mammalian, especially humans.

The present invention provides a pharmaceutical composition for the treatment of disease conditions mediated by bradykinin, in a mammalian subject, which comprises administering to said subject a therapeutically effective amount of a compound of formula (I).

Further, the present invention also provides a pharmaceutical composition for the treatment of inflammation, rheumatoid arthritis, cystitis, post-traumatic and post ischemic cerebral edema, liver cirrhosis, Alzheimer's disease, cardiovascular disease, pain, common cold, allergies, asthma, pancreatitis, burns, virus infection, head injury, multiple trauma, rhinitis, hepatorenal failure, diabetes, metastasis, pancreatitis, neovascularization, comeal haze, glaucoma, ocular pain, ocular hypertension or the like, which comprises a therapeutically effective amount of the 1,4-dihydropyridine compound of formula (I) or its pharmaceutically acceptable salt together with a pharmaceutically acceptable carrier.

Further, the present invention also provides a pharmaceutical composition for the treatment of Amyotrophic lateral sclerosis, Huntington's disease, Parkinson's disease, Multiple sclerosis, Stroke, head trauma, Post-surgical brain edema, Brain edema (general), Cytotoxic brain edema (such as that associated with brain tumors, stroke, head trauma, etc.), Brain edema associated with metabolic diseases (renal failure, pediatric metabolic diseases, etc.), Rheumatoid arthritis, Osteoarthritis, Migraine, Neuropathic Pain, Pruritis, Brain Tumor, Pseudotumor cerebri, Glaucoma, Hydrocephalus, Spinal cord trauma, Spinal cord edema, neurodegenerative diseases, respiratory diseases, diuresis, natriuresis calciuresis, COPD (chronic obstructive pulmonary disease), post-traumatic brain injury, itching or Sepsis, which comprises a therapeutically effective amount of a compound of formual (I) or its pharmaceutically acceptable carrier.

Also, the present invention provides a method for the treatment of disease conditions mediated by bradykinin, in a mammalian subject, which comprises administering to said subject a therapeutically effective amount of a compound of formula (I).

Further, the present invention provides a method for the treatment of inflammation, rheumatoid arthritis, cystitis, post-traumatic and post ischemic cerebral edema, liver cirrhosis, Alzheimer's disease, cardiovascular disease, pain, common cold, allergies, asthma, pancreatitis, burns, virus infection, head injury, multiple trauma, rhinitis, hepatorenal failure, diabetes, metastasis, pancreatitis, neovascularization, corneal haze, glaucoma, ocular pain, ocular hypertension or the like, in a mammalian subject, which comprises administering to said subject a therapeutically effective amount of a compound of formula (I).

Further, the present invention provides a pharmaceutical formulation comprising a compound of formula (I), a pharmaceutically acceptable carrier and, optionally, one or more other pharmacologically active ingredients.

## Detailed Description of the Invention

As used herein, the term "**halo**" is fluoro, chloro, bromo or iodo.

As used herein, the term "**alkyl**" means straight or branched chain saturated radicals, including, but not limited to methyl, ethyl, *n*-propyl, *iso*propyl, *n*-butyl, *iso*butyl, *secondary*-butyl, *tertiary*-butyl.

As used herein, an example of "$C_{7-9}$ **bicycloalkyl**" means bicyclo[3.2.1]octyl, octahydropentalenyl, bicyclo [2.2.1]heptyl, and the like.

As used herein, an example of "**propyl**" is n-propyl and isopropyl.

As used herein, an example of "**butyl**" is n-butyl, isobutyl, sec-butyl and tert-butyl.

As used herein, an example of "**$C_{7-9}$ bicycloalkyl**" means bicyclo[3.2.1]octyl, octahydropentalenyl, bicyclo [2.2.1]heptyl, and the like.

As used herein, the term "**$C_{5-7}$azacycloalkyl or $C_{7-9}$azabicycloalkyl**" means one or two carbons of mono-

or bicyclic alkyl ring components are substituted by nitrogen atoms, included, but not limited to, piperazinyl, piperidino, piperidinyl, pyrrolidinyl, azabicyclo[3.3.0]octyl, quinuclidinyl, azabicyclo[3.2.1]octyl, azabicyclo[3.3.1]nonyl, azabicyclo [2.2.2]octyl, and the like.

Preferred compounds of this invention are those of the formula (I) wherein
**$(A)_n$** is 2,6-dichloro;
**Y** is $-CH_2-$
**$R^4$** is 1,3-thiazol-2-yl, 1*H*-imidazol-2-yl, 1-methyl-1*H*-imidazol-2-yl, 1-ethyl-*1H* -imidazol-2-yl or 1,3-oxazol-2-yl; and
**$R^5$** is hydrogen.

Much preferred compounds of this invention are those of the formula (I) wherein
**$R^1$** and **$R^2$** are independently methyl or ethyl;
**$R^3$** is bicyclo[3.2.1]octyl, bicyclo[2.2.2]octyl, octahydropentalenyl, piperidinyl, 8-azabicyclo[3.2.1]octyl, 1-azabicyclo [2.2.2]octyl or octahydrocyclopenta[c]pyrrolyl, the bicyclo[3.2.1]octyl, bicyclo[2.2.2]octyl, octahydropentalenyl, piperidinyl, 8-azabicyclo[3.2.1]octyl, 1-azabicyclo[2.2.2]octyl or octahydrocyclopenta[*c*]pyrrolyl being optionally substituted with one, two or three substituents independently selected from oxo, hydroxyl, $C_{1-4}$ alkyl, $C_{1-4}$ alkyloxy, $C_{1-4}$ alkylcarbonyl, formyl, $C_{1-4}$ alkylenedioxy and phenyl-$C_{1-4}$ alkyl;
**$R^4$** is 1,3-thiazol-2-yl, 1-methyl-1*H*-imidazol-2-yl or 1,3-oxazol-2-yl.

Also, preferred compounds of this invention are those of the formula (I) wherein
**$R^3$** is 8-methylbicyclo[3.2.1]oct-3-yl, 8,8-ethylenedioxybicyclo[3.2.1]oct-3-yl, 8-oxobicyclo[3.2.1]oct-3-yl, 8-hydroxybicyclo[3.2.1]oct-3-yl, -3-yl, 8-hydroxy-8-methylbicyclo[3.2.1]oct-3-yl, 8-butyl-8-hydroxybicyclo[3.2.1]oct-3-yl, 8-hydroxy-8-isopropylbicyclo[3.2.1]oct-3-yl, 8-methoxybicyclo[3.2.1]oct-3-yl, bicyclo[2.2.2]oct-2-yl, 5-oxooctahydro-2-pentalenyl, 1-methyl-3-piperidinyl, 1-benzyl-3-piperidinyl, 8-methyl-8-azabicyclo[3.2.1]oct-3-yl, 8-ethyl-8-azabicyclo[3.2.1]oct-3-yl, 8-isopropyl-8-azabicyclo[3.2.1]oct-3-yl, 8-acetyl-8-azabicyclo[3.2.1]oct-3-yl, 1-azabicyclo[2.2.2]oct-3-yl, 2-methyloctahydrocyclopenta[*c*]pyrrol-5-yl, 2-acetyloctahydrocyclopenta[c]pyrrol-5-yl, 2-formyloctahydrocyclopenta[c]pyrrol-5-yl or 8-fonnyl-8-azabicyclo[3.2.1]oct-3-yl.

Preferred individual compounds of this invention are:

dimethyl 4-(2,6-dichlorophenyl)-2-[2-[4-(8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-oxobicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8,8-ethylenedioxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-hydroxy-8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-butyl-8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)2-[2-[4-(8-hydroxy-8-isopropylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl4-(2,6-dichlorophenyl)-2-[2-[4-(8-methoxylbicyelo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 2-[2-(4-bicyclo[2.2.2]oct-2-yl-1-piperazinyl)-2-oxoethyl]-4-(2,6-dichlorophenyl)-6-[2-(1,3-thiazol-2-yl) ethyl]-1,4-dihydro-3,5-pyridinedicarboxylate;
dimethyl 4-(2,6-dichlorophenyl)-2-[2-[4-(5-oxooctahydro-2-pentalenyl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(1-methyl-3-piperidinyl)-1-piperazinyl]-2-oxoethyl]-6-[2-(1,3-thiazol-2-yl) ethyl]-1,4-dihydro-3,5-pyridinedicarboxylate;
dimethyl 2-[2-[4-(1-benzyl-3-piperidinyl)-1-piperazinyl]-2-oxoethyl]-4-(2,6-dichlorophenyl)-6-[2-(1,3-thiazol-2-yl) ethyl]-1,4-dihydro-3,5-pyridinedicarboxylate;
dimethyl 4-(2,6-dichlorophenyl)-2-[2-[4-(8-methyl-8-azabicyclo[3.2.1]oct-3-yl)-1-piperazinyl]-2-oxoethyl]-6-[2-(1, 3-thiazol-2-yl)ethyl]-1,4-dihydro-3,5-pyridinedicarboxylate;
3-ethyl-5-methyl 4-(2,6-dichlorophenyl)-2-[2-[4-(8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-methyl-8-azabicyclo[3.2.1]oct-3-yl)-1-piperazinyl]-2-oxoethyl]-6-[2-(1-methyl-1*H*-imidazol-2-yl)ethyl]-1,4-dihydro-3,5-pyridinedicarboxylate;
dimethyl 4-(2,6-dichlorophenyl)-2-[2-[4-(8-methyl-8-azabicyclo[3.2.1]oct-3-yl)-1-piperazinyl]-2-oxoethyl]-6-[2-(1, 3-oxazol-2-yl)ethyl]-1,4-dihydro-3,5-pyridinedicarboxylate;

dimethyl 4-(2,6-dichlorophenyl)-2-[2-[4-(8-ethyl-8-azabicyclo[3.2.1 ]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-isoprppyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-acetyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-formyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 2-(2-[4-[(1-azabicyclo[2.2.2]oct-3-yl]-1-piperazinyl]-2-oxoethyl)4-(2,6-dichlorophenyl)-6-[2-(1,3-thiazol-2-yl)ethyl]-1,4-dihydro-3,5-pyridinedicarboxylate;
dimethyl 2-[2-[4-(2-methyloctahydrocyclopenta[*c*]pyrrol-5-yl)-1-piperazinyl]-2-oxoethyl]-4-(2,6-dichlorophenyl)-6-[2-(1,3-thiazol-2-yl)ethyl]-1,4-dihydro-3,5-pyridinedicarboxylate;
dimethyl 2-[2-[4-(2-acetyloctahydrocyclopenta[*c*]pyrrol-5-yl)-1-piperazinyl]-2-oxoethyl]-4-(2,6-dichlorophenyl)-6-[2-(1,3-thiazol-2-yl)ethyl]-1,4-dihydro-3,5-pyridinedicarboxylate; and
dimethyl 2-[2-[4-(2-formyloctahydrocyclopenta[*c*]pyrrol-5-yl)-1-piperazinyl]-2-oxoethyl]4-(2,6-dichlorophenyl)-6-[2((1,3-thiazol-2-yl)ethyl]-1,4-dihydro-3,5-pyridinedicarboxylate.

Most preferred individual compounds of this invention are:

dimethyl 4-(2,6-dichlorophenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-methoxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
(-)-dimethyl 4-(2,6-dichlorophenyl)-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridine-3,5-dicarboxylate;
dimethyl 4-(2,6-dichlorophenyl)-2-(2-([4-(8-ethyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinedicarboxylate;
dimethyl 4-(2,6-dichlorophenyl)-2-(2-([4-(8-isopropyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinedicarboxylate;
dimethyl 2-[2-[4-(8-acetyl-8-azabicyclo[3.2.1 ]oct-3-yl-1-piperazinyl]2-oxoethyl]-4-(2,6-dichlorophenyl)-6-[2-(1,3-thiazol-2-yl)ethyl]-1,4-dihydro-3,5-pyridinedicarboxylate;
dimethyl 4-(2,6-dichlorophenyl)-2-(2-([4-(8-formyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinedicarboxylate;
(-)-dimethyl 2-[2-[4-[(3*S*)-1-azabicyclo[2,2,2]oct-3-yl]-1-piperazinyl]-2-oxoethyl]-4-(2,6-dichlorophenyl)-6-[2-(1,3-thiazol-2-yl)ethyl]-1,4-dihydro-3,5-pyridinedicarboxylate;
dimethyl 4-(2,6-dichlorophenyl)-2-(2-([4-(2-acetyloctahydrocyclopenta[*c*]pyrrol-5-yl)-1-piperazinyl]-2-oxoethyl)-6-[2-(1,3-thiazol-2-yl)ethyl]-1,4-dihydro-3,5-pyridinedicarboxylate; and
(-)-dimethyl 4-(2,6-dichlorophenyl)-2-[2-[4-(*exo*-8-hydroxy-8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate.

## General Synthesis

The 1,4-dihydropyridine compounds of formula (I) of this invention may be prepared by a variety of synthetic methods known to those skilled in the art. For example, the 1,4-dihydropyridine compounds of formula (I), may be prepared by reaction of compound (II) with compound (III), followed, if desired, by conversion of a compound (III) in which $R^3$ is H into a compound (III) in which $R^3$ is other than H, as indicated in the following Preparation Method A.

Preparation Method A:

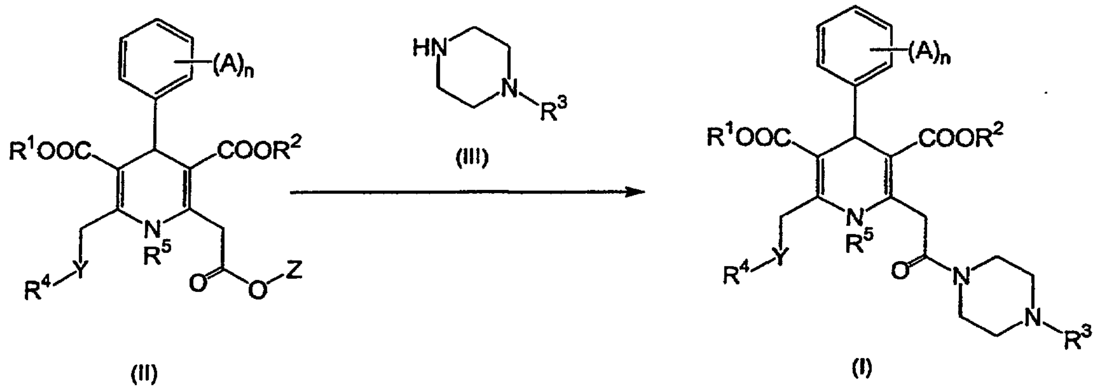

(wherein Z is hydrogen or lower alkyl (e.g., $C_{1-4}$ alkyl) such as methyl and ethyl; and the other symbols are as already defined)

In Preparation Method A, when Z is lower alkyl, the compound (II) may be first subjected to selective saponification of the ester residue at the 2-position of the dihydropyridine ring, followed by acidification to afford a free acid, which is coupled with the compound (III) to give the 1,4-dihydropyridine (I). When Z is H, the compound (II) may be directly coupled with the compound (III) to obtain the 1,4-dihydropyridine (I).

The selective saponification and the acidification may be carried out by conventional procedures. In a typical procedure, the selective saponification is carried out by treatment with sodium hydroxide in a suitable reaction-inert solvent at a temperature in the range from -20 to 40°C, usually from 10°C to 30°C for 3 minutes to 4 hours, usually 15 minutes to 1 hour. In a typical procedure, the acidification is carried out by treatment with diluted hydrochloric acid in a suitable reaction-inert solvent such as water at a temperature in the range from 0 to 30°C, usually from 5°C to 25°C for 1 minute to 1 hour, usually 5 minutes to 15 minutes.

The 1,4-dihydropyridine (I) can be obtained from the corresponding 1,4-dihydropyridine (II) wherein $R^3$ is H by a coupling reaction between the obtained acid and 4-N-substituted piperazine. The condensation may be carried out in a reaction-inert solvent such as aqueous or non-aqueous organic solvents (e.g., tetrahydrofuran, DMF, dioxane, acetone, dimethoxyethane and acetonitrile); halogenated hydrocarbons such as chloroform, dichloromethane and dichloroethane (preferably dichloromethane) using a coupling agent such as dicyclohexylcarbodiimide (DCC), water soluble carbodiimide (WSC), 2-ethoxy-N-ethoxycarbonyl-1,2-dihydroquinoline, benzotriazol-1-yloxy-tris(dimethylamino) phosphonium hexafluorophosphate (BOP), diethyl azodicarboxylate-triphenylphosphine, diethylcyanophosphonate (DEPC), diphenylphosphorylazide (DPPA), bromotripyrrolidino phosphonium hexafluorophosphate (PyBrop [trademark]), bis(2-oxo-3-oxazolidinyl) phosphinic chloride (BOPCl), benzotriazole-1-yl-oxy-tris-pyrrolidino-phosphonium hexafluorophosphate(PyBOP), 2-(1-H-benzotriazole-1-yl)-1,1,3,3,-tetramethyluronium hexafluorophosphate (HBTU) and ethyl chloroformate. This reaction may be carried out at a temperature in the range from -30 to 40 °C, usually from 0 °C to 25 °C for 10 minutes to 96 hours, usually 30 minutes to 24 hours.

In addition, when $R^3$ is substituted-alkyl, the 4-N-substituted piperazines (III) as used herein may be either known or may be prepared by known methods. For example, the 4-N-substituted piperazines may be prepared by means of (1) N-alkylation of 1-N-protected piperazine with appropriate alkyl halide, $R^3$-halo, (2) reductive amination of 1-N-protected piperazine with appropriate aldehyde or ketone in the presence of a reducing agent, followed by deprotection of the 1-N-protecting group, or (3) Michael addition of 1-N-protected piperazine with appropriate conjugated ketones, esters or amides, or (4) piperazine ring construction from N-substituted amine. Suitable 1-N-protecting groups include, for example, benzyl, benzyloxycarbonyl and t-butoxycarbonyl group.

The reductive alkylation may be carried out with appropriate aldehyde or ketone in a suitable reaction-inert solvent such as aqueous or non-aqueous organic solvents (e.g., tetrahydrofuran, dioxane, acetone, dimethoxyethane and acetonitrile); halogenated hydrocarbons such as chloroform, dichloromethane and dichloroethane (preferably dichloromethane), in the presence of a suitable reducing agent such as sodium borohydride, sodium cyanoborohydride or sodium triacetoxy borohydride at a temperature in the range from -20 to 120 °C, usually 0 to 80 °C for 10 minutes to 1 week, usually 30 minutes to 96 hours, optionally in the presence of molecular sieves. Alternatively, alkylation can be made by two step synthesis. A ketone may be treated with an amine in an inert solvent such as toluene or xylene, at a temperature in the range from 80 to 130°C, usually 100 to 120°C for 10 hours to 2 week, usually 1 days to 1 week, preferably 3 to 5 days. The product may be reduced by hydrogenation in the presence of appropriate catalyst such as

palladium on carbon and platinum oxide(IV), usually platinum oxide(IV) in an inert solvent such as ethanol and ethyl acetate, usually ethyl acetate, at a temperature in the range from 10 to 60 °C, usually 20 to 30 °C for 1 hour to 3 days, usually 3 hours to 10 hours.

Typical Micheal addition reaction may be carried out at a temperature in the range from 30 °C to 120 °C, usually from 60 °C to 100 °C for 5 hours to a week, usually 10 hours to 4 days.

Preparation Method B-I:

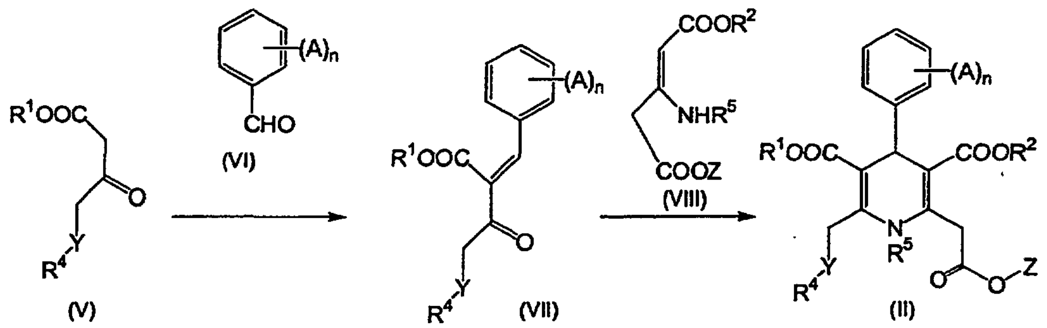

(wherein Z is lower alkyl such as methyl and ethyl; and the other symbols are as already defined)

Scheme B-I

This method utilizes the modified Hantzsch synthesis as described in A. Sausins and G. Duburs, *Heterocycles,* **1988**, *27,* 269. In this method, beta-keto ester (V) is first reacted with substituted benzaldehyde (VI) to obtain compound (VII). This reaction may be carried out in a suitable reaction-inert solvent. Suitable solvents include, for example, aromatic hydrocarbons such as benzene, toluene and xylene; alcohols such as methanol, ethanol, propanol and butanol; ethers such as diethyl ether, dioxane and tetrahydrofuran; halogenated hydrocarbons such as methylene dichloride, chloroform and dichloroethane; amides such as N,N-dimethylformamide; and nitriles such as acetonitrile. This reaction may be carried out at a temperature of 0 °C to 200 °C, preferably from 80 °C to 120 °C for 30 minutes to 24 hours, preferably 30 minutes to 6 hours. If desired, this reaction may be catalyzed by a base such as piperidine, pyridine or alkoxide, or by an acid catalyst such as acetic acid, $TiCl_4$ or p-toluenesulfonic acid.

Thereafter, the benzylidene (VII) as obtained above is reacted with enamine (VIII) in the presence of, or absence of a suitable condensing agent such as Lewis acids, to obtain the 1,4-dihydropyridine (II). This reaction may be carried out in the presence of, or absence of the reaction-inert solvent as listed above. However, this reaction may preferably carried out in the absence of a solvent. This reaction may be carried out at a temperature of 0 °C to 200 °C, preferably, from 60 °C to 150 °C for 30 minutes to 48 hours, preferably 10 hours to 20 hours.

In addition, the beta-keto esters (V) which can be used herein may be prepared by known methods as shown in, for example: (1) *J. Labelled Compds. Radiopharm.,* **1989**, *27,* 599; (2) *J. Org. Chem.,* **1989**, *54*, 3258; (3) *J. Am. Chem. Soc.,***1974**, *96*, 1082; (4) *J. C. S. Perkin I,* **1979**, 529; (5) *Synthesis,* **1986**, 37; (6) *J. C. S. Chem. Commun.,* **1977**, *932,* (7) *Angew. Chem. Int. Ed. Engl.,* **1979**, *18,* 72 and (8) *Tetrahedron Lett.,* **1983**, *24*, 5425. The benzaldehydes (VI) which can be used herein may be either already known or may be prepared according to the reported methods.

Preparation Method B-II:

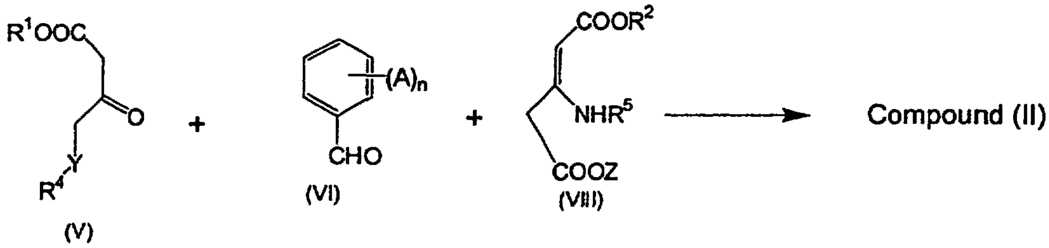


(V) (VI) (VIII)

(wherein all the symbols are as already defined)

Scheme B-II

This method utilizes the three components Hantzsch reaction. In a typical procedure, the beta-keto ester (V), the substituted benzaldehyde (VI) and the enamine (VIII) may be heated together in a suitable reaction-inert solvent as listed above (preferably lower alkanols such as methanol and ethanol). Preferably, a small amount of a lower alkanoic acid such as acetic acid is added as catalyst. The reaction mixture may be heated at 80 °C to 200 °C, preferably from 100 °C to 140 °C for 30 minutes to 1 week, usually 24 hours to 96 hours.

Preparation Method B-III:

Compounds of formula (VIII) may be prepared by a process of this invention according to Scheme B-III.

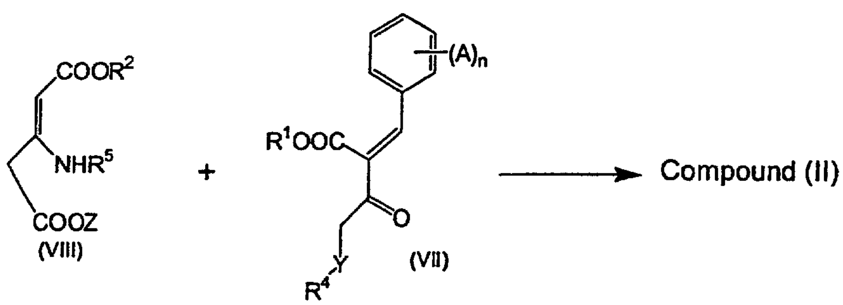


(VIII) (VII)

Scheme B-III

Scheme B-III

Scheme B-III exemplifies a process of this invention for preparing a compound of formula (II) comprising step (a): addition of an enamine compound of formula (VIII) to an alkylene compound of formula (VII) followed by step (b) acid catalyzed cyclization reaction of the resulting compound in step (a).

The former addition step (a) may be carried out under conditions applied to nucleophilic addition reactions using a suitable base in a reaction inert solvent. More preferably, the reaction may be carried out under conditions commonly used in Michael-type addition. Preferred bases for this reaction are those used in Michael-type reactions. Examples of the preferred bases include alkylmagnesium halides known as Grignard reagents and halomagnesium alkoxides. More preferred bases include $(C_1-C_6)$alkylmagnesium bromide and tert-butoxy-magnesium bromide. Preferred solvents used in this reaction include $(C_1-C_4)$alkanol, tetrahydrofuran (THF), diethyl ether, dioxane, hexane, toluene, 1,2-dimethoxy ethane (DME) and the like. This reaction may be carried out at a temperature from about -150°C to reflux, preferably from about -100° to 100°C. In view of convenience, this reaction may be carried out at about room temperature using, for example, halomagnesium$(C_1-C_4)$alkoxides, $(C_1-C_6)$alkylmagnesiumhalides, metalhydrides, metal$(C_1-C_3)$alkoxides, magnesium-di[$(C_1-C_3)$alkoxides], metal-n-butoxide, metal-sec-butoxide, metal-*tert*-butoxide or

a metalcarbonate such as $K_2CO_3$. In case of the base is $K_2CO_3$, the reaction is effectively run in THF. In case of the base is CsF or KF, the reaction is effectively run in THF or methanol (MeOH) at an elevated temperature such as at about 60°C. In case of using butyllithium (BuLi), the reaction is effectively run in THF at from about -78° to about -30°C. In case of using halomagnesium($C_1$-$C_4$)alkoxides or ($C_1$-$C_6$)alkylmagnesiumhalides, a preferred solvent is THF. Suitable reaction time ranges from about 3 minutes to about 2 days, preferably from about 30 minutes to about 40 hours.

The subsequent cyclization process step (b) may be carried out in the presence of a protonic acid. Suitable protonic acids include ($C_1$-$C_6$)alkanoic acid such as acetic acid, hydrochloric acid (HCl) and sulfonic acids such asp-toluenesulfonic acid. It is preferred to add a non-protonic Lewis acid to the reaction mixture in combination with the protonic acid, when the base used in Step (a) is other than magnesium (VIII) bases. This reaction may be carried out at a temperature from about -150°C to reflux, preferably from about -100° to 100°C. The reaction time ranges from about 1 second to 5 days, preferably 5 minutes to 20 houres.

Generally, those reactions illustrated in Scheme B-III may be carried out at about -78°C using dry-ice/acetone or dry-ice/methanol, about 0°C using an ice-bath, room temperature or 100°C, preferably at about 0°C or about room temperature.

The reaction steps (a) and (b) are performed in the same reaction vessel under mild conditions with high-yield.

An enamine compound of formula (VIII) may be prepared according to procedures known to those skilled in the art, such as those illustrated in Scheme B-III-a.

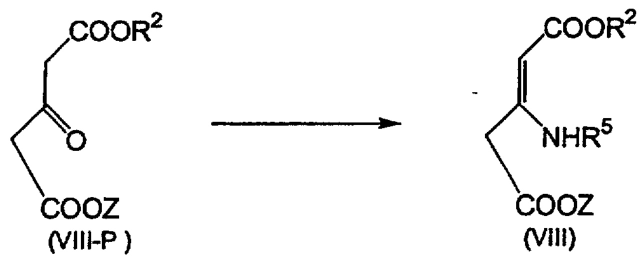

Scheme B-III-a

Typically, a beta-keto ester compound of formula (VIII-P) may be transformed to a compound of formula (VIII) wherein $R^2$, $R^5$ and Z are defined as above. This reaction may be carried out in a reaction inert solvent resolving ammonia gas at a temperature in the range of from about 0° to 60°C. Suitable reaction inert solvents include lower alkanols such as methanol and ethanol. Alternatively, an ammonia gas containing solution given above may be added to a solution containing a beta-keto ester (VIII-P). The mixture is reacted at a temperature in the range of from about 0 to 60 °C to yield the enamine compound (VIII). More conveniently, the compund of formula (VIII) may be synthesized by a reaction of the compound of formula (VIII-P) with ammonium hydrogencarbonate or ammonium acetate in a reaction inert solvent or neat at in a range of ambient temperature to 120 °C, preferablly, at 30 to 80 °C. Suitable reaction inert solvents include lower alkanols, such as methanol and ethanol, DMF, $CH_3CN$ or DMSO, but more easily the reaction may be run without solvent.

An alkylene compound of formula (VII) may be prepared according to procedures known to those skilled in the art. Scheme B-III-b illustrates one embodiment of the preparation process.

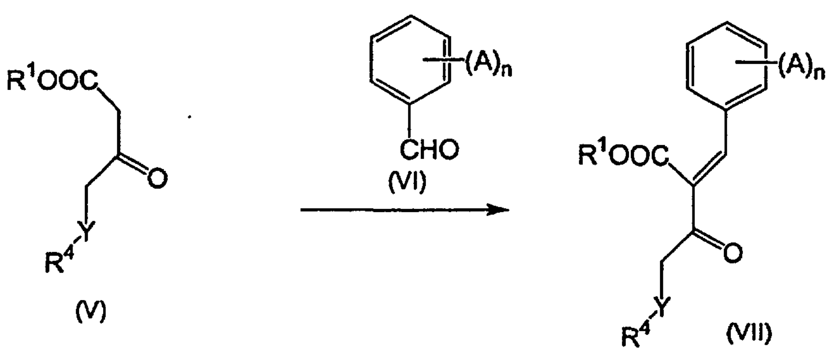

Scheme B-III-b

A carbonyl compound of formula (V) may be subjected to a coupling reaction with an aldehyde compound of formula (VI) to give the alkylene compound of formula (VII) according to a known procedure. For example, a compound of formula (V) may be reacted with a compound of formula (VI) according to a procedure reported by L. Tietze *et al.* Liebigs Ann. Chem., pp. 321-329, 1988. This reaction may be carried out in a suitable reaction inert-solvent for example an aromatic hydrocarbon such as benzene, toluene and xylene, an alcohol such as methanol, ethanol, propanol and butanol, an ether such as diethyl ether, dioxane and tetrahydrofuran (THF), a halogenated hydrocarbon such as methylene dichloride, chloroform and dichloroethane, an amide such as *N,N*-dimethylformamide (DMF), and a nitrile such as acetonitrile. This reaction may be carried out at a temperature in a range of from about 0°C to the reflux temperature of the reaction mixture, preferably from about 80° to the 120°C for from about 30 minutes to 24 hours, preferably from 30 minutes to 6 hours. This reaction may conveniently be carried in the presence of a base or acid catalyst. Suitable base catalysts are such as piperidine, pyridine and alkoxide, and suitable acid catalysts are such as acetic acid, $TiCl_4$ and *p*-toluenesulfonic acid.

An intermediate compound of formula (V) may be prepared starting from a known compound according to a procedure known to those skilled in the art. For example, a compound of formula (V) may be prepared according to the procedure described in Scheme B-III-c.

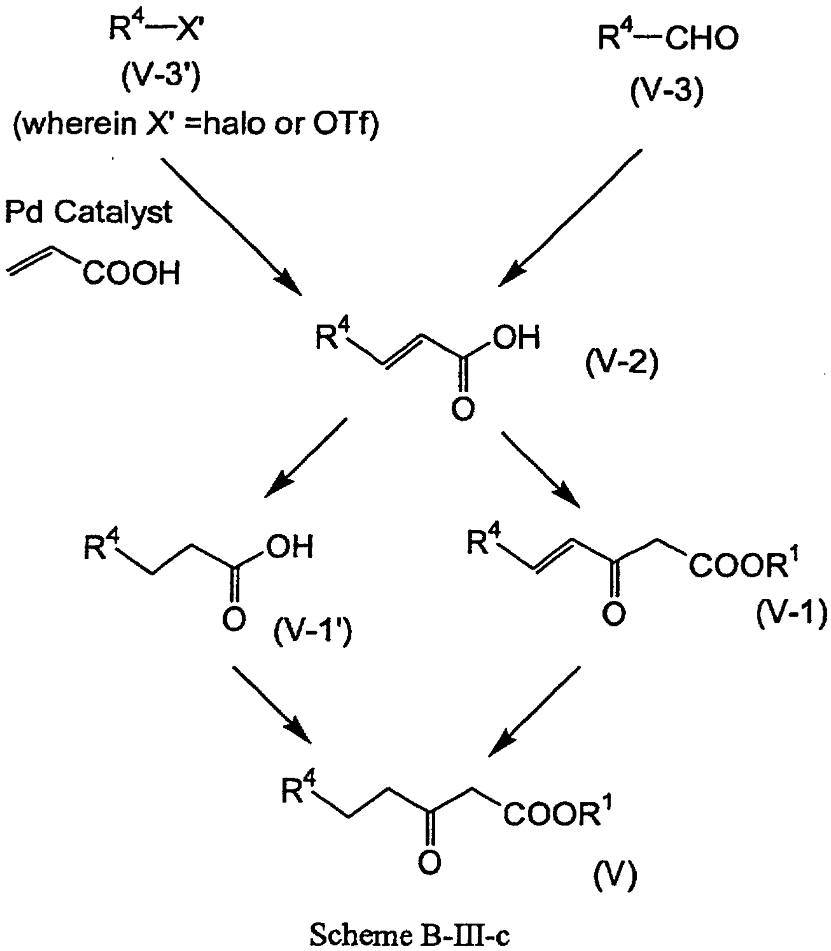


Scheme B-III-c

An aldehyde compound (V-3), wherein $R^4$ is defined as above, is reacted with malonic acid under a basic condition. For example, this reaction may be carried out in the presence of a weak base such as piperidine in a reaction inert solvent such as pyridine to give a carboxylic acid compound of formula (V-2). Alternatively, the compound of formula (V-2) may be synthesized by a so-called "Heck reaction". Thus, $R_4$-X' (X'=Cl, Br, I, trifluoromethanesulfonate (OTf) may be reacted with acrylic acid in the presence of appropriate Pd catalyst in a reaction inert solvent, such as DMF, $H_2O$, dimethylacetamide, N-ethylpiperidine, triethylamine, tributylamine, toluene, xylene, acetonitrile, 1,3-dimethyl-3,4,5,6-tetrahydropyrimidone, 1,3-dimethyl-2-imidazolinone, 1-methyl-2-pyrrolidinone, tetrahydrofuran, dimethoxyethane, t-butylmethylether, dimethylsulfoxide, sulforane, preferably DMF, $H_2O$ and tributylamine. The compound (V-2) thus obtained may be subjected to an aliphatic nucleophilic substitution reaction in the presence of a coupling agent to give a pentenoate compound of formula (V-1). This reaction may conveniently be carried out first by treating the compound of formula (V-1) with a coupling agent such as N,N'-carbonyldiimidazole in a reaction inert solvent such as dimethylformamide, then reacting with a neucleophilic reagent such as $CH_3O_2CCH_2COOK$ in the presence of a Lewis acid such as magnesium chloride. The former treatment may be carried out at a temperature in the range of 0° to 60°C, preferably at about room temperature for from about 1 minutes to 12 hours. The latter reaction may be carried out at the temperature in the range of from about 0° to 100°C, preferably from about room temperature to 60°C for from about 1 minutes to 12hours. The compound of formula (V-1) may be reduced over a metal catalyst under hydrogen atmosphere to give the compound of formula (V) according to a known procedure. Suitable catalysts are such as Raney nickel catalyst and a noble metal catalysts including palladium on carbon and palladium hydroxide. This reaction may be carried out in a reaction inert solvent such as methanol, at about room temperature under hydrogen atmosphere at an appropriate pressure for about 1 minutes to 12 hours. Alternatively, a compund of formula (V) may be synthesized by reduction of a compund of formula (V-2) and following nucleophilic coupling of the resulting a compund of formula (V-1') with $CH_3O_2CCH_2COOK$ as indicated reaction condition above.

A ketone compound of formula (V) and a substituted benzaldehyde compound of formula (VI) may also be prepared according to known procedures (e.g., (1) D. Scherling, *J. Labelled Compds. Radiopharm.,* Vol. 27, pp. 599-, 1989, (2) C. R. Holmquist *et al., J. Org. Chem.,* Vol. 54, pp. 3528-, 1989, (3) S. N. Huckin *et al., J. Am. Chem. Soc.,* Vol. 96, pp. 1082- , 1974, (4) *J. C. S. Perkin I*, pp. 529-, 1979, (5) *Synthesis* pp. 37, 1986, and (6) *J. C. S. Chem. Commun.,* pp. 932-, 1977).

Preparation Method B-IV:

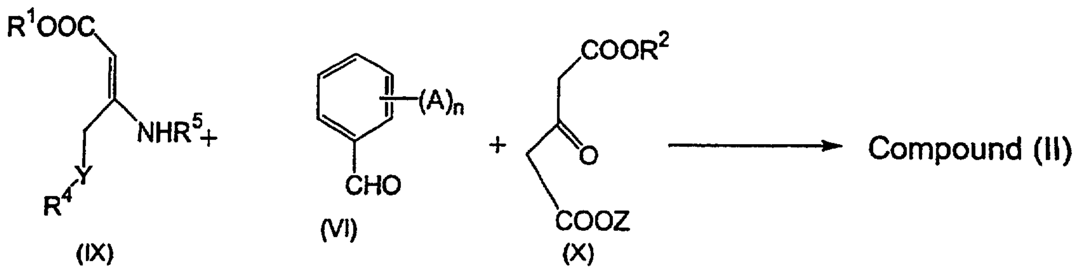

(wherein all the symbols are as already defined)

This method also utilizes the three components Hantzsch reaction as mentioned above. The reaction conditions similar to the above can be also used in this method.

The enamine (IX) may either be known compounds or may be prepared by known methods. For example, the enamine (IX) may be prepared by reacting the beta-keto ester (V) with ammonia or ammonium salt. More specifically, the beta-keto ester (V) may be dissolved in a suitable solvent such as lower alkanols (ex. methanol and ethanol). Excess amount of ammonia gas is introduced into the solution at a temperature of 0 to 60 °C. Alternatively, a solution containing ammonia dissolved in the above solvent is added to the solution containing the beta-keto ester (V), and the resultant mixture is reacted at a temperature of 0 to 60 °C, to obtain the enamine (IX). More conveniently, the compund of formula (VIII) may be synthesized by a reaction of the compound of formula (VIII-P) with ammonium hydrogencarbonate or ammonium acetate in a reaction inert solvent or neat at in a range of ambient temperature to 120 °C, preferablly, at 30 to 80 °C. Suitable reaction inert solvents include lower alkanols, such as methanol and ethanol, DMF, $CH_3CN$ or DMSO, but more easily the reaction may be run without solvent.

The compounds of formula (I), and the intermediates above-mentioned preparation methods can be isolated and purified by conventional procedures, such as recrystallization or chromatographic purification.

General Synthesis of the optical active 1,4-dihydropyridine

The optically active compounds of this invention can be prepared by several methods. For example, the optically active compounds of this invention may be obtained by chromatographic separation or fractional crystallization from the final compounds or the intermediates in racemic form thereof. Alternatively, the optically active compounds may be prepared by optically selective reaction, enzymatic hydrolysis or reactions using optically active intermediates.

For example, the optically active 1,4-dihydropyridine (I-o) may be prepared by reaction of the compound (II-o) with the compound (III), followed, if desired, by conversion of the compound (III) in which $R^3$ is H into the compound (III) in which $R^3$ is other than H, as indicated in the following Preparation Method A-o.

Preparation Method A-o:

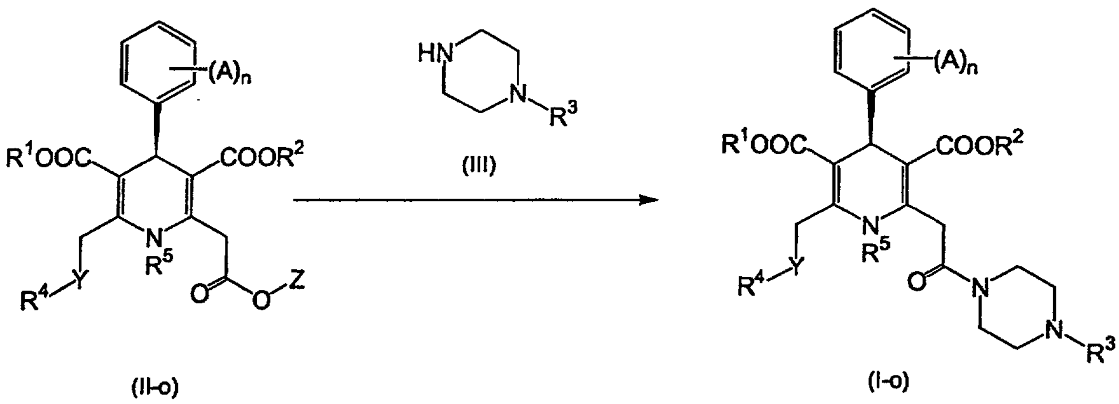

(wherein Z is hydrogen or lower alkyl (e.g., $C_{1-4}$ alkyl) such as methyl and ethyl; and the other symbols are as already defined)

In Preparation Method A-I, when Z is lower alkyl, the compound (II-o) may be first subjected to selective saponification of the ester residue at the 2-position of the dihydropyridine ring, followed by acidification to afford a free acid, which is coupled with the compound (III) to give the 1,4-dihydropyridine (I-o). When Z is H, the compound (II-o) may be directly coupled with the compound (III) to obtain the 1,4-dihydropyridine (I-o).

The selective saponification and the acidification may be carried out by conventional procedures. In a typical procedure, the selective saponification is carried out by treatment with sodium hydroxide in a suitable reaction-inert solvent such as methanol, dioxane and tetrahydrofuran (THF) at a temperature in the range from -20 to 40°C, usually from 10°C to 30°C for 3 minutes to 4 hours, usually 15 minutes to 1 hour. In a typical procedure, the acidification is carried out by treatment with diluted hydrochloric acid in a suitable reaction-inert solvent such as water at a temperature in the range from 0 to 30°C, usually from 5°C to 25°C for 1 minute to 1 hour, usually 5 minutes to 15 minutes.

A compound (I-o) can be obtained from the corresponding compound (II-o) wherein $R^3$ is H by a coupling reaction between the obtained acid and 4-N-substituted piperazine. The condensation may be carried out in a reaction-inert solvent such as aqueous or non-aqueous organic solvents (e.g., tetrahydrofuran, dioxane, acetone, DMF, dimethoxyethane and acetonitrile); halogenated hydrocarbons such as chloroform, dichloromethane and dichloroethane (preferably dichloromethane) using a coupling agent such as dicyclohexylcarbodiimide (DCC), water soluble carbodiimide (WSC), 2-ethoxy-N-ethoxycarbonyl-1,2-dihydroquinoline, benzotriazol-1-yloxy-tris(dimethylamino) phosphonium hexafluorophosphate (BOP), diethyl azodicarboxylate-triphenylphosphine, diethylcyanophosphonate (DEPC), diphenylphosphorylazide (DPPA), bromotripyrrolidino phosphonium hexafluorophosphate (PyBrop[trademark]) and ethyl chloroformate. This reaction may be carried out at a temperature in the range from -30 to 40 °C, usually from 0 °C to 25 °C for 10 minutes to 96 hours, usually 30 minutes to 24 hours.

In addition, when $R^3$ is substituted-alkyl, the 4-N-substituted piperazines (III) as used herein may be either known or may be prepared by known methods. For example, the 4-N-substituted piperazines may be prepared by means of (1) N alkylation of 4-N-protected piperazine with appropriate alkyl halide, $R^3$-halo, (2) reductive amination of 4-N-protected piperazine with appropriate aldehyde or ketone in the presence of a reducing agent, followed by deprotection of the amino-protecting group, or (3) Michael addition of 4-N-protected piperazine with appropriate conjugated ketone, ester or amide, or (4) piperazine ring construction from N-substituted amine. Suitable amino-protecting groups include, for example, benzyl, benzyloxycarbonyl and t-butoxycarbonyl group.

The reductive alkylation may be carried out with appropriate aldehyde or ketone in a suitable reaction-inert solvent such as aqueous or non-aqueous organic solvents (e.g., tetrahydrofuran, dioxane, acetone, dimethoxyethane, acetonitrile, methanol and ethanol); halogenated hydrocarbons such as chloroform, dichloromethane and dichloroethane (preferably dichloromethane), in the presence of a suitable reducing agent such as sodium borohydride, sodium cyanoborohydride or sodium triacetoxyborohydride at a temperature in the range from -20 to 120 °C, usually 0 to 80 °C for 10 minutes to 1 week, usually 30 minutes to 96 hours, optionally in the presence of molecular sieves. Alternatively, alkylation can be made by two step synthesis. A ketone may be treated with an amine in an inert solvent

such as toluene or xylene, at a temperature in the range from 80 to 130°C, usually 100 to 120°C for 10 hours to 2 week, usually 1 days to 1 week, preferably 3 to 5 days. The product may be reduced by hydrogenation in the presence of appropriate catalyst such as Palladium on carbon and platinum oxide (IV), usually platinum oxide in an inert solvent such as ethanol and ethyl acetate, usually ethyl acetate, at a temperature in the range from 10 to 60 °C, usually 20 to 30 °C for 1 hour to 3 days, usually 3 hours to 10 hours.

Typical Micheal addition reaction may be carried out at a temperature in the range from 30 °C to 120 °C, usually from 60 °C to 100 °C for 5 hours to a week, usually 10 hours to 4 days.

The optically active intermediates of formula (II) can be prepared by the following methods.

Preparation Method B-I-o (Fractional Crystallization):

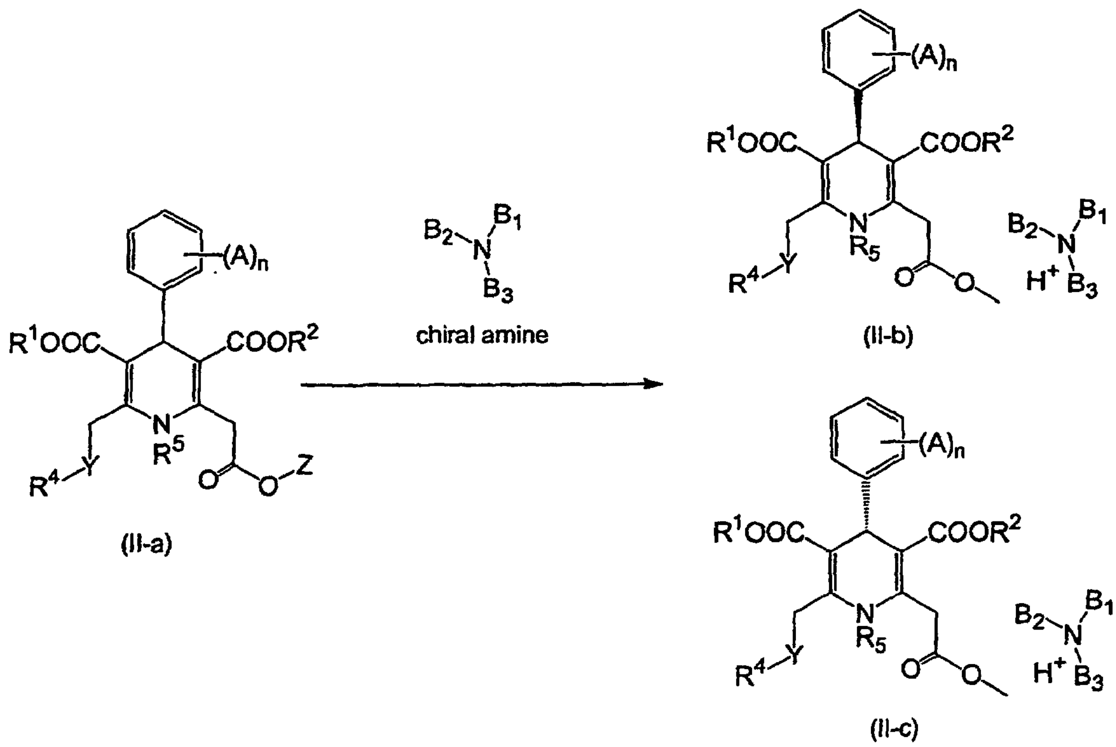


(II-a) (II-b) (II-c)

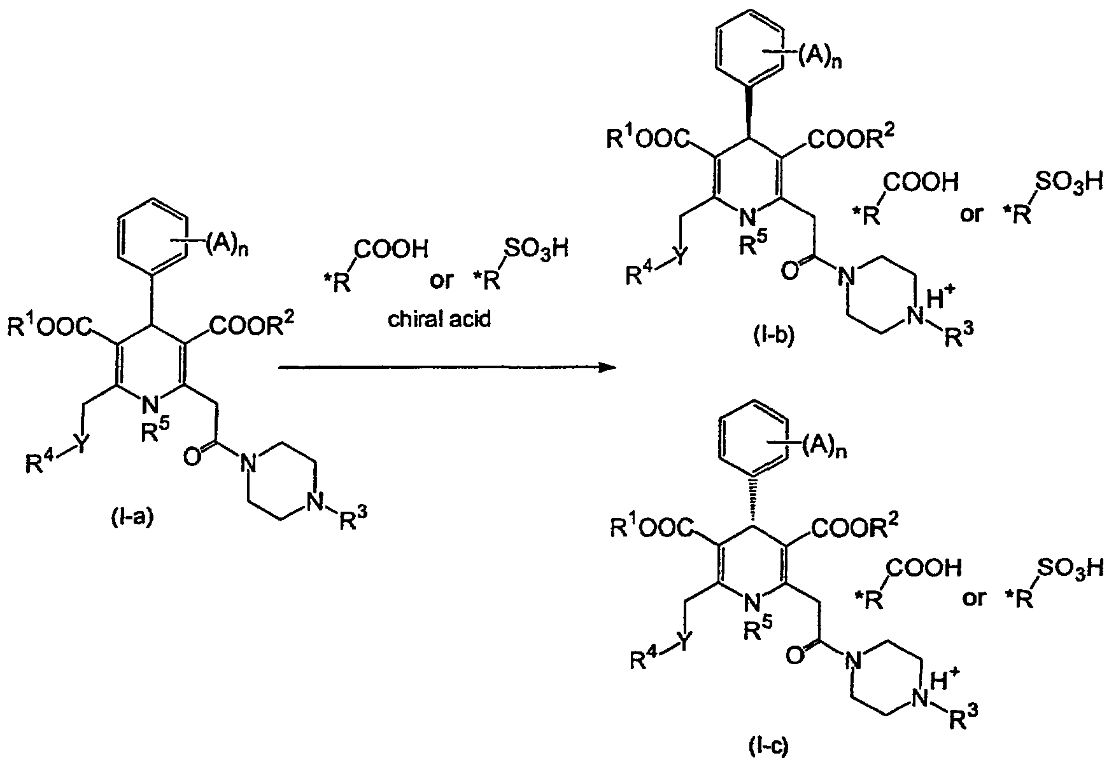


(I-a) (I-b) (I-c)

(wherein [**B**$^1$ **B**$^2$ **B**$^3$]$NH^+$ is a chiral amine residue; Z is hydrogen; R*COOH and $R^*SO_3H$ are chiral acids and the other symbols are already defined.)

In this method, an acid compound (II-a) may be subjected to a fractional crystallization with a chiral amine such as cinchonidine, cinchonine, quinine, burcine and phenethylamine or their derivatives, amino acids to obtain an amine salt (II-b). This reaction may be conducted in an organic solvent, preferably a pure or mixed alcoholic solvent selected from methanol, ethanol, 2-propanol and mixture thereof. The resulted salt may be further purified by several times recrystallization. The pure salt thus obtained may be converted to the corresponding carboxylic acid (an enantiomer of compound (II) wherein Z is H) by a partition between organic solvent such as ethyl acetate or dichloromethane and acid solution such as diluted hydrochloric acid followed by concentration. On the other hand, the salt of the antipode contained in the resulted mother liquid may be converted to the corresponding carboxylic acid (an enantiomer of compound (II) wherein Z is H) by the same procedure described above after concentration of the mother liquid. This acid may be further purified by crystallization in organic or inorganic solvents to give the antipode. This crystallization of the acid may be performed several times , if necessary, to improve its optical purity.

Furthermore, a final compound (I-a) may be resolved into each salt of both enantiomers by the same procedure described above using chiral acid. The resolved salts thus obtained may be converted to the corresponding amines (each enantiomer of I-a) by a partition between organic solvent such as dichloromethane and basic solution such as aqueous sodium hydrogencarbonate or sodium hydroxide.

Preparation Method B-II-o (Enzymatic Hydrolysis):

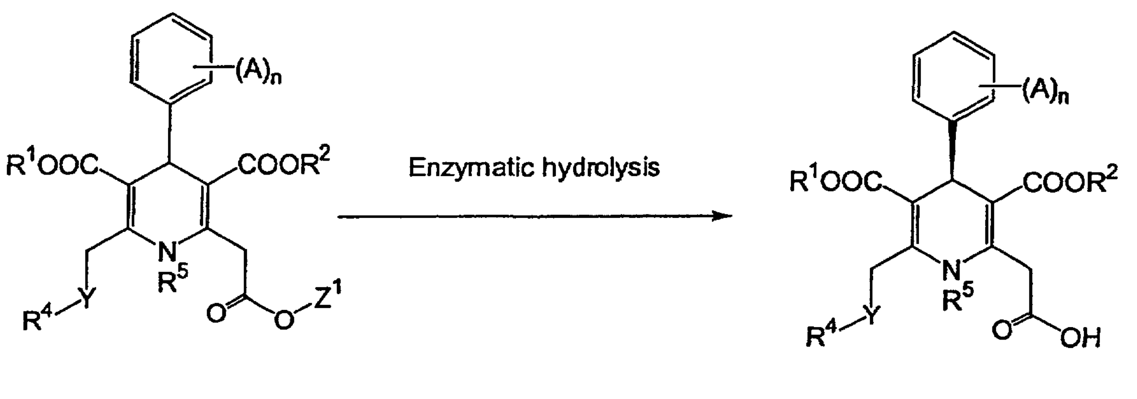


(II-d) (II-e)

(wherein $Z^1$ is, for example, an acyloxymethyl group; and the other symbols are already defined.)

In this method, an ester compound (II-d) is subjected to enzymatic hydrolysis to obtain an optically active carboxylic acid (II-e) (Compound (II) wherein Z is H). Application of lipase in 1,4-dihydropyridine for enantioselective hydrolysis is known in literature such as H. Ebiike, et. al., Tetrahedron Letters, 32, 5805 (1991). Suitable $Z^1$ groups may include acyloxymethyl groups such as pivaloyloxymethyl and propionyloxymethyl. The enzymatic hydrolysis may be carried out in an aqueous organic solvent, preferably a water saturated ethereal solution such as isopropyl ether, t-butyl methyl ether or diethyl ether. This reaction may be carried out at a temperature from 0 °C to 60°C, preferably from 30 °C to 45 °C for 10 minutes to 4 weeks, preferably 1 days to 2 weeks.

Preparation Method B-IV-o (enantioselective Hantzsch cyclization)

The compound (II) may be obtained using enantioselective Hantzsch cyclization. This cyclization may be carried out by a condensation with either enone or enamine attached chiral auxiliaries or by condensation of the enone (VII) and the enamine (VIII) in the presence of chiral catalyst. The main literature (Tetrahedron .Lett,(1988),6437) precedent for this process involves the Enders SAMP/RAMP-methodology (chiral hydrazone tautomer of enamine). Other variants exists in the patent (Bayer's DE 87/3714438 and DE 84/3423105) involving a chiral enamine formed from t-butylvaline.

The 1,4-dihydropyridine compounds of this invention possess an asymmetric center. Hence, the compounds can exist in separated (+)- and (-)-optically active forms, as well as in racemic one thereof. The present invention includes all such forms within its scope. Individual isomers can be obtained by known methods, such as optically selective reaction or chromatographic separation in the preparation of the final product or its intermediate.

The present invention includes salt forms of the compounds (I) as obtained above.

Insofar as the 1,4-dihydropyridine compounds of this invention are basic compounds, they are capable of forming a wide variety of different salts with various inorganic and organic acids.

The acids which are used to prepare the pharmaceutically acceptable acid addition salts of the aforementioned 1,4-dihydropyridine base compounds of this invention of formula (I) are those which form non-toxic acid addition salts, i.e., salts containing pharmaceutically acceptable anions, such as the chloride, bromide, iodide, nitrate, sulfate or bisulfate, phosphate or acid phosphate, acetate, lactate, citrate or acid citrate, tartrate or bi-tartrate, succinate, malate, fumarate, gluconate, saccharate, benzoate, methanesulfonate, ethanesulfonate, benzenesulfonate, p-toluenesulfonate and pamoate (i.e., 1.1'-methylene-bis-(2-hydroxy-3-naphthoate). The acid addition salts can be prepared by conventional procedures.

The 1,4-dihydropyridine compounds of the present invention of formula (I) exhibit significant bradykinin receptor-binding activity and therefore, are of value in the treatment of a wide variety of clinical conditions in mammals, especially human. Such conditions include inflammation, cardiovascular disease, pain, common cold, allergies, asthma, pancreatitis, burns, virus infection, head injury, multiple trauma and the like.

Therefore, these compounds are readily adapted to therapeutic use as bradykinin antagonists for the control and/or treatment of any of the aforesaid clinical conditions in mammals, including humans.

Also, the compounds of formula (I) may be expected more effective therapeutic effects with being co-administered with $H_1$-antagonist.

Further, the present invention also encompasses a pharmaceutical composition for the treatment of inflammation, rheumatoid arthritis, cystitis; post-traumatic and post ischemic cerebral edema, liver cirrhosis, Alzheimer's disease, cardiovascular disease, pain, common cold, allergies, asthma, pancreatitis, bums, virus infection, head injury, multiple trauma, rhinitis, hepatorenal failure, diabetes, metastasis, cystitis, pancreatitis, amyotrophic lateral sclerosis, Huntington's disease, Parkinson's disease, multiple sclerosis, stroke, head trauma, post-surgical brain edema, brain edema (general), cytotoxic brain edema (such as that associated with brain tumors, stroke, head trauma,etc.), brain edema associated with metabolic diseases (renal failure, pediatric metabolic diseases, etc.), rheumatoid arthritis, osteoarthritis, migraine, neuropathic pain, pruritis, brain tumor, pseudotumor cerebri, glaucoma, hydrocephalus, spinal cord trauma, spinal cord edema, neurodegenerative diseases, respiratory diseases, diuresis, natriuresis calciuresis, COPD (chronic obstructive pulmonary disease), post-traumatic brain injury, itching, sepsis, or the like, which comprises a therapeutically effective amount of the 1,4-dihydropyridine compound of formula (I) and H1-antagonist or their pharmaceutically acceptable salt together with a pharmaceutically acceptable carrier.

The compounds of the invention may advantageously be employed in combination with one or more other therapeutic ingredients selected from an antibiotic, anti-fungal, or anti-viral agent, an anti-histamine, a non-steroidal antiinflammatory drug or disease modifying anti-rheumatic drug.

The combination with an anti-histamine ($H_1$ antagonist) is particularly favoured for use in the prophylaxis and treatment of asthma and rhinitis. Examples of anti-histamine are chlorpheniramine, brompheniramine, clemastine, ketotifen, azatadine, loratadine, terfenadine, cetirizine, astemizole, tazifylline, levocabastine, diphenhydramine, temelastine, etolotifen, acrivastine, azelastine, ebastine, mequitazine, KA-398, FK-613, mizolastine, MDL-103896, levocetirizine, mometasone furoate, DF-1111301, KC-11404, carebastine, ramatroban, desloratadine, noberastine, selenotifen, alinastine, E-4716, efletirizine, tritoqualine, norastemizole, ZCR-2060, WY-49051, KAA-276, VUF-K-9015, tagorizine, KC-11425, epinastine, MDL-28163 terfenadine, HSR-609, acrivastine and BMY-25368.

**Method for assessing biological activities:**

The activity of the 1,4-dihydropyridine compounds of the present invention, as bradykinin antagonists, is determined by their ability to inhibit the binding of bradykinin at its receptor sites in recombinant human bradykinin $B_2$ receptor expressing CHO-K1 cells (from Receptor Biology, Inc.) employing radioactive ligands.

The bradykinin antagonist activity of the 1,4-dihydropyridine compounds is evaluated by using the standard assay procedure described in, for example, Baenziger N. L., Jong Y-J. I., Yocum S. A., Dalemar L. R., Wilhelm B., Vaurek R., Stewart J. M., *Eur. J. Cell Biol.*, **1992**, *58*, 71-80. This method essentially involves determining the concentration of the individual compound required to reduce the amount of radiolabelled bradykinin ligands by 50% at their receptor sites in CHO-K1 cells, thereby affording characteristic $IC_{50}$ values for each compound tested.

More specifically, the assay is carried out as follows. First, rat, guinea pig or monkey ileum tissues are minced and suspended in 25mM piperazine-N,N'-bis (2-ethanesulfonic acid (PIPES) buffer (pH 6.8) containing 0.1 mg/ml of soybean trypsin inhibitor. Then, the tissues are homogenized using a Polytron homogenizer at setting 7 for 30 seconds three times, and then rehomogenized with a Teflon-coated homogenizer. The homogenized suspension was centrifuged at 1,200 X g for 15 minutes. The pellet was rehomogenized and then centrifuged at 1,200 X g for 15 minutes. These supernatant were centrifuged at 10,000 X g for 60 minutes. The tissue pellets, CHO-K1 cell membrane are suspended in 25 mM PIPES buffer (pH6.8) containing 1.25 mM dithiothreitol, 1.75 μg/ml bacitracin, 1 mM *o*-phenanthroline, 18.75 μM captopril, 1.25 mg/ml bovine serum albumin (BSA), to prepare tissue/cell suspensions. Then, 10 μl of test compound solution dissolved in phosphate buffered saline (PBS, pH 7.5) containing 2% DMSO (final) and 0.1% BSA (w/v) or 10ml of 12.5 mM bradykinin in PBS (pH 7.5) containing 0.1% BSA (w/v) are placed in a reaction 96-well plate. 15 μl of 8.3 nM [3H]bradykinin is added to the compound solution or bradykinin solution in the 96-well plate. Finally 100 μl of the tissue or cell suspension are added to the mixture in the plate, and incubated at room temperature for 1 hour under the dark. After incubation, the resultant product in the reaction plates is filtered through 0.1% polyethylenimine presoaked LKB filermat. The filtrate is washed using a Skatron auto cell harvester. The tissue bound radioactivity is determined using a LKB betaplate counter. The $IC_{50}$ value is determined using the equation:

$$Bound=B_{max}/(1+[I]/IC_{50})$$

wherein [I] means the concentration of the test compound.

All compounds prepared in the working examples as described below were tested by this method, and showed an $IC_{50}$ value of 1 nM to 30 nM in CHO-K1 cells with respect to inhibition of binding at its receptor.

The bradykinin antagonist activity of the 1,4-dihydropyridine compounds *in vivo* is evaluated by a plasma

leakage test. This test essentially involve determining the concentration of the individual compound required to reduce by 50% the amount of bradykinin-induced plasma leakage in rat urinary bladder, thereby affording characteristic $ED_{50}$ values for each compounds tested.

More specifically, the assay is carried out as follows. 3.5-week old male Sprague-Dawlew rats are purchased from Charles River Japan Inc. The rats are fed on stock diet (CRF from Charles River Japan, Inc.) and maintained under the standard conditions (temperature, 23±1°C and humidity 55±5 %) for at least 3 days. The rats are fasted overnight prior to the experiments. Each test group consists of 5 rats.

Bradykinin, purchased from Peptide Ins., is dissolved in the physiological saline (0.9% sodium chloride) at a concentration of 10 nmol/ml. The test 1,4-dihydropyridine compounds are dissolved or suspended at different concentrations in the physiological saline solution containing 10 mg/ml Evans blue (Wako Pure Chemical, Japan).

Captopril (5 mg/kg of body weight) is intraperitoneally (i.p.) injected to the rats, and 20 minutes later the rats are anesthetized by an administration of Nembutal (Abbott) (2.5 mg/kg of body weight). 5 minutes later, the test compound solution containing Evans blue is intravenously (i.v.) injected to the rats at a dose of 3 ml/kg of body weight. Another 5 minutes later, bradykinin is i.v. injected at a dose of 10 nmol/kg body weight. Thereafter, the rats are killed by dislocation of the neck and the urinary bladders are obtained. The urinary bladders are individually treated with 1 ml of formamide at 60°C for at least 16 hours to extract Evans blue from the tissue. The absorbency of the extract is measured spectrophotometrically at 605 nm to determine the dye concentration. The effect of the individual test compound is calculated as a percentage of the amount of Evans blue leaked into the urinary bladder as compared to the control (saline for the test compounds). Some compounds prepared in the working examples as described below exhibited a remarkable activity at a concentration of 0.2 mg/kg in the inhibition of urinary bladder leakage in this test system.

Human liver microsome assay

$T_{1/2}$ value against human liver microsome was calculated by conventional procedure. More specifically, human liver microsomes (0.2 mg/ml) were mixed with 1 μM of kinin B2 antagonist and incubated with in the presence of 1.3 mM $NADP^+$, 0.9 mM NADH, 3.3 mM glucose-6-phosphate, 3.3 mM $MgCl_2$, and glucose-6-phosphate dehydrogenase (8 units/ml) in a total volume of 1.2 ml of 100 mM potassium phosphate buffer, pH 7.4, at 37 °C. At specified incubation times (0, 5, 10, 30 minutes), an aliquot of 100 μl was withdrawn from the reaction mixture and mixed with 1 ml of acetonitrile containing internal standard. Protein was precipitated by centrifugation at 1,800 x g for 10 minutes, and the resulting supernatant was taken.

Kinin $B_2$ antagonist in samples were analyzed by LS/MS/MS, in a Sciex API-300 mass spectrometer linked with a Hawlett-Pakkered HP1100 HPLC system. A sample of 20 μl was injected to the HPLC system equipped with a Wakosil II 5C18 HG column (2.0 x 150 mm). The mobile phase consisted of 80% acetonitorile including 10 mM ammonium acetate, and the elution was isocratic with a flow rate of 0.3 ml/min. Part of the eluent from the HPLC column was introduced into the atmospheric ionization source via an ion spray interface. $T_{1/2}$ value is determined using the equation:

$$T_{1/2} = 0.693/k$$

wherein k is elimination rate constant of the test compound.

The compounds of the formula (I) exhibit excellent biological activity *in vitro* and *in vivo* as bradykinin antagonists. Additionally, the compound of the formula (I) was more stable against metabolism compared to structurally related 1,4-dihydropiridine disclosed in WO 96/06082 in human liver microsomes assay experiments. Most of the compounds of Working Examples showed $T_{1/2}$ values of more than 10 minutes.

The compound of Example 14 showed a $T_{1/2}$ value of 33 minutes, whereas a structurally similar compound (Dimethyl 4-(2,6-dichlorophenyl)-2-[2-[4-(8-methyl-8-azabicyclo[3.2.1]oct-3-yl]-1-piperazinyl]-2-oxoethyl]-6-[2-(2-thienyl)ethyl]-1,4-dihydro-3,5-pyridinedicarboxylate) showed a $T_{1/2}$ value of two minutes.

The 1,4-dihydropyridine compounds of formula (I) of this invention can be administered via either the oral, parenteral or topical routes to mammals. In general, these compounds are most desirably administered to humans in doses ranging from 0.3 mg to 750 mg per day, preferably from 10 mg to 500 mg per day, although variations will necessarily occur depending upon the weight and condition of the subject being treated, the disease state being treated and the particular route of administration chosen. However, for example, a dosage level that is in the range of from 0.06 mg to 2 mg per kg of body weight per day is most desirably employed for treatment of inflammation.

The compounds of the present invention may be administered alone or in combination with pharmaceutically acceptable carriers or diluents by either of the above routes previously indicated, and such administration can be carried out in single or multiple doses. More particularly, the novel therapeutic agents of the invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various nontoxic organic solvents, etc. Moreover, oralpharmaceutical compositions can be suitably sweetened and/or flavored. In general, the therapeutically-effective compounds of this invention are present in such dosage forms at concentration levels ranging 5% to 70% by weight, preferably 10% to 50% by weight.

For oral administration, tablets containing various excipients such as microcrystalline cellulose, sodium citrate, calcium carbonate, dipotassium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH>8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

## Examples

The invention is illustrated in the following examples in which, unless stated otherwise: all operations were carried out at room or ambient temperature, that is, in the range of 18-25 °C; evaporation of solvent was carried out using a rotary evaporator under reduced pressure with a bath temperature of up to 60 °C; reactions were monitored by thin layer chromatography (tlc) and reaction times are given for illustration only; melting points (m.p.) given are uncorrected (polymorphism may result in different melting points); the structure and purity of all isolated compounds were assured by at least one of the following techniques: tic (Merck silica gel 60 $F_{254}$ precoated TLC plates or Merck $NH_2$ $F_{254s}$ precoated HPTLC plates), mass spectrometry, nuclear magnetic resonance (NMR), infrared red absorption spectra (IR) or microanalysis. Yields are given for illustrative purposes only. Flash column chromatography was carried out using Merck silica gel 60 (230-400 mesh ASTM) or Fuji Silysia Chromatorex® DU3050 (Amino Type, 30~50 µm). Low-resolution mass spectral data (EI) were obtained on a Automass 120 (JEOL) mass spectrometer. Low-resolution mass spectral data (ESI) were obtained on a Quattro II (Micromass) mass spectrometer. NMR data was determined at 270 MHz (JEOL JNM-LA 270 spectrometer) or 300 MHz (JEOL JNM-LA300) using deuterated chloroform (99.8% D) or dimethylsulfoxide (99.9% D) as solvent unless indicated otherwise, relative to tetramethylsilane (TMS) as internal standard in parts per million (ppm); conventional abbreviations used are: s = singlet, d = doublet, t = triplet, q = quartet, m = multiplet, br. = broad, etc. IR spectra were measured by a Shimazu infrared spectrometer (IR-470). Optical rotations were measured using a JASCO DIP-370 Digital Polarimeter (Japan Spectroscopic CO, Ltd.).
Chemical symbols have their usual meanings; b.p. (boiling point), m.p. (melting point), 1(liter(s)), ml (milliliter(s)), g (gram(s)), mg(milligram(s)), mol (moles), mmol (millimoles), eq. (equivalent(s)).

Example 1

**Dimethyl 4-(2,6-dichlorophenyl)-2-[2-[4-(8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

**A. methyl 3-oxo-5-(1,3-thiazole-2-yl)-4-pentenoate**

Methyl 3-oxo-5-(1,3-thiazole-2-yl)-4-pentenoate was prepared from 3-(1,3-thazol-2-yl)-2-propenoic acid *(Bull. Chem. Soc. Jap.* **1974**, *47*, 151.) according to the literature procedure *(Heterocycles* 1994, *38,* 751.). To a stirred solution of 3-(1,3-thiazol-2-yl)-2-propenoic acid (100.0 g, 644.4 mmol) in DMF (1000 ml) was added 1,1'-carbonyldiimidazole (115.0 g, 708.9 mmol) in small portions. After stirring at room temperature for 5 h, to the reaction mixture

were added anhydrous magnesium chloride (73.6 g, 773.0 mmol) and monomethyl malonate potassium salt (120.8 g, 773.0 mmol). The resulting suspension was heated at 55 °C with stirring for 14 h. After cooling to room temperature, the reaction mixture was poured into 1500 ml of 2 *N*HCl, and extracted with a mixture of EtOAc (1500 ml) and toluene (500 ml). The organic phase was separated and the aqueous phase was extracted with a 3 : 1 mixture of EtOAc and toluene (2000 ml). The combined organic phase was washed with $H_2O$ (1000 ml) and brine (1000 ml), dried ($Na_2SO_4$) and evaporated to afford 132.0 g of methyl 3-oxo-5-(1,3-thiazole-2-yl)-4-pentenoate (1/2 of keto/enol form)
$^1$H NMR ($CDCl_3$) δ: 11.77 (s, 2/3 H), 7.97 (d, *J* = 3.1 Hz, 1/3 H), 7.90 (d, *J* = 3.1 Hz, 2/3 H), 7.72 (d, *J* = 16.0 Hz, 1/3 H), 7.55 (d, *J* = 15.6 Hz, 2/3 H), 7.51 (d, *J* = 3.1 Hz, 1/3 H), 7.39 (d, *J* = 3.1 Hz, 2/3 H), 7.06 (d, *J* = 16.0 Hz, 1/3 H), 6.80 (d, *J* = 15.6 Hz, 2/3 H), 5.28 (s, 2/3 H), 3.79 (s, 3 x 2/3 H), 3.77 (s, 3 x 1/3 H), 3.45 (s, 2 x 1/3 H).

**B. Methyl 3-oxo-5-(1,3-thiazole-2-yl)pentanoate**

A mixture of methyl 3-oxo-5-(1,3-thiazole-2-yl)-4-pentenoate (132.0 g) and palladium hydroxide, 20 wt% on carbon (13 g) in MeOH (2600 ml) was stirred under hydrogen atmosphere at atmospheric pressure for 4 h. Catalyst was removed by filtration and the filtrate evaporated to give 130.0 g of methyl 3-oxo-5-(1,3-thiazole-2-yl)pentanoate as a brown liquid.
$^1$H NMR ($CDCl_3$) δ: 7.65 (d, *J* = 3.3 Hz, 1 H), 7.20 (d, *J* = 3.3 Hz, 1 H), 3.73 (s, 3 H), 3.53 (s, 2 H), 3.33 (t, *J* = 6.9 Hz, 2 H), 3.13 (t, *J* = 6.9 Hz, 2 H).

**C. Methyl 3-(2,6-dichlorophenyl)-2-[(1,3-thiazol-2-yl)propanoyl]-2-propenoate**

To a solution of methyl 3-oxo-5-(1,3-thiazole-2-yl)pentanoate (130 g) in toluene (600 ml) were added 2,6-dichlorobenzaldehyde (113.0 g, 644 mmol), acetic acid (5 ml) and piperidine (5 ml). This mixture was distilled for removal of the initial distillate (about 100 ml) then replaced the distillation apparatus to Dean-Stark trap and heated under reflux temperature with azeotropic removal of $H_2O$ for 4 h. The mixture was washed with $H_2O$ (200 ml) and brine (200 ml), dried ($Na_2SO_4$) and evaporated to give a crude mixture. This was purified by column chromatography on silica gel (1800 g, hexane/EtOAc = 3/1 as eluent) to afford 165.3 g (69%, 3 steps) of methyl 3-(2,6-dichlorophenyl)-2-[1,3-thiazol-2yl)propanoyl]-2-propenoate as a brown oil. This is a 1 : 1 mixture of the double bond isomers.
$^1$H NMR ($CDCl_3$) δ: 7.70-7.15 (m, 6 H), 3.91 and 3.66 (apparently two synglets, 3 H), 3.44 and 3.28 (apparently two synglets, 4 H).

**D. Dimethyl 4-(2,6-dichlorophenyl)-2-(2-methoxy-2-oxoethyl)-6-[2-(1,3-thiazol-2-yl)ethyl]-1,4-dihydropyridine-3,5-dicarboxylate**

To a stirred solution of 2-methyl-2-propanol (92.8g, 1252 mmol) in THF (1100 ml) was added a 1.0 M solution of EtMgBr in THF (1192 ml, 1192 mmol) dropwise slowly at 0 °C under nitrogen atmosphere over a 2 h period. The resulting solution was stirred at room temperature for 1 h. Then, to the mixture was added a solution of dimethyl 3-amino-2-pentenedioate (113.5 g, 655 mmol) in THF (550 ml) dropwise slowly at 0 °C for 20 min. The resulting pale yellow solution was stirred at the same temperature for 1 h, then a solution of methyl 3-(2,6-dichlorophenyl)-2-[(1,3-thiazol-2-yl)propanoyl]-2-propenoate (219.9 g, 594 mmol) in THF (550 ml) was added at 0 °C for 30 min. The reaction mixture was stirred at room temperature for 16 h under nitrogen atmosphere, then acetic acid (170 ml) was added at 0 °C. The resulting mixture was stirred at room temperature for 6 h. The mixture was poured into 2 NNaOHaq. (1000 ml), the organic phase was separated and the aqueous phase was extracted with EtOAc (2000 ml). The combined organic phase was washed with $H_2O$ (1000 ml) and brine (1000 ml), dried ($Na_2SO_4$) and concentrated to give a crude mixture. Purification on silica gel column chromatography (3 times 1700 g) eluted with hexane/EtOAc (2/1 to 1/2) afforded 246.0 g (85%) of dimethyl 4-(2,6-dichlorophenyl)-2-(2-methoxy-2-oxoethyl)-6-[2-(1,3-thiazol-2-yl)ethyl]-1,4-dihydropyridine-3,5-dicarboxylate as a brown oil.
$^1$H NMR ($CDCl_3$) δ: 8.33 (s, 1 H), 7.67 (d, *J* = 3.3 Hz, 1 H), 7.24 (t, *J* = 8.0 Hz, 2 H), 7.24 (d, *J* = 3.3 Hz, 1 H), 6.98 (dd, *J* = 8.0, 8.0 Hz, 1 H), 5.99 (s, 1 H), 3.86-3.65 (m, 5 H), 3.51 (s, 3 H), 3.54 (s, 3 H), 3.45-3.25 (m, 3 H), 3.14-2.96 (m, 1 H).

**E. 2-(4-(2,6-Dichlorophenyl)-3,5-bis(methoxycarhonyl)-6-(2-(1,3-thiazol-2-yl)ethyl]-1,4-dihydro-2-pyridinyl] acetic acid**

To a stirred solution of dimethyl 4-(2,6-dichlorophenyl)-2-(2-methoxy-2-oxoethyl)-6-[2-(1,3-thiazol-2-yl)ethyl]-1,4-dihydropyridine-3,5-dicarboxylate (264.0 g, 502.5 mmol) in MeOH (1800 ml) was added 2 *N* NaOHaq. (630 ml, 1260 mmol) dropwise with ice cooling. The reaction mixture was stirred at room temperature for 2 h. The mixture was acidified with 2 *N*HCl (700 ml) with ice cooling. The whole mixture was extracted with $CH_2Cl_2$ (600 ml x 4), and the organic layers were washed with brine (1000 ml), dried ($Na_2SO_4$) and then evaporated to give 267 g of yellow solids.

The solids were recrystallized from 2-propanol to afford 206.5 g (80%) of 2-[4-(2,6-dichlorophenyl)-3,5-bis(methoxycarbonyl)-6-[2-(1,3-thiazol-2-yl)ethyl]-1,4-dihydro-2-pyridinyl]acetic acid as yellow solids.
$^1$H NMR ($CDCl_3$) δ: 8.54 (br.s, 1 H), 7.72 (d, *J* = 3.3 Hz, 1 H), 7.31-7.15 (m, 3 H), 7.01 (dd, *J* = 8.0, 8.0 Hz, 1 H), 6.00 (s, 1 H), 3.76-3.20 (m, 5 H), 3.59 (s, 3 H), 3.56 (s, 3 H), 3.04-2.90 (m, 1 H).

**F. 3-(4-Benzyl-1-piperazinyl)bicyclo[3.2.1]octan-8-one ethyleneketal**

A mixture of 8,8-ethylendioxy-oxobicyclo[3.2.1]octan-3-one (*Tetrahedron,* **1963**, 677 and *Tetrahedron Lett.*, **1984**, *25*, 1311, 8.2 g, 46.6 mmol), benzylpiperazine (8.1 ml, 46.6 mmol) and $Ti(O^iPr)_4$ (18.5 ml, 69.9 mmol) was stirred for 2 h. The mixture was diluted with MeOH (94 ml), and then the solution was cooled to 5 °C. To this solution, $NaBH_4$ (2.6 g, 69.9 mmol) was added portionwise carefully. After the addition, the mixture was stirred for 1 h. 2 *N* NaOHaq. (100 ml) was added and the resulting white suspension was filtered through a pad of celite. $CH_2Cl_2$ (500 ml) was added to the filtrate and the organic layer was separated. The organic layer was washed with $H_2O$ (50 ml) and brine (50 ml) successively, and then was dried ($MgSO_4$), filtered, and concentrated *in vacuo.* The residue was purified by column chromatography (NH gel, 100g, $CH_2Cl_2$) to give 9.8 g of 3-(4-benzyl-1-piperazinyl)bicyclo[3.2.1]octan-8-one ethyleneketal as a white solid (62%).
$^1$H NMR ($CDCl_3$) δ: 7.32-7.20 (m, 5 H), 3.91 (s, 4 H), 3.51 (s, 2 H), 2.60-1.40 (m, 19 H).

**G.1 -Benzyl-4-(8-methylenebicyclo[3.2.1]oct-3-yl)piperazine**

To a solution of 3-(4-benzyl-1-piperazinyl)bicyclo[3.2.1]octan-8-one ethyleneketal (2.0 g, 5.85 mmol) in acetone (20 ml) was added 2 NHCl (10 ml) and the mixture was heated at 90 °C for 8 h. After cooling down, the mixture was basified with sat. $NaHCO_3$aq. and was extracted with $CH_2Cl_2$ (100 ml x 2). The combined extracts were washed with brine (30 ml), dried ($MgSO_4$), filtered, and concentrated *in vacuo*. To a suspension of methyltriphenylphosphonium bromide (4.2 g, 11.7 mmol) in THF (100 ml) was added 1.54 M of n-BuLi solution (7.6 ml, 11.7 mmol) at 0 °C. After the resulting yellow solution was stirred for 1 h, a solution of the crude in THF (50 ml) was added to the yellow solution. The resulting mixture was stirred for 2h at ambient temperature and refluxed for 2 h. After cooling down, the mixture was poured into $H_2O$ (100 ml). The aqueous mixture was extracted with EtOAc (100 ml x 2). The combined extracts were washed with brine (40 ml), dried ($MgSO_4$), filtered, and concentrated *in vacuo*. The residue was purified by column chromatography (NH gel, 100 g, $CH_2Cl_2$) to give 1.0 g of 1-benzyl-4-(8-methylenebicyclo[3.2.1]oct-3-yl)piperazine as a pale yellow oil (58%).
$^1$H NMR ($CDCl_3$) δ: 7.34-7.20 (m, 5 H), 4.60 (s, 2 H), 3.50 (s, 2 H), 2.80-2.42 (m, 9 H), 1.94-1.40 (m, 10 H).

**H. 4-(8-Methylbicyclo[3.2.1]oct-3-yl)piperazine**

To a solution of 1-benzyl-4-(8-methylenebicyclo[3.2.1]oct-3-yl)piperazine (3.76g, 12.6 mmol) in MeOH (80 ml) was added 1.0 g of $Pd(OH)_2$ (20 wt% on carbon) and the mixture was stirred for 3 h under $H_2$ (3 kgf/$m^3$) atmosphere at room temperature. The reaction mixture was filtered through a pad of celite and the filtrate was concentrated *in vacuo* to give 2.28 g (86%) of 4-(8-methylbicyclo[3.2.1]oct-3-yl)piperazine as a white solid.
$^1$H NMR ($CDCl_3$) δ: 2.92-2.84 (m, 4 H), 2.56-2.30 (m, 5 H), 2.00-1.20 (m, 11 H), 1.07 (d, *J* = 6.9 Hz, 2 H), 0.81 (d, *J* = 6.9 Hz, 1 H).

**I. Dimethyl 4-(2,6-dichlorophenyl)-2-[2-[4-(8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

To a solution of 2-[4-(2,6-dichlorophenyl)-3,5-bis(methoxycarbonyl)-6-[2-(1,3-thiazol-2-yl)ethyl]-1,4-dihydro-2-pyridinyl]acetic acid (169 mg, 0.33 mmol) in dry $CH_2Cl_2$ (5 ml) was added N-1-ethyl-3-(3-dimethyl-aminopropyl)carbodiimide hydrochloride (WSC, 63 mg, 0.33 mmol) at 0 °C and the reaction mixture was stirred at the same temperature for 30 min under nitrogen atmosphere. 4-(8-methylbicyclo[3.2.1]oct-3-yl)piperazine (69 mg, 0.33 mmol) was added to the resultant solution above at 0 °C. The mixture was stirred for 16 h at room temperature. The mixture was poured into $H_2O$ (50 ml) and the organic phase was separated. The aqueous phase was extracted with $CH_2Cl_2$ (30ml x 3). The combined organic layers were washed with $H_2O$ (50 ml x 2) and brine (50 ml), dried ($Na_2SO_4$) and concentrated *in vacuo* to give a yellow oil. Chromatography on $NH_2$-propyl silica-gel (20 g) eluted with $CH_2Cl_2$/MeOH (100/0 to 30/1) gave dimethyl 4-(2,6-dichlorophenyl)-2-[2-[4-(8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate as a yellow oil (169 mg, 73%).
$^1$H NMR ($CDCl_3$) δ: 8.38 (br.s, 1 H), 7.72 (d, *J* = 3.5 Hz, 1 H), 7.23 (d, *J* = 8.1 Hz, 2 H), 7.20 (d, *J* = 3.3 Hz, 1 H), 7.00 (t, *J* = 7.6 Hz, 1 H), 6.00 (s, 1 H), 4.12 (d, *J* = 15.0 Hz, 1 H), 3.78 (d, *J* = 15.0 Hz, 1 H), 3.70-3.50 (m, 4 H), 3.55 (s, 3 H), 3.54 (s, 3 H), 3.40-3.26 (m, 3 H), 3.10-2.94 (m, 1 H), 2.62-2.38 (m, 5 H), 2.00-1.15 (m, 11 H), 1.05 (d, *J* = 6.9 Hz,

2 H), 0.81 (d, *J* = 6.9 Hz, 1 H).

monohydrochloride form

m.p. 200-205 °C (dec.)
IR (KBr) ν: 3213, 3093, 2949, 2876, 1693, 1624, 1508, 1433, 1296, 1186, 1103 $cm^{-1}$.

**Example 2**

**Dimethyl 4-(2,6-dichlorophenyl)-2-[2-oxo-2-[4-[(8,8-ethylenedioxy)bicyclo[3.2.1]oct-3-yl]piperazinyl]ethyl]-6-[2-(1,3-thiazol-2- yl)ethyl-1,4-dihydro-3,5-pyridinedicarboxylate**

**A. 3-(4-Benzyl-1-piperazinyl)bicyclo[3.2.1]octan-8-one ethyleneketal**

A mixture of 8,8-ethylendioxy-oxobicyclo[3.2.1]octan-3-one (*Tetrahedron,* **1963**, 677 and *Tetrahedron Lett.,* **1934,** *25,* 1311, 8.2 g, 46.6 mmol), benzylpiperazine (8.1 ml, 46.6 mmol) and $Ti(O^iPr)_4$ (18.5 ml, 69.9 mmol) was stirred for 2 h. The mixture was diluted with MeOH (94 ml) and then the solution was cooled to 5 °C. To this solution, $NaBH_4$ (2.6 g, 69.9 mmol) was added portionwise carefully. After the addition, the mixture was stirred for 1 h. 2 *N* NaOHaq. (100 ml) was added and the resulting white suspension was filtered through a pad of celite. $CH_2Cl_2$ (500 ml) was added to the filtrate and the organic layer was separated. The organic layer was washed with $H_2O$ (50 ml) and brine (50 ml) successively, and then was dried ($MgSO_4$), filtered, and concentrated *in vacuo*. The residue was purified by column chromatography (NH gel, 100g, $CH_2Cl_2$) to give 9.8 g of 3-(4-benzyl-1-piperazinyl)bicyclo[3.2.1]octan-8-one ethyleneketal as a white solid (62%).
$^1$H NMR ($CDCl_3$) δ: 7.32-7.20 (m, 5 H), 3.91 (s, 4 H), 3.51 (s, 2 H), 2.60-1.40 (m, 19 H).

**B. 3-(1-Piperazinyl)bicyclo[3.2.1]octan-8-one ethyleneketal**

3-(4-Benzyl-1-piperazinyl)bicyclo[3.2.1]octan-8-one ethyleneketal was deprotected by a procedure similar to that described in example 1,H to afford 3-(1-piperazinyl)bicyclo[3.2.1]ocean-8-one ethyleneketal.
$^1$H NMR ($CDCl_3$) δ: 3.92 (s, 4 H), 2.95-2.80 (m, 4 H), 2.62-2.44 (m, 5 H), 2.05-1.75 (m, 6 H), 1.70-1.60 (m, 2 H), 1.50-1.40 (m, 2 H).

**C. Dimethyl 4-(2,6-dichlorophenyl)-2-[2-oxo-2-[4-[(8,8-ethylenedioxy) bicyclo[3.2.1]oct-3-yl]piperazinyl]ethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 3-(1-piperazinyl)bicyclo[3.2.1]octan-8-one ethyleneketal according to the procedure described in example 1, I.
$^1$H NMR ($CDCl_3$) δ: 8.36 (br.s, 1 H), 7.72 (d, *J* = 3.3 Hz, 1 H), 7.23 (d, *J* = 8.2 Hz, 2 H), 7.20 (d, *J* = 3.3 Hz, 1 H), 7.00 (t, *J* = 8.2 Hz, 1 H), 6.00 (s, 1 H), 4.12 (d, *J* = 15.0, 1 H), 3.92 (s, 4 H), 3.79 (d, *J* = 15.0 Hz, 1 H), 3.68-3.58 (m, 4 H), 3.55 (s, 3 H), 3.54 (s, 3 H), 3.40-3.26 (m, 3 H), 3.07-2.95 (m, 1 H), 2.64-2.44 (m, 5 H), 1.96-1.76 (m, 6 H), 1.69-1.56 (m, 2 H), 1.49-1.40 (m, 2 H).
MS (ESI) 745.21 $(M+H)^+$, 743.26 $(M-H)^-$

**Example 3**

**Dimethyl 4-(2,6-dichlorophenyl)-2-[2-[4-(8-oxobicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 4-(8-oxobicyclo[3.2.1]oct-3-yl)-1-piperazine according to the procedure described in example 1, I.

monohydrochloride form

$^1$H NMR ($CDCl_3$) δ 8.31 (br.s, 1 H), 7.72 (d, *J* = 3.5 Hz, 1 H), 7.23 (d, *J* = 8.1 Hz, 2 H), 7.20 (d, *J* = 3.5 Hz, 1 H), 7.00 (t, *J* = 7.6 Hz, 1 H), 5.99 (s ,1 H), 4.14 (d, *J* = 15.0 Hz, 1 H), 3.75 (d, *J* = 14.8 Hz, 1 H), 3.66-3.58 (m, 4 H), 3.55 (s, 3 H), 3.54 (s, 3 H), 3.40-3.24 (m, 3 H), 3.12-2.94 (m, 2 H), 2.61-2.43 (m, 4 H), 2.30-2.20 (m, 2 H), 2.12-1.72

(m, 8 H).
IR (KBr) ν: 3225, 3103, 2947, 2862, 1746, 1695, 1628, 1506, 1425, 1294, 1188, 1103 $cm^{-1}$.
MS (ESI) 701.13 $(M+H)^+$, 699.16 $(M-H)^-$

**Example 4**

**Dimethyl 4-(2,6-dichlorophenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

To a solution of dimethyl 4-(2,6-dichlorophenyl)-2-[2-[4-(8-oxobicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate (228 mg, 0.33 mmol) in MeOH (5.0 ml) was added $NaBH_4$ (24 mg, 0.66 mmol) at ice cooling temperature. After the reaction was complete, the excess $NaBH_4$ was quenched with acetone. Then, the mixture was extracted with $CH_2Cl_2$ (50 ml x 2). The combined extracts were washed with brine (10 ml), dried ($MgSO_4$), filtered, and concentrated *in vacuo.* The residue was purified by column chromatography (NH gel, 20 g, $CH_2Cl_2$/MeOH = 100/0 to 100/5) to give 100 mg of dimethyl 4-(2,6-dichlorophenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate a pale yellow amorphous (44%).
$^1$H NMR ($CDCl_3$) δ 8.36 (br.s, 1 H), 7.71 (d,*J* = 3.3 Hz, 1 H), 7.23 (d, *J* = 7.9 Hz, 2 H), 7.20 (d, *J* = 3.5 Hz, 1 H), 6.99 (t, *J* = 8.2 Hz, 1 H), 6.00 (s, 1 H), 4.14 (d, *J* = 15.1 Hz, 1 H), 3.91 (t, *J* = 4.8 Hz, 1 H), 3.81 (d, *J* = 15.0 Hz, 1 H), 3.70-3.55 (m, 4 H), 3.55 (s, 3 H), 3.54 (s, 3 H), 3.40-3.25 (m, 3 H), 3.10-2.94 (m, 1 H), 2.66-2.42 (m, 5 H), 2.16-1.40 (m, 11 H).
IR (KBr) ν: 3627, 2945, 1695, 1627, 1506, 1434, 1294, 1186,1103 $cm^{-1}$.

**Example 5**

**Dimethyl 4-(2,6-dichlorophenyl)-2-[2-[4-(8-hydroxy-8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

**A.3-(4-Benzyl-1-piperazinyl)-8-methylbicyclo[3.2.1]octan-8-ol**

To a solution of 3-(4-benzyl-1-piperazinyl)bicyclo[3.2.1]octan-8-one ethyleneketal (2.0 g, 5.85 mmol) in acetone (20 ml) was added 2 *N* HCl (10 ml) and the mixture was heated at 90 °C for 8 h. After cooling down, the mixture was basified with sat. $NaHCO_3$aq. and was extracted with $CH_2Cl_2$ (100 ml x 2). The combined extracts were washed with brine (30 ml), dried ($MgSO_4$), filtered, and concentrated *in vacuo.* The resulting crude was dissolved in THF (100 ml) and the solution was cooled to -78 °C. To this solution, 1.18 *M* solution of MeLi (10 ml, 11.8 mmol) was added and the solution was allowed to warm to ambient temperature. $H_2O$ (100 ml) was added to the solution and the solution was extracted with EtOAc (100 ml x 2). The combined extracts were washed with brine (20 ml), dried ($MgSO_4$), filtered, and concentrated *in vacuo.* The residue was purified by column chromatography (NH gel, 50 g, $CH_2Cl_2$) to give 1.5 g of the title compound as a colorless oil (82%).
$^1$H NMR ($CDCl_3$) δ: 2.94-2.84 (m, 4 H), 2.60-2.40 (m, 5 H), 2.00-1.40 (m, 10 H), 1.25(s,3 H).

**B. 3-(1-Piperazinyl)-8-methylbicyclo[3.2.1]octan-8-ol**

3-(4-Benzyl-1-piperazinyl)-8-methylbicyclo[3.2.1]octan-8-ol ethyleneketal was deprotected by a procedure similar to that described in example1,H to afford 3-(1-piperazinyl)-8-methylbicyclo[3.2.1]octan-8-ol.
$^1$H NMR ($CDCl_3$) δ: 2.94-2.84 (m, 4 H), 2.60-2.40 (m, 5 H), 2.00-1.40 (m, 10 H), 1.25(s,3H).

**C. Dimethyl 4-(2,6-dichlorophenyl)-2-[2-[4-(8-hydroxy-8-methylbicyclo [3.2.1]oct-3-yl)-1-piperazinyl]-2-oxoethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 3-(1-piperazinyl)-8-methylbicyclo[3.2.1]octan-8-ol according to the procedure described in example 1, I.
$^1$H NMR ($CDCl_3$) δ: 8.38 (br.s, 1 H), 7.74-7.67 (m, 1 H), 7.23 (d, *J* = 7.9 Hz, 2 H), 7.24-7.17 (m, 1 H), 7.04-6.94 (m, 1 H), 5.99 (s, 1 H), 4.07 (d, *J* = 15.1 Hz, 1 H), 3.81 (d, *J* = 15.1 Hz, 1 H), 3.81 (d, *J* = 15.0 Hz, 1 H), 3.74-3.50 (m, 4 H), 3.55 (s, 3 H), 3.53 (s, 3 H), 3.40-3.20 (m, 3 H), 3.10-2.92 (m, 1 H), 2.64-2.30 (m, 5 H), 2.00-1.60 (m, 6 H), 1.60-1.40 (m, 4 H), 1.25 (s, 3 H).
IR (KBr) ν: 3225, 2949, 1695, 1627, 1506, 1434, 1294, 1186, 1103 $cm^{-1}$.
MS (ESI) 717.17 $(M+H)^+$, 715.23 $(M-H)^-$

**Example 6**

**Dimethyl 4-(2,6-dichlorophenyl)-2-[2-[4-(8-butyl-8-hydroxybicyclo[3.2.1]oct-3-yl)-1-piperazinyl]-2-oxoethyl]-6-[2-1,3-thiazol-2-yl)ethyl]-1,4-dihydro-3,5-pyridinedicarboxylate**

**A. 4-(8-Butyl-8-hydroxybicyclo[3.2.1]oct-3-yl)piperazine**

This compound was prepared by a procedure similar to that described in example 5, A & B.
$^1$H NMR ($CDCl_3$) δ: 2.94-2.84 (m, 4 H), 2.60-2.46 (m, 5 H), 2.00-1.35 (m, 16 H), 0.96-0.86 (m, 3 H).

**B. Dimethyl 4-(2,6-Dichlorophenyl)-2-[2-[4-(8-butyl-8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 4-(8-butyl-8-hydroxybicyclo[3.2.1]oct-3-yl)piperazine according to the procedure described in example 1, I.
$^1$H NMR ($CDCl_3$) δ: 8.37 (br.s, 1 H), 7.72 (d, *J* = 3.5 Hz, 1 H), 7.23 (d, *J* = 7.9 Hz, 2 H), 7.20 (d, *J* = 3.3 Hz, 1 H), 6.99 (t, *J* = 8.2 Hz, 1 H), 5.99 (s, 1 H), 4.10 (d, *J* = 15.0 Hz, 1 H), 3.80 (d, *J* = 15.0 Hz, 1 H), 3.70-3.50 (m, 4 H), 3.55 (s, 3 H), 3.54 (s, 3 H), 3.40-3.24 (m, 3 H), 3.10-2.96 (m, 1 H), 2.64-2.42 (m, 5 H), 2.08-1.20 (m, 16 H), 0.97-0.85 (m, 3 H).

monohydrochloride form

$^1$H NMR (DMSO-$d_6$) δ: 7.76 (d, *J* = 3.1 Hz, 1 H), 7.65 (d, *J* = 3.3 Hz, 1 H), 7.35 (d, *J* = 8.1 Hz, 2 H), 7.16 (t, *J* = 8.4 Hz, 1 H), 5.87 (s, 1 H), 4.50-1.00 (m, 31 H), 3.39 (s, 3 H), 0.94-0.82 (m, 3 H).
m.p. 174-177 °C (dec.)
IR (KBr) ν: 3400, 2951, 1695, 1627, 1508, 1434, 1234, 1188 $cm^{-1}$.
MS (ESI) 758.98 $(M+H)^+$

**Example 7**

**Dimethyl 4-(2,6-dichlorophenyl)-2-[2-[4-(8-hydroxy-8-isopropylbicyclo [3.2.1]oct-3-yl)-1-piperazinyl]-2-oxoethyl]-6-[2-(1,3-thiazol-2-yl)ethyl]-1,4-dihydro-3,5-pyridinedicarboxylate**

**A. 4-(8-Isopropyl-8-hydroxybicyclo[3.2.1]oct-3-yl)piperazine**

This compound was prepared by a procedure similar to that described in example 5, A & B.
$^1$H NMR ($CDCl_3$) δ: 2.94-2.86 (m, 4 H), 2.64-2.48 (m, 5 H), 2.20-1.30 (m, 11 H), 0.91 (d, *J* = 6.8 Hz, 6 H).

**B. Dimethyl 4-(2,6-dichlorophenyl)-2-[2-[4-(8-hydroxy-8-isopropyl bicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 4-(8-isopropyl-8-hydroxybicyclo[3.2.1]oct-3-yl)piperazine according to the procedure described in example 1, I.
$^1$H NMR ($CDCl_3$) δ: 8.37 (br.s, 1 H), 7.72 (d, *J* = 3.5 Hz, 1 H), 7.23 (d, *J* = 7.9 Hz, 2 H), 7.20 (d, *J* = 3.3 Hz, 1 H), 6.99 (t, *J* = 7.7 Hz, 1 H), 6.00 (s, 1 H), 4.10 (d, *J* = 14.8 Hz, 1 H), 3.81 (d, *J* = 14.8 Hz, 1 H), 3.70-3.50 (m, 4 H), 3.55 (s, 3 H), 3.54 (s, 3 H), 3.40-3.26 (m, 3 H), 3.08-2.95 (m, 1 H), 2.64-1.20 (m, 16 H), 0.91 (d, *J* = 6.8 Hz, 6 H).

monohydrochloride form

$^1$H NMR (DMSO-$d_6$) δ: 7.76 (d, *J* = 3.1 Hz, 1 H), 7.65 (d, *J* = 3.3 Hz, 1 H), 7.35 (d, *J* = 8.7 Hz, 2 H), 7.16 (t, *J* = 8.7 Hz, 1 H), 5.87 (s, 1 H), 4.50-1.34 (m, 31 H), 3.43 (s, 3 H), 3.39 (s, 3 H), 0.84 (d, *J* = 6.0 Hz, 6 H).
m.p. 178-180 °C (dec.)
IR (KBr) ν: 3400, 2949, 1695, 1627, 1508, 1435, 1294, 1190 $cm^{-1}$.
MS (ESI) 744.98 $(M+H)^+$

**Example 8**

**Dimethyl 4-(2,6-dichlorophenyl)-2-[2-[4-(8-methoxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

**A. 1-Benzyl-4-(8-methoxybicyclo[3.2.1]oct-3-yl)-1-piperazine**

To a solution of 3-(4-benzyl-1-piperazinyl)bicyclo[3.2.1]octan-8-one ethyleneketal (2.0 g, 5.85 mmol) in acetone (20 ml) was added 2 *N* HCl (10 ml) and the mixture was heated at 90 °C for 8 h. After cooling down, the mixture was basified with sat. $NaHCO_3$aq. and was extracted with $CH_2Cl_2$ (100 ml x 2). The combined extracts were washed with brine (30 ml), dried ($MgSO_4$), filtered, and concentrated *in vacuo.*

A part of the resulting crude (1.87 g, 6.28 mmol) was dissolved in MeOH (40 ml) and $NaBH_4$ (238 mg, 6.28 mmol) was added portionwise to the solution at 0 °C. After 3h, acetone (1ml) followed by $H_2O$ (30 ml) was added. The resulting aqueous mixture was extracted with $CH_2Cl_2$ (50 ml x 3). The combined extracts were dried ($MgSO_4$), filtered, and concentrated to give a white solid (1.9 g/quant).

A part of the resulting crude (490 mg, 1.63 mmol) was dissolved in THF (10 ml) and KH (98 mg, 2.45 mmol) was added to this solution at 0 °C. After 10 min, methyliodie (0.2 ml, 3.21 mmol) was added at the same temperature and the resulting mixture was stirred for 2h at ambient temperature. The mixture was poured into $H_2O$ (30 ml) and the resulting mixture was extracted with EtOAc (50 ml x 2). The combined extracts were washed with brine (10 ml), dried ($MgSO_4$), filtered, and concentrated. The residue was purified by column chromatography (NH gel, $CH_2Cl_2$ to give 200 mg of the title compound as a white solid (39%).

$^1$H NMR ($CDCl_3$) δ: 7.34-7.20 (m, 5 H), 3.51 (s, 2 H), 3.36-3.32 (m, 1 H), 3.33 (s, 3 H), 2.65-1.40 (m, 19 H).

MS (EI) 314 ($M^+$)

**B. 1-(8-Methoxybicyclo[3.2.1]oct-3-yl)-1-piperazine**

1-Benzyl-4-(8-methoxybicyclo[3.2.1]oct-3-yl)-1-piperazine was deprotected by a procedure similar to that described in example 1,H to afford 1-(8-methoxybicyclo[3.2.1]oct-3-yl)-1-piperazine

$^1$H NMR ($CDCl_3$) δ: 3.66 (t, *J* = 4.6 Hz, 1 H), 3.35 (s, 3 H), 2.94-2.84 (m, 4 H), 2.60-2.40 (m, 4 H), 2.00-1.40 (m, 10 H).

**C. Dimethyl 4-(2,6-dichlorophenyl)-2-[2-|4-(8-methoxybicyclo [3.2.1]oct-3-yl)-1piperazinyl]-2-oxoethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 1-(8-methoxybicyclo[3.2.1]oct-3-yl)-1-piperazine according to the procedure described in example 1, I.

$^1$H NMR ($CDCl_3$) δ: 8.37 (br.s, 1 H), 7.72 (d, *J* = 3.3 Hz, 1 H), 7.23 (d, *J* = 8.1 Hz, 2 H), 7.20 (d, *J* = 3.3 Hz, 1 H), 6.99 (t, *J* = 7.6 Hz, 1 H), 6.00 (s, 1 H), 4.12 (d, *J* = 15.0 Hz, 1 H), 3.79 (d, *J* = 15.0 Hz, 1 H), 3.70-3.50 (m, 4 H), 3.55 (s, 3 H), 3.54 (s, 3 H), 3.40-3.28 (m, 4 H), 3.34 (s, 3 H), 3.08-2.95 (m, 1 H), 2.64-1.36 (m, 15 H).

nomohydrochloride form

$^1$H NMR (DMSO-$d_6$) δ: 7.73 (d, *J* = 3.3 Hz, 1 H), 7.63 (d, *J* = 3.3 Hz, 1 H), 7.35 (d, *J* = 7.9 Hz, 2 H), 7.16 (t, *J* = 7.4 Hz, 1 H), 5.87 (s, 1 H), 4.50-1.50 (m, 26 H), 3.43 (s, 3 H), 3.39 (s, 3 H), 3.28 (s, 3 H).

IR (KBr) ν: 3649, 3213, 2947, 1695, 1624, 1508, 1434, 1296 $cm^{-1}$.

MS (ESI) 716.86 $(M+H)^+$

**Example 9**

**Dimethyl 2-[2-(4-bicyclo[2.2.2]oct-2-yl-1-piperazinyl)-2-oxoethyl]-4-(2,6-dichlorophenyl)-6-[2-(1,3-thiazol-2-yl) ethyl]-1,4-dihydro-3,5-pyridinedicarboxylate**

**A. 1-Benzyl-4-bicyclo[2.2.2]oct-5-en-2-yl-1-piperazine**

To a solution of bicyclo[2.2.2]oct-5-en-2-one (*J. Org. Chem.,* **1983**, 1000, 2.5 g, 20.5 mmol) and N-benzylpiperazine (3.82 ml, 22 mmol) in 1,2-dichloroethane (70 ml) were added $NaBH(OAc)_3$ (5.3 g, 25 mmol) and acetic acid (2 ml) at room temperature, under nitrogen atmosphre. The resulting suspension was stirred at reflux temperature for 5 h under nitrogen atmosphere. After cooled to room temperature, the reaction mixture was poured into 2 *N* NaOHaq.

and extracted with $CH_2Cl_2$. Organic layer was washed with brine and dried ($MgSO_4$), then concentrated. Residue was purified by column chromatography ($SiO_2$, EtOAc/hexane = 1/7 to 1/5) to give 1.28 g (22 %) of the title compound.
$^1H$ NMR ($CDCl_3$) δ 7.32-7.22 (m, 5 H), 6.32-6.15 (m, 2 H), 3.51 (s, 2 H), 2.68 (m, 1 H), 2.47 (m, 8 H), 1.88-0.94 (m, 8 H)

**B. 1-Bicyclo[2.2.2]oct-2-yl-1-piperazine**

The mixture of 1.27 g (4.49 mmol) of 1-Benzyl-4-bicyclo[2.2.2]oct-5-en-2-ylpiperazine and 400 mg of $Pd(OH)_2$ (20 wt% on carbon) in 50 ml of MeOH was stirred at room temperature for 6 h under hydrgen atmosphere, then filtered through a pad of celite. The filtrate was concentrated to give 837.4 mg (96 %) of the title compound.
$^1H$ NMR ($CDCl_3$) δ 2.89 (t, *J* = 4.7 Hz, 2 H), 2.41 (bs, 3 H), 2.04 (m, 1 H), 1.77-1.24 (m, 7 H)

**C. Dimethyl 2-[2-(4-bicyclo[2.2.2]oct-2-yl-1-piperazinyl)-2-oxoethyl]-4-(2,6-dichloropbenyl)-6[2-(1,3-thiazol-2-yl)ethyl]-1,4-dihydro-3,5-pyridinedicarboxylate**

The title compound was prepared from [4-(2,6-dichlorophenyl)-3,5-bis(methoxycarbonyl)-6-[2-(1,3-thiazol-2-yl)ethyl]-1,4-dihydro-2-pyridinyl]acetic acid and 1-bicyclo[2.2.2]oct-2-ylpiperazine according to the procedure described in example 1, I.
$^1H$ NMR ($CDCl_3$) δ 8.38 (s, 1 H), 7.71 (d, *J* = 3.2 Hz, 1 H), 7.26-7.19 (m, 3 H), 7.00 (t, *J* = 7 Hz), 5.99 (s, 1 H), 4.08 (dd, *J* = 4.8, 14 Hz, 1 H), 3.83 (dd, *J* = 4.8, 14 Hz, 1 H), 3.62 (m, 3 H), 3.55 (s, 3 H), 3.53 (s, 3 H), 3.40-3.25 (m, 4 H), 3.01 (m, 1 H), 2.41 (m, 1 H), 2.06 (m, 1 H), 1.73-1.26 (m, 15 H)

monohydrochloride form

IR (KBr) ν: 3392, 3219, 3091, 2945, 2868, 2596, 2457, 1693, 1624, 1577, 1560, 1508, 1433, 1296, 1259, 1230, 1190, 1103, 1056, 991, 966, 929, 862, 842, 767 $cm^{-1}$
MS (ESI) 687.30 $(M+H)^+$
m.p. 165-168°C

**Example 10**

**Dimethyl 4-(2,6-dichlorophenyl)-2-[2-oxo-2-[4-(5-oxooctahydro-2-pentalenyl)-1-piperazinyl]ethyl]-6-(2-(1,3-thiazol-2-yl)ethyl)-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 4-(5-oxooctahydro-2-pentalenyl)-1-piperazine according to the procedure described in example 1, I.
$^1H$ NMR ($CDCl_3$) δ: 8.34 (br.s, 1 H), 7.71 (d, *J* = 3.3 Hz, 1 H), 7.24 (d, *J* = 8.1 Hz, 2 H), 7.21 (d, *J* = 3.3 Hz, 1 H), 7.00 (t, *J* = 7.4 Hz, 1 H), 6.00 (s, 1 H), 4.05 (d, *J* = 15.0 Hz, 1 H), 3.84 (d, *J* = 15.1 Hz, 1 H), 3.74-3.20 (m, 8 H), 3.55 (s, 3 H), 3.53 (s, 3 H), 3.08-2.92 (m, 1 H), 2.80-2.00 (m, 12 H), 1.44-1.27 (m, 2 H).

nomohydrochloride form

$^1H$ NMR (DMSO-$d_6$) δ: 7.71 (d, *J* = 3.5 Hz, 1 H), 7.61 (d, *J* = 3.3 Hz, 1 H), 7.35 (d, *J* = 8.1 Hz, 2 H), 7.16 (t, *J* = 8.6 Hz, 1 H), 5.87 (s, 1 H), 3.94 (s, 6 H), 4.50-1.50 (m, 25 H).
IR (KBr) ν: 3566, 3546, 3524, 3369, 1733, 1717, 1695, 1647, 1508, 1435, 1296, 1230, 1180, 1101 $cm^{-1}$.
m.p. 214-220 °C (dec.)
MS (ESI) 701.16 $(M+H)^+$, 699.18 $(M-H)^-$

**Example 11**

**Dimethyl 4-(2,6-dichlorophenyl)-2-[2-[4-(1-methyl-3-piperidinyl)-1-piperazinyl]-2-oxoethyl]-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 4-(1-methyl-3-piperidinyl)-1-piperazine according to the procedure described in example 1, I.
$^1H$ NMR ($CDCl_3$) δ: 8.34 (s, 1 H), 7.71 (d, *J* = 3 Hz, 1 H), 7.23 (d, *J* = 7 Hz, 2 H), 7.20 (d, *J* = 3 Hz, 1 H), 6.99 (t, *J* = 7 Hz, 1 H), 5.99 (s, 1 H), 4.14 (dd, *J* = 14, 2 Hz, 1 H), 3.74 (dd, *J* = 14, 2 Hz, 1 H), 3.61 (m, 4 H), 3.55 (s, 3 H), 3.53 (s,

3 H), 3.38-3.24 (m, 3 H), 3.09-2.90 (m, 2 H), 2.76-2.72 (m, 1 H), 2.58-2.46 (m, 6 H), 2.27 (s, 3 H), 1.84-1.72 (m, 5 H), 1.62-1.48 (m, 1 H), 1.22-1.13 (m, 1 H)

dihydrochloride form

IR (KBr) ν: 3421, 3224, 2567, 1691, 1624, 1560, 1508, 1434, 1296, 1188, 1105, 1053, 767 $cm^{-1}$
m.p. 170-175 °C (dec.)

**Example 12**

**Dimethyl 2-[2-[4-(1-benzyl-3-piperidinyl)-1-piperazinyl]-2-oxoethyl]-4-(2,6-dichlorophenyl)-6-[2-(1,3-thiazol-2-yl)ethyl]-1,4-dihydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 4-(1-benzyl-3-piperidinyl)-1-piperazine according to the procedure described in example 1, I.
$^1$H NMR ($CDCl_3$) δ: 8.33 (s, 1 H), 7.71 (d, *J* = 3 Hz, 1 H), 7.31-7.19 (m, 8 H), 6.99 (m, 1 H), 5.99 (s, 1 H), 4.12 (d, *J* = 14 Hz, 1 H), 3.74 (d, *J* = 15 Hz, 1 H), 3.61-3.50 (m, 12 H), 3.39-3.27 (m, 3 H), 3.04-2.95 (m, 2 H), 2.80-2.75 (m, 1 H), 2.58-2.48 (m, 7 H), 1.91-1.27 (m, 4 H)

dihydrochloride form

IR (KBr) ν: 3394, 2949, 2549, 1691, 1624, 1508, 1433, 1296, 1234, 1190, 1105, 1055, 1020, 767, 754, 702 $cm^{-1}$
m.p. 161-164 °C (dec.)

**Example 13**

**Dimethyl 4-(2,6-dichlorophenyl-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridine-3,5-dicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 1-(*exo*-8-methyl-8-azabicyclo[3.2.1]oct-3-yl)piperazine according to the procedure described in example 1, I.
NMR ($CDCl_3$) δ 8.35 (br.s, 1 H), 7.71 (d, *J* = 3.3 Hz, 1 H), 7.23 (d, *J* = 8.1 Hz, 2 H), 7.20 (d, *J* = 3.4 Hz, 1 H), 7.00 (dd, *J* = 7.5, 8.4 Hz, 1 H), 5.99 (s, 1 H), 4.10 (d, *J* = 15.3 Hz, 1 H), 3.77 (d, *J* = 14.8 Hz, 1 H), 3.56-3.60 (m, 4 H), 3.55 (s, 3 H), 3.53 (s, 3 H), 3.22-3.36 (m, 3 H), 3.16-3.25 (m, 2 H), 2.95-3.06 (m, 1 H), 2.43-2.64 (m, 5 H), 2.28 (s, 3 H), 1.98-2.07 (m, 2 H), 1.48-1 70 (m, 6 H).

**Example 14**

**(-)-Dimethyl 4-(2,6-dichlorophenyl)-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridine-3,5-dicarboxylate**

The racemic mixture of example 13 (dimethyl 4-(2,6-dichlorophenyl)-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridine-3,5-dicarboxylate) was separated by Daicel chiral-pac AD (20mmx250mm) eluted with hexane/EtOH/$Et_2NH$ = 85/15/0.1 at a flow late was 6 ml/min. Samples were injected every 25 min. and 720 mg of earlier fraction (RT 31 min) and 752 mg of later fractions (RT = 42 min) were obtained out of 1.6 g of the racemic mixture. Both isomers were independently concentrated *in vacuo.* The residue of the later fraction (RT = 42 min) was dissolved in $CH_2Cl_2$ (50 ml) and the solution was washed with $H_2O$ (50 ml). The solution was dried ($Na_2SO_4$) and concentrated *in vacuo* to give a yellow oil. Chromatography on NH-propyl silica gel (100 g) eluted with $CH_2Cl_2$/MeOH (100/0 to 200/1 to 100/1) to give a pale yellow solid (626 mg).
NMR spectrum of the free base was identical to that of the racemic one.

dihydrochloride form

626 mg of the free base obtained above was dissolved in MeOH (5 ml) and 5% metanolic HCl solution (22ml) was added. The mixture was filtered on cotton, then stirred for 30 min at room temperature. The mixture was concentrated *in vacuo* and the residue was dissolved in isopropanol (20 ml) and diisopropyl ether (1 ml). The precipitate was

collected by suction filtration and dried at 90°C under vacuum for 24 hours to give 540.8 mg of dihydrochloride as off white solids (78%).
$[\alpha]^D$ = -47.244 (c=0.1, MeOH)
m.p. 200-202 °C (dec.)
NMR (DMSO-$d_6$) δ 10.80 (br.s, 1 H), 9.48-9.33 (m, 1 H), 7.71 (d, 1 H, *J* = 3.3 Hz), 7.61 (d, 1 H, *J* = 3.3 Hz), 7.37 (s, 1 H), 7.16 (t, 1 H, *J* = 8.0 Hz), 5.87 (s, 1 H) (higher field protons were overlapped and could not be assigned)
IR (KBr); ν 3415, 1683, 1649, 1624, 1502, 1467, 1433, 1294, 1178, 1103 $cm^{-1}$.
MS (ESI) 702.19 $(M+H)^+$

**Example 15**

**Dimethyl 4-(2,6-dichlorophenyl)-2-[2-[4-(*endo*-8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate**

**A. Methyl 2-[(8-methyl-8-azabicyclo[3.2.1]oct-3-yl)amino]acetate**

To a suspension of $NaBH_4$ (3.8 g, 100 mmol) in $CH_2Cl_2$ (150 ml) was added dropwise ethylhexanoic acid (56 ml, 350 mmol) at ambient temperature over a period of 2 h. The resulting suspension was stirred for 15 h. The suspension turned to a clear solution. To this solution was added glycine methylester hydrochloride (9.4 g, 75 mmol) followed by tropinone (6.95 g, 50 mmol), and then the resulting mixture was stirred overnight. The mixture was basified with 2 N NaOHaq. and extracted with $CH_2Cl_2$ (150 ml x 2). The combined extracts were washed with brine (30 ml), dried ($MgSO_4$), filtered, and concentrated to give a pale yellow oil (11 g/quant.).
$^1$H NMR ($CDCl_3$) δ: 3.72 (s, 3 H), 3.67 (s, 2 H), 3.15 (br.s 2 H), 2.82 (bt, *J* = 6.1 Hz, 1 H), 2.32 (s, 3 H), 2.20-1.90 (m, 6 H), 1.60-1.48 (m, 2 H), 1.35-1.20 (m, 1 H).

**B. Methyl 2-[(2-chloroacetyl)(8-methyl-8-azabicyclo[3.2.1]oct-3-yl)amino]acetate**

To a solution of methyl 2-[(8-methyl-8-azabicyclo[3.2.1]oct-3-yl)amino]acetate (3.1 g, 14.6 mmol) in $CH_2Cl_2$ (73 ml) was added chloroacethylchloride (1.4 ml, 17.5 mmol) at 0 °C. The mixture was stirred for 4h at ambient temperature and sat.$NaHCO_3$aq. (30 ml) was added. The mixture was extracted with $CH_2Cl_2$ (100 ml x 4) and the combined extracts were dried ($MgSO_4$), filtered, and concentrated to give 3.8 g of the title compound as a pale brown oil (90%).
$^1$H NMR ($CDCl_3$) δ: 4.47 (s, 2 H), 4.35-4.10 (m, 1 H), 3.72 (s, 2 H), 3.70 (s, 3 H), 3.28-3.13 (m, 2 H), 2.52-2.38 (m, 2 H), 2.14 (s, 3 H), 2.50-1.00 (m).
MS (EI) 288 $(M^+)$

**C. 1-(*endo*-8-methyl-8-azabicyclo[3.2.1]oct-3-yl)-2,5-piperazinedione**

To a solution of methyl 2-[(2-chloroacetyl)(8-methyl-8-azabicyclo[3.2.1]oct-3-yl)amino]acetate (3.79 g, 13.1 mmol) in 2-propanol (13.1 ml) was added 2*M* $NH_3$ in MeOH (13.1 ml) and the resulting solution was stand for 2 days at ambient temperature. The solution was saturated with NaCl and extracted with $CH_2Cl_2$ (50 ml x 4). The combined extracts were dried ($MgSO_4$), filtered, and concentrated to give 2.1 g of the title compound as a white solid (68%).
$^1$H NMR ($CDCl_3$) δ: 7.06 (br.s, 1 H), 4.74 (tt, *J* = 10.9, 8.2 Hz, 1 H), 3.96 (br.s, 2 H), 3.80 (s, 2 H), 3.30-3.18 (m, 3 H), 2.40-2.10 (m), 2.17 (s, 3 H), 1.60-1.40 (m), 1.34-1.18 (m).
MS (EI) 237 $(M^+)$

**D. 1-(*endo*-8-methyl-8-azabicyclo[3.2.1]oct-3-yl)piperazine**

A mixture of 1-(8-methyl-8-azabicyclo[3.2.1]oct-3-yl)-2,5-piperazinedione (2.0 g, 8.4 mmol) and $LiAlH_4$ (1.28 g, 33.8 mmol) in dioxane (42 ml) was refluxed for 5 h. After cooling, 2 N NaOHaq. was added for quenching and the resulting suspension was stirred overnight. The suspension was filtered and the filtrated was dried ($MgSO_4$), filtered, and concentrated. The residue was purified by column chromatography (NH gel, $CH_2Cl_2$/MeOH = 100/4) to give a pale yellow oil.
$^1$H NMR ($CDCl_3$) δ: 3.15-3.00 (m, 2 H), 2.88 (t, *J* = 4.9 Hz, 4 H), 2.55-1.50 (m, 15 H), 2.20 (s, 3 H).

**E. Dimethyl 4-(2,6-dichlorophenyl)-2-[2-[4-(*endo*-8-methyl-8-azabicyclo[3.2.1]oct-3-yl)-1-piperazinyl]-2-oxoethyl]-6-[2-(1,3-thiazol-2-yl)ethyl]-1,4-dibydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 1-(*endo*-8-methyl-8-azabicyclo[3.2.1]oct-3-yl)piperazine according to the procedure described in example 1, I.
$^1$H NMR ($CDCl_3$) δ: 8.37 (br.s, 1 H), 7.71 (d, *J* = 3.3 Hz, 1 H), 7.24 (d, *J* = 7.9 Hz, 2 H), 7.21 (d, *J* = 3.5 Hz, 1 H), 7.00 (t, *J* = 8.2 Hz, 1 H), 5.99 (s, 1 H), 4.08 (d, *J* = 15.0 Hz, 1 H), 3.83 (d, *J* = 15.0 Hz, 1 H), 3.55 (s, 3 H), 3.54 (s, 3 H), 3.70-3.50 (m, 4 H), 3.42-3.25 (m, 3 H), 3.15-2.94 (m, 3 H), 2.53-2.30 (m, 5 H), 2.21 (s, 3 H), 2.14-1.90 (m, 4 H).

<u>dihydrochloride form</u>

$^1$H NMR (DMSO-$d_6$) δ: 7.74 (d, *J* = 3.3 Hz, 1 H), 7.63 (d, *J* = 3.3 Hz, 1 H), 7.35 (d, *J* = 7.7 Hz, 2 H), 7.16 (t, *J* = 7.6 Hz, 1 H), 5.87 (s, 1 H), 3.43 (s, 3 H), 3.39 (s, 3 H).
IR (KBr) ν: 3398, 3213, 2949, 1693, 1653, 1508, 1433, 1294 $cm^{-1}$.
MS (ESI) 702.23 $(M+H)^+$

**Example 16**

**<u>3-Ethyl 5-methyl 4-(2,6-dichlorophenyl)-2-[2-[4-(*exo*-8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate</u>**

**<u>A. 3-Ethyl 5-methyl 4-(2,6-dichlorophenyl)-2-(2-ethoxy-2-oxoethyl)-6-[2-(1,3-thiazol-2-yl)ethyl]-1,4-dihydropyridine-3,5-dicarboxylate</u>**

A mixture of methyl 3-(2,6-dichlorophenyl)-2-[1,3-thiazol-2-yl)propanoyl]-2-propenoate (1.08 g, 2.9 mmol) and diethyl 3-amino-2-pentenedioate (*J. Med. Chem.* **1975**, *18*, 509, 587 mg, 2.9 mmol) was heated at 120°C (bath temp) for 16 h. After cooling down to room temperature, the reaction mixture was purified by column chromatography on silica gel (50 g, hexane/EtOAc = 2/1 to 3/2) to afford 635 mg (40%) of 3-ethyl 5-methyl 4-(2,6-dichlorophenyl)-2-(2-ethoxy-2-oxoethyl)-6-[2-(1,3-thiazol-2-yl)ethyl]-1,4-dihydropyridine-3,5-dicarboxylate as a brown oil.
$^1$H NMR ($CDCl_3$) δ: 8.31 (br.s, 1 H), 7.67 (d, *J* = 3.5 Hz, 1 H), 7.28-7.20 (m, 3 H), 6.99 (dd, *J* = 7.6, 7.6 Hz, 1 H), 5.99 (s, 1 H), 4.15 (q, *J* = 7.1 Hz, 2 H), 4.01 (q, *J* = 7.1 Hz, 2 H), 3.82 (d, *J* = 16.6 Hz, 1 H), 3.55 (s, 3 H), 3.53 (d, *J* = 16.6 Hz, 1 H), 3.42-3.25 (m, 3 H), 3.11-2.97 (m, 1 H), 1.24 (t, *J* = 7.1 Hz, 3 H), 1.08 (t, *J* = 7.1 Hz, 3 H).

**<u>B. 2-[4-(2,6-Dichlorophenyl)-3-ethoxycarbonyl-5-methoxycarbonyl-6-[2-(1,3-thiazol-2-yl)ethyl]-1,4-dihydro-2-pyridinyl]acetic acid</u>**

The title compound was prepared by the method of example 1, E. To a stirred solution of 3-ethyl 5-methyl 4-(2,6-dichlorophenyl)-2-(2-ethoxy-2-oxoethyl)-6-[2-(1,3-thiazol-2-yl)ethyl]-1,4-dihydropyridine-3,5-dicarboxylate (630 mg, 1.1 mmol) in dioxane (10 ml) was added 2 NNaOHaq. (2 ml, 4.0 mmol) and the reaction mixture was stirred for 3 h at room temperature. The mixture was pertitioned between water (30 ml) and $Et_2O$ (50 ml). The aqueous phase was separated and then acidified with 2 *N* HCl (3 ml). The aqueous mixture was extracted with $CH_2Cl_2$ (50 ml x 2). The organic layers were washed with brine, dried ($Na_2SO_4$) and evaporated to give 500 mg (87%) of 2-[4-(2,6-dichlorophenyl)-3-ethoxycarbonyl-5-methoxycarbonyl-6-[2-(1,3-thiazol-2-yl)ethyl]-1,4-dihydro-2-pyridinyl]acetic acid as yellow solids.
$^1$H NMR ($CDCl_3$) δ: 8.71 (br.s, 1 H), 7.79 (d, *J* = 3.4 Hz, 1 H), 7.35-7.20 (m, 3 H), 7.03 (t, *J* = 7.6 Hz, 1 H), 5.99 (s, 1 H), 4.10 (q, *J* = 7.1 Hz, 2 H), 3.71-3.62 (m, 2 H), 3.56 (s, 3 H), 3.50-3.38 (m, 2 H), 3.36-3.22 (m, 1 H), 3.10-2.95 (m, 1 H), 1.15 (t, *J* = 7.1 Hz, 3 H).

**<u>C. 3-Ethyl 5-methyl 4-(2,6-dichlorophenyl)-2-[2-[4-(*exo*-8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate</u>**

The title compound was prepared from 2-[4-(2,6-dichlorophenyl)-3-ethoxycarbonyl-5-methoxycarbonyl-6-[2-(1,3-thiazol-2-yl)ethyl]-1,4-dihydro-2-pyridinyl]acetic acid and 1-(*exo*-8-methyl-8-azabicyclo[3.2.1]oct-3-yl)piperazine according to the procedure described in example 1, I.
$^1$H NMR ($CDCl_3$) δ: 8.31 (br.s, 1 H), 7.72 (d, *J* = 3.3 Hz, 1 H), 7.40-7.15 (m, 3 H), 7.00 (dd, *J* = 7.6, 7.6 Hz, 1 H), 5.98 (s, 1 H), 4.15-3.94 (m, 3 H), 3.79 (d, *J* = 14.8 Hz, 1 H), 3.70-3.46 (m, 7 H), 3.42-3.29 (m, 3 H), 3.29-3.16 (m, 2 H), 3.08-2.94 (m, 1 H), 2.66-2.40 (m, 5 H), 2.32 (s, 3 H), 2.09-1.88 (m, 2 H), 1.73-1 45 (m, 6 H), 1.09 (t, *J* = 7.6 Hz, 3 H).
IR (KBr) ν: 3398, 3084, 2978, 2557, 1686, 1637, 1508, 1433, 1292, 1229, 1190, 1101, 1051, 964, 768 $cm^{-1}$.
MS (ESI) 716.44 $(M+H)^+$

**Example 17**

**Dimethyl 4-(2,6-dichlorophenyl)-2-(4-(*exo*-8-methyl-8-azabicyclo[3.2.1]oct-3-yl)-1-piperazinyl]-2-oxoethyl-6-[2-(1-methyl-*1H*-imidazol-2-yl)ethyl]-1,4-dihydropyridine-3,5-dicarboxylate**

**A. Methyl 3-oxo-5-(1-methyl-*1H*-imidazol-2-yl)pentanoate**

A solution of methyl acetoacetate (4.87 g, 42 mmol) in THF (10 ml) was added dropwise to a stirred suspension of NaH (60% in oil, 1.68 g, 42 mmol) in THF (40 ml) at 0°C under nitrogen atmosphere and stirred for 30 min. A solution of n-BuLi in hexane (1.54 *M,* 27.3 ml, 42 mmol) was added to the mixture at 0°C and stirred for 30 min. 2-chloromethyl-1-methyl-*1H*-imidazole hydrochloride (3.5 g, 21 mmol), prepared according to the literature (*Tetrahedron* **1996**, *52,* 15171.), was added portionwise to the mixture at 0°C and stirred for 3 hours. The mixture was poured into an aqueous saturated solution of sodium dihydrogenphosphate (50 ml) and acidified with 2 N HCl aq. until pH 1. The whole was washed with EtOAc (50 ml). The aqueous layer was basified with into an aqueous saturated solution of sodium hydrocarbonate until pH 9 and extracted with $CH_2Cl_2$ (100 ml x 2). The combined prganic layers was washed with brine, dried ($MgSO_4$), and concentrated *in vacuo.* The residue was purified on silica gel, eluting with $CH_2Cl_2$/MeOH = 30/1, to afford 1.56 g of the title compound as a brown oil.
$^1$H NMR ($CDCl_3$) δ 6.88 (d, *J* = 1.2 Hz, 1 H), 6.78 (d, *J* = 1.2 Hz, 1 H), 3.73 (s, 3 H), 3.60 (s, 3 H), 3.54 (s, 2 H), 3.16 (t, *J* = 7.1 Hz, 2 H), 2.91 (t, *J* = 7.1 Hz, 2 H)

**B. Methyl 2-[4-(2,6-dichlorophenyl)-3,5-bis(methoxycarbonyl)-6-[2-(1-methyl-*1H*-imidazol-2-yl)ethyl]-1,4-dihydro-2-pyridinyl]acetate**

The title compound was prepared from methyl 3-oxo-5-(1-methylimidazol-2-yl)pentanoate according to the procedure of example 1, C & D.
$^1$H NMR ($CDCl_3$) δ 9.51 (br.s, 1 H), 7.22 (d, *J* = 8.0 Hz, 2 H), 6.98 (t, *J* = 8.0 Hz, 1 H), 6.88 (d, *J* = 1.3 Hz, 1 H), 6.80 (d, *J* = 1.3 Hz, 1 H), 5.98 (s, 1 H), 3.80 (d, *J* = 16.3 Hz. 1 H), 3.68 (s, 3 H), 3.58 (s, 3 H), 3.54 (s, 3 H), 3.50 ( s, 3 H), 3.46 (d, *J* = 16.3 Hz. 1 H), 3.40-3.27 (m, 1 H), 3.15-3.04 (m, 1 H), 3.04-2.95 (m, 2 H)

**C. Dimethyl 4-(2,6-dichlorophenyl)-2-[4-(*exo*-8-methyl-8-azabicyclo[3.2.1]oct-3-yl)-1-piperaziny]-2-oxoethyl-6-[2-(1-methylimidazol-2-yl)ethyl]-1,4-dihydropyridine-3,5-dicarboxylate**

The title compound was prepared from methyl 2-[4-(2,6-dichlorophenyl)-3,5-bis(methoxycarbonyl)-6-[2-(1-methylimidazol-2-yl)ethyl]-1,4-dihydro-2-pyridinyl]acetate and according to the procedure described in example 1, E & I.
$^1$H NMR ($CDCl_3$) δ 8.97 (br.s, 1 H), 7.22 (d, *J* = 8.0 Hz, 2 H), 6.98 (t, *J* = 8.0 Hz, 1 H), 6.90 (s, 1 H), 6.77 (s, 1 H), 5.99 (s, 1 H), 3.96 (d, *J* = 16.2 Hz. 1 H), 3.46 (d, *J* = 16.2 Hz, 1 H), 3.58 (br.s, 4 H), 3.53 (s, 3 H), 3.54 (s, 3 H), 3.50 (s, 3 H), 3.32-3.16 (m, 3 H), 3.06-2.90 (m, 5 H), 2.62-2.44 (m, 5 H), 2.27 (s, 3 H), 2.04-1.96 (s, 2 H), 1.73-1.48 (m,6 H).

trihydrochloride form

$^1$H NMR (DMSO-$d_6$) δ 10.98 (br.s, 1 H), 7.61 (d, *J* = 1.5 Hz, 1 H), 7.58 (d, *J* = 1.5 Hz, 1 H), 7.35 (d, *J* = 7.9 Hz, 2 H), 7.16 (t, *J* = 7.9 Hz, 1 H), 5.83 (s, 1 H), 4.35-3.90 (m, 6 H), 3.83 (s, 3 H), 3.75-2.86 (m, 11 H), 3.40 (s, 3 H), 3.39 (s, 3 H), 2.64 (s, 3 H), 2.49-1.86 (m, 8 H).
m.p. 232 °C (decomposed)

**Example 18**

**Dimethyl 4-(2,6-dichlorophenyl)-2-[2-[4-[8-(*exo*-methyl-8-azabicyclo[3.2.1]oct-3-yl)-1-piperazinyl]-2-oxoethyl]-6-[2-(1,3-oxazol-2-yl)ethyl]-1,4-dihydro-3,5-pyridinedicarboxylate**

**A. 2-[(Benzyloxy)methyl]-1,3-oxazole**

Benzyloxyacetyl chloride (25.0 g, 129 mmol) was added dropwise to a solution of 1,2,3-triazole (9.16 g, 129 mmol) and triethylamine (18 ml, 129 mmol) in benzene (300 ml) at 0 °C and the mixture was stirred for 1 h. After filtration, the reaction mixture washed with water (100 ml) and brine (100 ml), dried over $MgSO_4$, and concentrated *in vacuo.* The residue was dissolved with sulfolune (40 g) and the solution was stirred for 30 min at 150 °C. The reaction mixture was poured into water (150 ml) and the whole was extracted with ether (100 ml x 3). The combined organic

layers were washed with water (100 ml) and brine (100 ml), dried over $MgSO_4$, and concentrated *in vacuo.* The residue was purified by column chromatography ($SiO_2$, hexane/EtOAc = 85/15) to give 7.07 g of the title compound as a colorless oil (29%).
$^1$H NMR ($CDCl_3$) δ 7.66 (d, *J* = 0.8 Hz, 1H), 7.45-7.20 (m, 5 H), 7.11 (d, *J* = 0.8 Hz, 1 H), 4.70-4.55 (m, 4 H).

**B. 1,3-Oxazol-2-ylmethanol**

A mixture of 2-[(benzyloxy)methyl]-1,3-oxazole (7.00 g, 37 mmol), 20% palladium hydroxide on carbon (1 g) was stirred for 2 days under hydrogen (4 kg/$cm^2$) atmosphere. After filtration, the filtrate was concentrated *in vacuo.* The residue was purified by column chromatography ($SiO_2$, EtOAc/hexane = 1/5 to 1/1) to give 0.77 g of the title compound as a colorless oil (21%).
$^1$H NMR ($CDCl_3$) δ 7.65-7.63 (m, 1 H), 7.09-7.07 (m, 1 H), 4.90-4.65 (m, 3 H).

**C. 1,3-Oxazol-2-ylmethyl 4-methylbenzenesulfonate**

To a solution of 1,3-oxazol-2-ylmethanol (0.77 g, 7.77 mmol) in THF (15 ml) was added dropwise 2.5 M solution of n-BuLi in hexane (4.7 ml, 11.7 mmol) at -78 °C under nitrogen atmosphere and the reaction mixture was stirred for 30 min at the same temperature. Then to the mixture was added dropwise a solution of p-toluenesulfonyl chloride (1.48 g, 7.77 mmol) in THF (5 ml) -78 °C, and the mixture was stirred for 1 h at the same temperature. The mixture was poured into saturated aqueous solution of sodium dihydrogenphosphate and the whole was extracted with EtOAc (10 ml x 2). The combined organic layers were washed with brine (10 ml), dried over $MgSO_4$, and concentrated *in vacuo.* The residue was purified by column chromatography ($SiO_2$, EtOAc/hexane = 1/3) to give 550 mg of the titled compound (28%).
$^1$H-NMR ($CDCl_3$) δ 7.81 (d, *J* = 8.1 Hz, 2 H), 7.63 (d, *J* = 0.8 Hz, 1 H), 7.35 (d, *J* = 8.1 Hz, 2 H), 7.08 (d, *J* = 0.8 Hz, 1 H), 5.12 (s, 2 H), 2.45 (s, 3 H).

**D. Methyl 5-(1,3-oxazol-2-yl)-3-oxopentanoate**

To a suspension of sodium hydride (60% in oil, 96 mg, 2.39 mmol) in THF was added a solution of methyl acetoacetate (0.26 ml, 2.39 mmol) in THF (5 ml) at 0 °C and the reaction mixture was stirred for 30 min at the same temperature. To the reaction suspension was added dropwise 2.5 M solution of n-BuLi in hexane (0.96 ml, 2.39 mmol) at 0 °C and the reaction mixture was stirred for 30 min at the same temperature. Then to the reaction mixture was added dropwise a solution of 1,3-oxazol-2-ylmethyl 4-methylbenzenesulfonate in THF (5 ml) at 0 °C and the reaction mixture was stirred for 1 h at the same temperature. The reaction was quenched with the saturated aqueous solution of sodium dihydrogenphosphate and the aqueous mixture was extracted with ether (10 ml x 2). The combined organic layers were washed with brine (10 ml), dried over $MgSO_4$, and concentrated *in vacuo.* The residue was purified on $SiO_2$, eluting with EtOAc/hexane (1/3) to give 140 mg of the titled compound (33%).
$^1$H-NMR ($CDCl_3$) δ 7.56 (s, 1 H), 6.99 (s, 1 H), 3.75 (s, 3 H), 3.54 (s, 2 H), 3.08 (s, 4 H).

**E. Mehtyl 3-(2,6-dichlorophenyl)-2-[3-(1,3-oxazol-2-yl)propanoyl]-2-propenoate**

The title compound was prepared from methyl 5-(1,3-oxazol-2-yl)-3-oxopentanoate according to the procedure described in example 1, C.
$^1$H-NMR ($CDCl_3$) δ 7.69 (s, 0.5 H), 7.67 (s, 0.5 H), 7.57 (d, *J* = 0.8 Hz, 0.5 H), 7.51 (d, *J* = 0.8 Hz, 0.5 H), 7.36-7.18 (m, 3 H), 7.02 (d, *J* = 0.8 Hz, 0.5 H), 6.97 (d, *J* = 0.8 Hz, 0.5 H), 3.90 (s, 1.5 H), 3.64 (s, 1.5 H), 3.40-2.95 (m, 4 H).

**F. Dimethyl 4-(2,6-dichlorophenyl)-2-(2-methoxy-2-oxoethyl)-6-[2-(1,3-oxazol-2-yl)ethyl]-1,4-dihydro-3,5-pyridinedicarboxylate**

The title compound was prepared from mehtyl 3-(2,6-dichlorophenyl)-2-[3-(1,3-oxazol-2-yl)propanoyl]-2-propenoate according to the procedure described in example 1, D.
$^1$H-NMR ($CDCl_3$) δ 8.23 (br.s, 1 H), 7.60 (s, 1 H), 7.23 (d, *J* = 8.0 Hz, 2 H), 7.02 (s, 1 H), 6.98 (t, *J* = 8.0 Hz, 1 H), 5.98 (s, 1 H), 3.84 (d, *J* = 16.5 Hz, 1 H), 3.70 (s, 3 H), 3.55 (s, 3 H), 3.54 (d, *J* = 16.5 Hz, 1 H), 3.51 (s, 3 H), 3.39-3.30 (m, 1 H), 3.14 (t, *J* = 6.4 Hz, 2 H), 3.02-2.92 (m, 1 H).

**G. [4-(2,6-Dichlorophenyl)-3,5-bis(methoxycarbonyl)-6-[2-(1,3-oxazol-2-yl)ethyl]-1,4-dihydro-2-pyridinyl]acetic acid**

The title compound was prepared from dimethyl 4-(2,6-dichlorophenyl)-2-(2-methoxy-2-oxoethyl)-6-[2-(1,3-oxazol-2-yl)ethyl]-1,4-dihydro-3,5-pyridinedicarboxylate according to the procedure described in example 1, E.
$^1$H-NMR ($CDCl_3$) δ 8.26 (br.s, 1 H), 7.58 (d, *J* = 0.8 Hz, 1 H), 7.23 (d, *J* = 6.9 Hz, 2 H), 7.07 (d, *J* = 0.8 Hz, 1 H), 7.00 (t, *J* = 6.9 Hz, 1 H), 6.01 (s, 1 H), 3.82 (d, *J* = 14.5 Hz, 1 H), 3.67 (d, *J* = 14.5 Hz, 1 H), 3.58 (s, 3 H), 3.56 (s, 3 H), 3.46-2.88 (m, 4 H).

**H. Dimethyl 4-(2,6-dichlorophenyl)-2-[2-[4-[8-(*exo*-methyl-8- azabicyclo[3.2.1]oct-3-yl)-1-piperazinyl]-2-oxoethyl]-6-[2-(1,3-oxazol-2-yl)ethyl]-1,4-dihydro-3,5-pyridinedicarboxylate**

The title compound was prepared from [4-(2,6-dichlorophenyl)-3,5-bis(methoxycarbonyl)-6-[2-(1,3-oxazol-2-yl)ethyl]-1,4-dihydro-2-pyridinyl]acetic acid according to the procedure described in example 1, I.
$^1$H-NMR ($CDCl_3$) δ 8.32 (br.s, 1 H), 7.58 (d, *J* = 0.8 Hz, 1 H), 7.23 (d, *J* = 6.9 Hz, 2 H), 7.03 (d, *J* = 0.8 Hz, 1 H), 7.00 (t, *J* = 6.9 Hz, 1 H), 5.98 (s, 1 H), 4.12 (d, *J* = 15.0 Hz, 1 H), 3.77 (d, *J* = 15.0 Hz, 1 H), 3.64-3.59 (m, 4 H), 3.54 (s, 3 H), 3.53 (s, 3 H), 3.31-2.90 (m, 6 H), 2.64-2.44 (m, 5 H), 2.28 (s, 3 H), 2.04-1.96 (m, 2 H), 1.73-1.47 (m, 6 H).

dihydrochloride form

m.p. 225-230 °C (dec.)
IR (KBr) 1680, 1508, 1433, 1296, 1192, 768 $cm^{-1}$.
MS ($FAB^+$) 686 $(M+H)^+$

**Example 19**

**Dimethyl 4-(2,6-dichlorophenyl)-2-(2-([4-(8-ethyl-8-azabicyclo[3.2.1]oct-3-yl)1-piperazinyl]-2-oxoethyl)-6-[2-(1,3-thiazol-2-yl)ethyl]-1,4-dihydro-3,5-pyridinedicarboxylate**

**A. *tert*-Butyl 4-(8-acetyl-8-azabicyclo[3.2.1]oct-3-yl)-1-piperazine carboxylate**

To a solution of *tert*-butyl 4-(8-azabicyclo[3.2.1]oct-3-yl)-1-piperazine carboxylate (900 mg, 3.05 mmol) in $CH_2Cl_2$ (12 ml) was added $Ac_2O$ (0.43 ml, 4.57 mmol) and $Et_3N$ (0.85 ml, 6.09 mmol), succesively. The reaction mixture was stirred for 6 h at room temperature and then the reaction mixture was poured into $CH_2Cl_2$ (25 ml). The whole was washed with $H_2O$ (5 ml) and brine (5 ml). The organic layer was dried ($MgSO_4$), filtered, and concentrated *in vacuo.* The residue was purified by column chromatography (NH gel, $CH_2Cl_2$/MeOH = 100/0 to 100/1) to give 977 mg (95%) of *tert*-butyl 4-(8-acetyl-8-azabicyclo[3.2.1]oct-3-yl)-1-piperazine carboxylate as white solids.
$^1$H NMR ($CDCl_3$) δ 4.74-4.72 (m, 1 H), 4.18-4.16 (m, 1 H), 3.42-3.39 (m, 4 H), 2.85-2.73 (m, 1 H), 2.46-2.43 (m, 4 H), 2.05 (s, 3 H), 2.12-1.40 (m, 8 H), 1.46 (s, 9 H).

**B. 8-Ethyl-3-(1-piperazinyl)-8-azabicyclo[3.2.1]octane**

To a mixture of 8-acetyl-3-(1-piperazinyl)-8-azabicyclo[3.2.1]octane hydrochloride (990 mg, 3.19 mmol), was prepared from *tert*-butyl 4-(8-acetyl-8-azabicyclo[3.2.1]oct-3-yl)-1-piperazine carboxylate by the hydrogenolysis according to the procedure described in example 4 as a pink solid, in THF (30 ml) was added $LiAlH_4$ (363 mg, 9.57 mmol) at 0 °C. The suspension was stirred for 5 min at the same temperature and heated to reflux for 4 h. After cooling to 0 °C, an additional $LiAlH_4$ (180 mg, 4.79 mmol) was added to the reaction mixture and the resultig mixture was refluxed for further 4 h. After cooling to 0 °C, the excess reagent was quenched by the addition of 2 *N* NaOHaq. (7 ml) and the mixture was stirred for 1.5 h at room temperature. After filteration through a pad of celite, the filterate was concentrated *in vacuo.* The residue was purified by column chromatography (NH gel, $CH_2Cl_2$/MeOH = 100/1 to 50/1) to give 460 mg (64%) of 8-ethyl-3-(1-piperazinyl)-8-azabicyclo[3.2.1]octane as a yellow oil.
$^1$H NMR ($CDCl_3$) δ 3.34-3.32 (m, 2 H), 2.89-2.86 (m, 4 H), 2.62-2.44 (m, 6 H), 2.07-1.50 (m, 10 H), 1.07 (t, *J* = 7.3 Hz, 3 H).

**C. Dimethyl 4-(2,6-dichlorophenyl)-2-(2[4-(8-ethyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinecarboxylate**

The titled compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 8-ethyl-3-(1-piperazinyl)-8-azabicyclo[3.2.1]octane according to

the procedure described in example 1,I.
$^1$H NMR ($CDCl_3$) δ 8.35 (s, 1 H), 7.71 (d, *J* = 3.5 Hz, 1 H), 7.26-7.19 (m, 2 H), 7.02-6.97 (m, 1 H), 5.99 (s, 1 H), 4.12 (d, *J* = 15.2 Hz, 1 H), 3.76 (d, *J* = 15.0 Hz, 1 H), 3.64-3.59 (m, 4 H), 3.55 (s, 3 H), 3.54 (s, 3 H), 3.35-3.24 (m, 5 H), 3.06-2.97 (m, 1 H), 2.61-2.46 (m, 7 H), 1.95-1.51 (m, 8 H), 1.08 (t, *J* = 7.1 Hz, 3 H).

dihydrochloride form

$^1$H NMR (DMSO-$d_6$) δ 10.49 (m), 9.39-9.31 (m), 7.71 (d, *J* = 3.3 Hz, 1 H), 7.61 (d, 3.3 Hz, 1 H), 7.36 (d, *J* = 7.9 Hz, 2 H), 7.19-7.13 (m, 1 H), 5.87 (s, 1 H).
IR (KBr) ν: 3392, 2949, 2592, 1691, 1652, 1627, 1508, 1434, 1296, 1230, 1188, 1103, 1047, 966, 767 $cm^{-1}$
MS (ESI) 715.77 $(M+H)^+$
m.p. 185-189 °C

**Example 20**

**Dimethyl 4-(2,6-dichlorophenyl)-2-(2-([4-(8-isopropyl-8-azabicyclo[3.2.1]oct-3- yl)-1-piperazinyl]-2-oxoethyl)-6-[2-(1,3-thiazol-2-yl)ethyl]-1,4-dihydro-3,5-pyridinedicarboxylate**

**A. *tert*-Butyl 4-(8-benzyl-8-azabicyclo[3.2.1]oct-3-yl)-1-piperazine carboxylate**

To a mixture of 8-benzyl-8-azabicyclo[3.2.1]octan-3-one (11.9 g, 858 mmol) and *tert*-butoxycarbonyl piperazine (17.6 g, 943 mmol) was added $Ti(OiPr)_4$ (34 ml, 1.3 mol) at room temperature and the resulting mixure was stirred for 3 h. The mixture was diluted with MeOH (200 ml) and then to this solution was added $NaBH_4$ portionwise at 0 °C. The mixture was allowed to warm to room temperature with stirring for 30 min. The reaction was quenched by the addition of sat. $NaHCO_3$ aq. The resulting mixture was filtered through a pad of celite and the filter cake was washed with $CH_2Cl_2$. The combined whole mixture was partitioned and the organic layer was separated. The organic layer was washed with brine, dried ($MgSO_4$) and concentrated *in vacuo.* The residue was purified by column chromatography ($SiO_2$, $CH_2Cl_2$/MeOH = 10/1) to give 14.9 g (45%) of *tert*-butyl 4-(8-benzyl-8-azabicyclo[3.2.1]oct-3-yl)-1-piperazine carboxylate as a yellow oil.
$^1$H NMR ($CDCl_3$) δ 7.39-7.18 (m, 5 H), 3.57 (s, 2 H), 3.42 (t, *J* = 4.9 Hz, 4 H), 3.24 (br.s, 2 H), 2.55-2.43 (m, 5 H), 2.00 (t, *J* = 4.3 Hz, 2 H),1.75-1.51 (m, 6 H), 1.45 (s, 9 H).

**B. *tert*-Butyl 4-(8-azabicyclo[3.2.1]oct-3-yl)-1-piperazine carboxylate**

The titled compound was prepared from tert-butyl 4-(8-benzyl-8-azabicyclo[3.2.1]oct-3-yl)-1-piperazine carboxylate according to the procedure described in example 1.
$^1$H NMR ($CDCl_3$) δ 3.69 (br.s, 2 H), 3.41 (t, *J* = 4.9 Hz, 4 H), 3.10 (br.s, 1 H), 2.66-2.58 (m, 1 H), 2.46 (t, *J* = 4.9 Hz, 4 H), 1.90-1.48 (m, 8 H), 1.45 (s, 9 H).

**C. *tert*-Butyl 4-(8-isopropyl-8-azabicyclo[3.2.1]oct-3-yl)-1-piperazine carboxylate**

To a solution of *tert*-butyl 4-(8-azabicyclo[3.2.1]oct-3-yl)-1-piperazine carboxylate (900 mg, 3.05 mmol) and acetone (1.1 ml, 15.2 mmol) in MeOH (10 ml) was added 3A MS (576 mg) at room temperature. To this mixture was added $NaBH_3CN$ (403 mg, 6.09 mmol) in small portions and the mixture was heated to reflux for 17 h. After cooled down, the reaction mixture was poured into 2 *N* HCl. The aqueous mixture was washed with EtOAc and then basified with 2 *N* NaOHaq. The aqueous layer was extracted with $CH_2Cl_2$. The combined organic extracts were washed with brine, dried ($MgSO_4$) and concentrated *in vacuo.* The residue was purified by column chromatography (NH gel, $CH_2Cl_2$/MeOH = 100/0 to 200/1) to give 904 mg (88%) of *tert*-butyl 4-(8-isopropyl-8-azabicyclo[3.2.1]oct-3-yl)-1-piperazine carboxylate as white solids.
$^1$H NMR ($CDCl_3$) δ 3.53 (br.s, 2 H), 3.41 (t, *J* = 5.0 Hz, 4 H), 2.93-2.83 (m, 1 H), 2.60-2.43 (m, 5 H), 1.94-1.41 (m, 8 H), 1.46 (s, 9 H), 1.08 (d, *J* = 6.2 Hz, 6 H).

**D. 8-Isopropyl-3-(1-piperazinyl)-8-azabicyclo[3.2.1]octane hydrochloride**

To a solution of *tert*-butyl 4-(8-isopropyl-8-azabicyclo[3.2.1]oct-3-yl)-1-piperazine carboxylate (884 mg, 2.62 mmol) in MeOH (2 ml) was added HCl-MeOH (5 ml) and the mixture was stirred for 1 h at room temperature. To the mixture was added an additional HCl-MeOH (3 ml) and the mixture was stirred for further 17 h.
The volatiles were removed *in vacuo* to give 1.0 g of 8-isopropyl-3-(1-piperazinyl)-8-azabicyclo[3.2.1]octane

hydrochloride was obtained as a pink white solid.
$^1$H NMR (DMSO-$d_6$) δ 10.82-10.66 (m), 9.88 (m), 4.34-3.14 (m), 2.51-1.92 (m), 1.37-1.33 (m, 6H).

**E. Dimethyl 4-(2,6-dichlorophenyl)-2-(2-[4-(8-isopropyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinecarboxylate**

The titled compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 8-isopropyl-3-(1-piperazinyl)-8-azabicyclo[3.2.1]octane according to the procedure described in example 1,I.
$^1$H NMR ($CDCl_3$) δ 8.34 (s, 1 H), 7.72 (d, *J* = 3.3 Hz, 1 H), 7.26-7.20 (m, 2 H), 7.03-6.97 (m, 1 H), 5.99 (s, 1 H), 4.16-3.74 (m, 2 H), 3.75-3.24 (m, 9 H), 3.55 (s, 3 H), 3.54 (s, 3 H), 3.04-2.83 (m, 2 H), 2.57-2.46 (m, 5 H), 2.22-1.44 (m, 8 H), 1.08 (d, *J* = 6.1 Hz, 6 H).

dihydrochloride form

$^1$H NMR (DMSO-$d_6$) δ 9.22 (br.s, 1 H), 8.01 (s, 1 H), 7. 58 (d, *J* = 3.3 Hz, 1 H), 7.48 (d, *J* = 3.3 Hz, 1 H), 7.45-7.21 (m, 2 H), 7.07-7.01 (m, H), 5.74 (s, 1 H).
IR (KBr) ν: 3215, 2949, 1691, 1654, 1508, 1433, 1294, 1230, 1188, 1103, 1053, 767 $cm^{-1}$
MS (ESI) 729.97 $(M+H)^+$
m.p. 194-198 °C

**Example 21**

**Dimethyl 2-[2-[4-(8-acetyl-8-azabicyclo[3.2.1]oct-3-yl-1-piperazinyl]-2-oxoethyl]-4-(2,6-dichlorophenyl)-6-[2-(1,3-thiazol-2-yl)ethyl]-1,4-dihydro-3,5-pyridinedicarboxylate**

**A. *tert*-butyl 4-(8-benzyl-8-azabicyclo[3.2.1]oct-3-yl)-1-piperazinecarboxylate**

To a solution of 526 mg of 8-benzyl-3-(1-piperazinyl)-8-azabicyclo[3.2.1]octane, according to the patent procedure (WO 9606083), and 769 μL of $Et_3N$ in 25 ml of $CH_2Cl_2$ was added 803 mg of $Boc_2O$ dropwise at room temperature under nitrogen atmosphere. The reaction solution was stirred at room temperature for 30 min and quenched with $H_2O$. Extracted organic layer was washed with brine, dried ($MgSO_4$), and concentrated. The residue was purified by column chromatography (NH gel, EtOAc/hexane = 1/5 to 1/3) to give 430.4 mg (61%) of the title compound.

**B. 1-[3-(1-Piperazinyl)-8-azabicyclo[3.2.1]oct-8-yl]-1-ethanone**

The mixture of 430 mg of tert-butyl 4-(8-benzyl-8-azabicyclo[3.2.1]oct-3-yl)-1-piperazinecarboxylate and 400 mg of $Pd(OH)_2$ (20 wt% on carbon) in 25 ml of MeOH was stirred at room temperature for 3 h under hydrogen atmosphere (~4 atm), then filtered through a pad of celite. The filtrate was concentrated under reduced pressure to give crude tert-butyl 4-(8-azabicyclo[3.2.1] oct-3-yl)-1-piperazinecarboxylate. The crude product was then dissolved in 20 ml of $CH_2Cl_2$. To the solution were added 418 μL of $Et_3N$ and 188 μL of $Ac_2O$ at room temperature under nitrogen atmosphere and the reaction mixture was stirred for 30 min. The reaction was quenched with $H_2O$ and the aqueous mixture was extracted with $CH_2Cl_2$. Organic layer was washed with brine, dried ($MgSO_4$), and concentrated *in vacuo.* Residue was purified by column chromatography on silica gel to give crude tert-butyl 4-(8-azabicyclo[3.2.1] oct-3-yl)-1-piperazinecarboxylate. The product was dissolved in 20 ml of EtOAc and 1 ml of conc. HCl was added at room temperature and the solution was stirred at room temperature for 1 h, quenched with 2 *N* NaOHaq. and tried to extract with large amount of $CH_2Cl_2$. The extraction of this compound from aqueous layer was found to be difficult, so $H_2O$ was removed azeotropicallky with toluene. Residue was purified by column chromatography (NH gel, $CH_2Cl_2$/MeOH = 99/1 to 97/3 to 94/6) to give 117.5 mg (45%) of the title compound.
$^1$H NMR ($CDCl_3$); δ 4.73 (m, 1 H), 4.17 (m, 1 H), 2.88 ( m, 4 H), 2.73 (m, 1 H), 2.48 (m, 4 H), 2.05 (s, 1 H), 2.04-1.50 (m, 8 H)

**C. Dimethyl 2-[2-[4-(8-acetyl-8-azabicyclo[3.2.1]oct-3-yl-1-piperazinyl]2-oxoethyl]-4-(2,6-dichlorophenyl)-6-[2-(1,3-thiazol-2-yl)ethyl]-1,4-dihydro-3,5-pyridinedicarboxylate**

The title compound was prepared from [4-(2,6-dichlorophenyl)-3,5-bis(methoxycarbonyl)-6-[2-(1,3-thiazol-2-yl)ethyl]-1,4-dihydro-2-pyridinyl]acetic acid and 1-[3-(1-piperazinyl)-8-azabicyclo[3.2.1]oct-8-yl]-1-ethanone according to the procedure described in example 1, I.

$^1H$ NMR ($CDCl_3$) δ 8.31 (d, *J* = 2.8 Hz, 1 H), 7.72 (d, *J* = 3Hz, 1 H), 7.28-7.20 (m, 3 H), 7.00 (dt, *J* = 2.9, 7Hz, 1 H), 5.99 (s, 1 H), 4.71 (bs, 1 H), 4.20-4.10 (m, 2 H), 3.80-3.25 (m, 12 H), 3.01 (m, 1 H), 2.79 (m, 1 H), 2.47 (m, 4 H), 2.08-1.50 (m, 13 H)

monohydrochloride form

IR (KBr) ν: 3402, 3220, 3095, 2949, 2889, 2597, 2457, 1693, 1622, 1560, 1510, 1433, 1296, 1236, 1190, 1103, 1035, 954, 873, 842, 767 $cm^{-1}$
MS (ESI) 730.30 $(M+H)^+$
m.p. 175-179 °C

**Example 22**

**Dimethyl 4-(2,6-dichlorophenyl)-2-(2-([4-(8-formyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinedicarboxylate**

**A. *tert*-Butyl 4-(8-formyl-8-azabicyclo[3.2.1]oct-3-yl)-1-piperazine carboxylate**

To a solution of *tert*-butyl 4-(8-azabicyclo[3.2.1]oct-3-yl)-1-piperazine carboxylate (1.03 g, 3.50 mmol) in $CH_2Cl_2$ (6 ml) was added WSC (1.10 g, 5.25 mmol) and HCOOH (0.20 ml,5.25 mmol). The mixture was stirred for 17 h at room temperature. The mixture was partitioned between $H_2O$ and $CH_2Cl_2$ and the organic phase was separated. The aqueous phase was extracted with $CH_2Cl_2$. The combined organic layer was washed with $H_2O$, dried ($MgSO_4$) and concentrated *in vacuo.* The residue was purified by column chromatography (NH gel, $CH_2Cl_2$/MeOH = 200/1 to 100/1) to give 852 mg (86%) of *tert*-butyl 4-(8-formyl-8-azabicyclo[3.2.1]oct-3-yl)-1-piperazine carboxylate as white solids.
$^1H$ NMR ($CDCl_3$) δ 8.09 (s, 1 H), 4.66 (br.s, 1 H), 4.12 (br.s, 1 H), 3.40 (t, *J* = 5.0 Hz, 4 H), 2.88-2.76 (m, 1 H), 2.44 (t, *J* = 4.9 Hz, 4 H), 1.92-1.50 (m, 8 H), 1.45 (s, 9 H).

**B. 8-Formyl-3-(1-piperazinyl)-8-azabicyclo[3.2.1]octane hydrochloride**

The titled compound was prepared from *tert*-butyl 4-(8-formyl-8-azabicyclo[3.2.1]oct-3-yl)-1-piperazine carboxylate according to the procedure described in example 21,B.
$^1H$ NMR (DMSO-$d_6$) δ 9.93 (br.s), 9.38 (s), 4.12-3.17 (m), 2.51 (s), 2.25-2.16 (m), 1.94-1.90 (m).

**C. Dimethyl 4-(2,6-dichlorophenyl)-2-(2-[4-(8-formyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinecarboxylate**

8-Formyl-3-(1-piperazinyl)-8-azabicyclo[3.2.1]octane hydrochloride was dissolved with $CH_2Cl_2$ and the solution was washed with sat. $NaHCO_3$ aq. The organic phase was dried and the solvent was removed *in vacuo.* The residue was purified with columm chromatography (NH gel, MeOH) to give 363 mg (81%) of 8-formyl-3-(1-piperazinyl)-8-azabicyclo[3.2.1]octane as white solids. The titled compound was prepared from 2-[4-(2,6-dichlorophenyl)-3,5-bis (methoxycarbonyl)-6-[2-(1,3-thiazol-2-yl)ethyl]-1,4-dihydro-2-pyridinyl)acetic acid and 8-formyl-3-(1-piperazinyl)-8-azabicyclo[3.2.1]octane according to the procedure described in example 1.
$^1H$ NMR ($CDCl_3$) δ 8.35 (s, 1 H), 8.08 (s, 1 H), 7.71 (d, *J* = 3.3 Hz, 1 H), 7.30-7.20 (m, 3 H), 7.00 (dt, *J* = 2.8, 8.0 Hz, 1 H), 6.00 (s, 1 H), 4.66 (br.s, 1 H), 4.14-4.07 (m, 2 H), 3.80-3.72 (m, 1 H), 3.60-3.48 (m, 10 H), 3.37-3.28 (m, 3 H), 3.08-2.96 (m, 1 H), 2.84-2.78 (m, 1 H), 2.48 (m, 4 H), 1.93-1.51 (m, 8 H).

**D. Dimethyl 4-(2,6-dichlorophenyl)-2-(2-[4-(8-formyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinecarboxylate**

The titled compound was prepared from dimethyl 4-(2,6-dichlorophenyl)-2-(2-[4-(8-formyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinecarboxylate according to the procedure described in example 1, I.

monohydrochloride form

$^1H$ NMR (DMSO-$d_6$) δ 11.31-11.18 (m), 9.50-9.33 (m), 8.08 (s, 1 H), 7.75 (d, *J* = 3.3 Hz, 1 H), 7.65 (d, 3.30 Hz, 1 H), 7.36 (d, *J* = 8.1 Hz, 2 H), 7.19-7.13 (m, 1 H), 5.87 (s, 1H).

IR (KBr) ν: 3394, 3087, 2949, 2528, 1693, 1654, 1564, 1512, 1433, 1294, 1232, 1190, 1103, 1047, 767 $cm^{-1}$
MS (ESI) 716.21 $(M+H)^+$
m.p. 174-180 °C

**Example 23**

**Dimethyl 2-[2-[4-[(3S)-1-azabicyclo[2.2.2]oct-3-yl]piperazinyl]-2-oxoethyl]-4-(2,6-dichlorophenyl)-6-[2-(1,3-thiazol-2-yl)ethyl]-1,4-dihydro-3,5-pyridinedicarboxylate**

**A. 1-[(3S)-1-azabicyclo[2.2.2]oct-3-yl]-4-benzyl-2,6-piperazinedione**

To a solution of *N*-benzyliminodiacetic acid (2.23 g) in THF (30 ml) was added 1,1'-carbonylbis-1*H*-imidazole (3.57 g) at room temperature under nitrogen atmosphere. The resulting mixture was stirred at reflux temperature for 30 min (until the evolution of $CO_2$ gas ceased, giving a clear solution), then cooled to room temperature. To the resulting mixture was added a solution of (3S)-3-aminoquinuclidine dihydrochloride (2.00 g) and triethylamine (3.06 ml) in THF (10 ml) stirred at room temperature under nitrogen atmosphere for 30 min via a cannula. The combined reaction mixture was stirred at reflux for 24 h, then cooled to room temperature and quenched with $H_2O$. Organic layer was extracted with EtOAc and the combined organic layer was dried ($MgSO_4$). After the filtration, the filtrate was concentrated *in vacuo.* The residue was purified by column chromatography (NH gel, EtOAc) to give 2.17 g (69%) of imide as white solid.
$^1H$ NMR ($CDCl_3$) δ: 7.39-7.27 (m, 5 H), 4.73-4.66 (m, 1 H), 3.77-3.69 (m, 1 H), 3.60 (s, 2 H), 3.38 (s, 4 H), 3.34-3.29 (m, 1 H), 3.02-2.93 (m, 1 H), 2.90-2.75 (m, 3 H), 1.91-1.61 (m,4H), 1.38-1.28(m, 1 H)

**B. (3S)-3-(4-benzyl-1-pipeazinyl)-1-azabicyclo[2.2.2]octane**

To a solution of 1-[(3S)-1-azabicyclo[2.2.2]oct-3-yl]-4-benzyl-2,6-piperazinedione (1.9 g, 6.0 mmol) in 1,4-dioxane (40 ml) was added $LiAlH_4$ (911 mg, 24 mmol) at room temperature under nitrogen atmosphere. The resulting suspension was stirred at reflux temperature for 3.5 h, then cooled to 0 °C. The mixture was diluted with $Et_2O$ (80 ml), then treated carefully with $Na_2SO_4 \cdot 10\ H_2O$ (9.1 g) and anhydrous KF (1 g). After the resulting white suspension was stirred vigorously at room temperature for 30min, the white precipitate was removed by filtration through a pad of celite. The filtrate was evaporated and the residue was purified by column chromatography on silica gel (NH gel, EtOAc) to give 1.39 g (81%) of amine as white solids.
$^1H$ NMR ($CDCl_3$) δ: 7.32-7.24 (m, 5 H), 3.51 (s, 2 H), 3.01-1.98 (m, 16 H), 1.83-1.59 (m, 2 H), 1.48-1.40 (m, 1 H), 1.30-1.21(m, 1 H)

**C. (3S)-3-(1-piperazinyl)-1-azabicyclo[2.2.2]octane**

A mixture of (3,S)-3-(4-benzyl-1-pipeazinyl)-1-azabicyclo[2.2.2]octane (1.25 g, 4.37 mmol) and 400 mg of Pd $(OH)_2$ (20 wt% on carbon) in MeOH (60 ml) was stirred at room temperature under hydrogen atmosphere (4 $kg/cm^2$) for 6 h. The reaction mixture was filtered through a pad celite and the filtrate was concentrated *in vacuo* to give 850.2 mg (4.35 mmol; quant.) of the deprotected amine.
$^1H$ NMR ($CDCl_3$) δ: 3.01-2.00 (m, 16 H), 1.81-1.65 (m, 2 H), 1.50-1.36 (m, 1 H), 1.36-1.20 (m, 1 H)

**D. Dimethyl 2-[2-[4-[[3S)-1-azabicyclo[2.2.2]oct-3-yl]piperazinyl]-2-oxoethyl]-4-(2,6-dichlorophenyl)-6-[2-(1,3-thiazol-2-yl)ethyl]-1,4-dibydro-3,5-pyridinedicarboxylate**

The title compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and (3S)-3-(1-piperazinyl)-1-azabicyclo[2.2.2]octane according to the procedure described in example 1, I.
$^1H$ NMR ($CDCl_3$) δ 8.36 (s, 1 H), 7.71 (d, *J* = 3.5 Hz, 1 H), 7.29-7.19 (m,3 H), 7.00 (t, *J* = 7.0 Hz, 1 H), 5.99 (s, 1 H), 4.10-4.04 (m, 1 H), 3.87-3.78 (m, 1 H), 3.76-3.50 (m, 4 H), 3.55 (s, 3 H), 3.53 (s, 3 H), 3.38-3.10 (m, 4 H), 3.03- 2.60 (m, 7 H), 2.59-2.30 (m, 4 H), 2.03-1.93 (m, 1 H), 1.85-1.60 (m, 2 H), 1.49-1.31 (m, 1 H), 1.30-1.23 (m, 1 H)

dihydrochloride form

$^1H$ NMR (DMSO-$d_6$) δ 10.79 (br.s, 1 H), 7.71 (d, *J* = 3.3 Hz, 1 H), 7.61 (d, *J* = 3.3 Hz, 1 H), 7.35 (d, *J* = 7.8 Hz, 2 H), 7.16 (t, *J* = 7.8 Hz, 1 H), 5.87 (s, 1 H), 3.95-1.65 (m, 32 H).
m.p. 190-195 °C (dec.)

**Example 24**

**Dimethyl 2-[2-[4-[(3*R*-1-azabicyclo[2.2.2]oct-3-yl]piperazinyl]-2-oxoethyl]-4-(2,6-dichlorophenyl)-6-[2-(1,3-thiazol-2-yl)ethyl]-1,4-dihydro-3,5-pyridinedicarboxylate**

The title compound was prepared according to the procedure of example 23, using (3*R*)-3-aminoquinuclidine dihydrochloride instead of (3*S*)-3-aminoquinuclidine dihydrochloride.
$^1$H NMR ($CDCl_3$) δ: 8.33 (s, 1 H), 7.72 (d, *J* = 3 Hz, 1 H), 7.26 (m, 3 H), 6.99 (t, *J* = 7 Hz, 1 H), 5.99 (s, 1 H), 4.13-4.06 (m, 1 H), 3.84-3.75 (m, 1 H), 3.65 (m, 4 H), 3.55 (s, 3 H), 3.53 (s, 3 H), 3.35-3.33 (m, 3 H), 3.01- 2.73 (m, 8 H), 2.41-2.30 (m, 4 H), 2.03-1.93 (m, 1 H), 1.82-1.60 (m, 2 H), 1.49-1.31 (m, 1 H), 1.30-1.23 (m, 1 H)

dihydrochloride form

IR (KBr) ν: 3413, 2949, 2588, 1691, 1624, 1508, 1433, 1296, 1234, 1190, 1105, 1055, 767 $cm^{-1}$
m.p. 172-175 °C (dec.)

**Example 25**

**(-)-Dimethyl 2-[2-[4-[(3*S*)-1-azabicyclo[2,2,2]oct-3-yl]piperazino]-2-oxoethyl]-4-(2,6-dichlorophenyl)-6-[2-(1,3-thiazol-2-yl)ethyl]-1,4-dihydro-3,5-pyridinedicarboxylate**

The title compound was separated by the optical resolution of racemic dimethyl 2-[2-[4-[(3*S*)-1-azabicyclo [2.2.2]oct-3-yl]piperazinyl]-2-oxoethyl]-4-(2,6-dichlorophenyl)-6-[2-(1,3-thiazol-2-yl)ethyl]-1,4-dihydro-3,5-pyridinedicarboxylate with chiral HPLC (Daicel chiralpak AD).
$^1$H NMR ($CDCl_3$) δ 8.33 (s, 1 H), 7.72 (d, *J* = 3.3 Hz, 1 H), 7.26-7.19 (m,3 H), 6.99 (t, *J* = 7.0 Hz, 1 H), 5.99 (s, 1 H), 4.13-4.06 (m, 1 H), 3.84-3.75 (m, 1 H) , 3.65 (m, 4 H), 3.55 (s, 3 H), 3.53 (s, 3 H), 3.35-3.33 (m, 3 H), 3.01- 2.73 (m, 8 H), 2.41-2.30 (m, 4 H), 2.03-1.93 (m, 1 H), 1.82-1.60 (m, 2 H), 1.49-1.31 (m, 1 H), 1.30-1.23 (m, 1 H)

dihydrochloride form

m.p. 173-176 °C (dec.)

**Example 26**

**Dimethyl 4-(2,6-dichlorophenyl)-2-(2-([4-(2-methyloctahydrocyclopenta[c]pyrrol-5-yl)-1-piperazinyl]-2-oxoethyl)-6-[2-(1,3-thiazol-2-yl)ethyl]-1,4-dihydro-3,5-pyridinedicarboxylate**

**A. 5-(4-Benzyl-1-piperazinyl)-2-methyloctahydrocyclopenta[*c*]pyrrole**

To a solution of 5-(4-benzyl-1-piperazinyl)octahydrocyclopenta[*c*]pyrrole (947 mg, 3.32 mmol) in $HCO_2H$ (10 ml) was added $CH_2O$ (0.55 ml, 6.64 mmol) at room temperature and the mixture was refluxed for 1.5 h. After cooling down, the mixture was basified with 2 N NaOHaq. and extracted with $CH_2Cl_2$. The organic layer was dried ($MgSO_4$) and concentrated *in vacuo* to give 861 mg of 5-(4-benzyl-1-piperazinyl)-2-methyloctahydrocyclopenta[c]pyrrole (87%) as white solids.
$^1$H NMR ($CDCl_3$) δ 7.31-7.20 (m, 5 H), 3.50 (s, 2 H), 2.79-2.02 (m, 20 H), 1.36-1.24 (m, 2 H).

**B. 5-(1-Piperazinyl)-2-methyloctahydrocyclopenta[*c*]pyrrole**

The titled compound was prepared from 5-(4-benzyl-1-piperazinyl)-2-methyloctahydrocyclopenta[*c*]pyrrole by the hydrogenolysis according to the procedure described in example 1, H.
$^1$H NMR ($CDCl_3$) δ 2.90-2.86 (m, 4 H), 2.50-2.40 (m, 10 H), 2.29-2.27 (m, 3 H), 2.16-196 (m, 4 H), 1.35-1.24 (m, 2 H).

**C. Dimethyl 4-(2,6-dichlorophenyl)-2-(2-([4-(2-methyloctahydrocyclopenta[c]pyrrol-5-yl)-1-piperazinyl]-2-oxoethyl)-6-[2-(1,3-thiazol-2-yl)ethyl]-1,4-dihydro-3,5-pyridinedicarboxylate**

The titled compound was prepared from 2-[4-(2,6-dichlorophenyl)-3,5-bis(methoxycarbonyl)-6-[2-(1,3-thiazol-2-yl)ethyl]-1,4-dihydro-2-pyridinyl)acetic acid and 5-(1-piperazinyl)-2-methyloctahydrocyclopenta[*c*]pyrrole according to the procedure described in example 1, I.

$^1H$ NMR ($CDCl_3$) δ 8.35 (s, 1 H), 7.71 (d, *J* = 3.3 Hz, 1 H), 7.26-7.19 (m, 3 H), 7.02-6.96 (m, 1 H), 5.99 (s, 1 H), 4.10 (d, *J* = 15.0 Hz, 1 H), 3.79 (d, *J* = 15.0 Hz, 1 H), 3.59-3.53 (m, 11 H), 3.37-3.24 (m, 3 H), 3.09-2.97 (m, 1 H), 2.47-2.27 (m, 14 H), 2.17-2.07 (m, 2 H), 1.34-1.23 (m, 2 H).

dihydrochloride form

$^1H$ NMR (DMSO-$d_6$) δ 11.01 (br.s, 1 H), 9.41-9.24 (m, 1 H), 7.59-7.58 (m, 1 H), 7.49-7.47 (m, 1 H), 7.40-7.22 (m, 2 H), 7.06-7.01 (m, 1 H), 5.74 (s, 1 H).
IR (KBr) ν: 3394, 3215, 2949, 1691, 1654, 1629, 1508, 1433, 1294, 1230, 1188, 1103, 759 $cm^{-1}$
MS (ESI) 702.22 $(M+H)^+$
m.p. 178-185 °C

**Example 27**

**Dimethyl 4-(2,6-dichlorophenyl)-2-(2-([4-(2-acetyloctahydrocyclopenta[c]pyrrol-5-yl)-1-piperazinyl]-2-oxoethyl)-6-[2-(1,3-thiazol-2-yl)ethyl]-1,4-dihydro-3,5-pyridinedicarboxylate**

**A. *tert*-Butyl 5-(4-benzyl-1-piperazinyl)hexahydrocyclopenta[*c*]pyrrole-2(1*H*)carboxylate**

To a solution of *tert*-butyl 5-oxohexahydrocyclopenta[c]pyrrole-2(1*H*)carboxylate (*Tetrahedron,* **1993**, 49, 5047*,* 1.86 g, 8.26 mmol) and benzylpiperazine (1.45 g, 8.26 mmol) in MeOH (10 ml) was added $Ti(OiPr)_4$ (3.15 g, 12.4 mmol) at room temperature and the resulting mixture was stirred for 3.5 h. The mixture was diluted with MeOH (20 ml). To the mixture was added $NaBH_4$ (468 mg, 12.4 mmol) in small portions at room temperature and the mixture was stirred for 30 min. The excess reagent was quenched by the addition of sat. $NaHCO_3$ aq. The resulting aqueous mixture was filtered through a pad of celite and the filter cake was washed with $CH_2Cl_2$. The combined whole mixture was partitioned and the organic layer was separated. The organic layer was washed with brine, dried ($MgSO_4$) and concentrated *in* vacuo to give tert-butyl 5-(4-benzyl-1-piperazinyl)hexahydrocyclopenta[*c*]pyrrole-2(1*H*)-carboxylate. This was dissolved with trifluoroacetic acid (10 ml) and the solution was stirred for 1 h at room temperature. The mixture was partitioned between 2 NNaOHaq. and $CH_2Cl_2$. The organic layer was washed with brine, dried ($MgSO_4$) and concentrated *in vacuo.* The residue was purified by column chromatography (NH gel, $CH_2Cl_2$/MeOH = 100/1 to 40/1) to give 645 mg (27%) of 5-(4-benzyl-1-piperazinyl)octahydrocyclopenta[c]pyrrole as white solids.
$^1H$ NMR ($CDCl_3$) δ 7.31-7.24 (m, 5 H), 3.50(s, 2 H), 2.86-2.33 (m, 16 H), 2.31-2.16 (m, 2 H), 1.09-0.97 (m, 2 H).

**B. 1-(5-(4-Benzyl-1-piperazinyl)hexahydrocyclopenta[*c*]pyrrol-2(1*H*)-yl]-1-ethanone**

To a solution of 5-(4-benzyl-1-piperazinyl)octahydrocyclopenta[c]pyrrole (207 mg, 0.73 mmol) in $CH_2Cl_2$ (3 ml) was added $Et_3N$ (0.2 ml, 1.50 mmol). To this mixture was added $Ac_2O$ (0.1 ml, 1.09 mmol) at room temperature and the reaction mixture was stirred overnight. After diluted with $CH_2Cl_2$ (25 ml), the mixture was washed with $H_2O$ (3 ml) and brine (2 ml). The organic layer was dried ($MgSO_4$) and concentrated *in vacuo.* The residue was purified by column chromatography ($SiO_2$, $CH_2Cl_2$/MeOH = 10/1 to 8/1) to give 231 mg (97%) of 1-[5-(4-benzyl-1-piperazinyl) hexahydrocyclopenta[*c*]pyrrol-2(1*H*)-yl]-1-ethanone as white solids.
$^1H$ NMR ($CDCl_3$) δ 7.31-7.24 (m, 5 H), 3.63-3.46 (m, 2 H), 3.51 (s, 2 H), 3.32 (dd, *J* = 4.3, 10.7 Hz, 1 H), 2.73-2.50 (m, 10 H), 2.04 (s, 2 H), 2.22-2.09 (m, 2 H), 2.02 (s, 3 H), 1.42-01.31 (m, 2 H).

**C. 5-(1-Piperazinyl)hexabydrocyclopenta[*c*]pyrrol-2(1*H*)-yl-1-ethanone**

To a solution of 1-[5-(4-benzyl-1-piperazinyl)hexahydrocyclopenta[*c*]pyrrol-2(1*H*)-yl]-1-ethanone (226 mg, 0.69 mmol) in MeOH (15 ml) was added 100 mg of $Pd(OH)_2$ (20 wt% on carbon) and the mixture was stirred for 30 h under $H_2$ (1 atm) atmosphere at room temperature. After the reaction mixture was filtered through a pad of celite, the filterate was concentrated *in vacuo.* The residue was purified by column chromatography (NH gel, $CH_2Cl_2$/MeOH = 15/1 to 10/1) to give 142 mg (87%) of 5-(1-piperazinyl)hexahydrocyclopenta[*c*]pyrrol-2(1*H*)-yl-1-ethanone as a colorless oil.
$^1H$ NMR ($CDCl_3$) δ 3.65-3.47 (m, 4 H), 3.33 (dd, *J* = 4.3 Hz*,* 1 H), 2.90 (t, *J* = 4.8 Hz, 3 H), 2.77-2.45 (m, 6 H), 2.28-1.98 (m, 4H), 2.03 (s, 3 H), 1.45-1.26 (m, 2 H).

**D. Dimethyl 4-(2,6-dichlorophenyl)-2-(2-([4-2-acetyloctahydrocyclopenta[*c*]pyrrol-5-yl)-1-piperazinyl]-2-oxoethyl)-6-[2-(1,3-thiazol-2-yl)ethyl]-1,4-dihydro-3,5-pyridinedicarboxylate**

The titled compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 5-(1-piperazinyl)hexahydrocyclopenta[*c*]pyrrol-2(1*H*)-yl-1-ethanone according to the procedure described in example 1, I.
$^1$H NMR ($CDCl_3$) δ 8.31 (br.s, 1 H), 7.72 (d, *J* = 3.3 Hz, 1 H), 7.27-7.20 (m, 2 H), 7.00 (t, *J* = 8.0 Hz, 1 H), 5.99 (s, 1 H), 4.13 (d, *J* = 15.0, 1 H), 3.78 (d, *J* = 15.0 Hz, 1 H), 3.65-3.47 (m, 8 H), 3.55 (s, 3 H), 3.54 (s, 3 H), 3.37-3.27 (m, 4 H), 3.04-2.97 (m, 1 H), 2.71-2.47 (m, 7 H), 2.19-2.12 (m, 2 H), 2.03 (s, 3 H), 1.39-1.25 (m, 2 H).

monohydrochloride form

$^1$H NMR (DMSO-$d_6$) δ 11.83 (br.s, 1 H), 9.62 (m, 1 H), 9.38 (m, 1 H), 7.81 (s, 1 H), 7.69 (s, 1 H), 7.37-7.34 (m, 2 H), 7.19-7.13 (m, 1 H), 6.56 (s, 1 H), 5.87 (s, 1 H).
IR (KBr) ν: 3408, 3097, 2950, 2534, 1693, 1624, 1508, 1433, 1294, 1188, 1103, 1053, 767 $cm^{-1}$
MS (ESI) 729.91 ($M^+$)
m.p. 160-165 °C

**Example 28**

**Dimethyl 4-(2,6-dichlorophenyl)-2-(2-([4-(2- formyloctahydrocyclopenta[c]pyrrol-5-yl)-1-piperazinyl]-2-oxoethyl)-6-[2-(1,3-thiazol-2-yl)ethyl]-1,4-dihydro-3,5-pyridinedicarboxylate**

**A. 5-(4-Benzyl-1-piperazinyl)-2-hexahydrocyclopenta[*c*]pyrrol-1(1H)carbaldehyde**

To a solution of 5-(4-benzyl-1-piperazinyl)octahydrocyclopenta[*c*]pyrrole (1.0 g, 3.50 mmol) in $CH_2Cl_2$ (10 ml) was added WSC (1.10 g, 5.26 mmol) at room temperature in small portions. Then to the mixture was added HCO2H (0.2 ml, 5.25 mmol) via pipet and the reaction mixture was stirred for 2.5 h at room temperature. The reaction mixture was partitioned between $H_2O$ and $CH_2Cl_2$. The organic phase was separated and the aqueous phase was saturated with NaCl and extracted with $CH_2Cl_2$. The combined organic layer was dried ($MgSO_4$) and concentrated *in vacuo.* The residue was purified by column chromatography (NH gel, $CH_2Cl_2$/MeOH = 100/0 to 100/1) to give 881 mg (80%) of 5-(4-benzyl-1-piperazinyl)-2-hexahydrocyclopenta[*c*]pyrrol-2(1H)-carbaldehyde as a white solid.
$^1$H NMR ($CDCl_3$) δ 8.15 (s, 1 H), 7.31-7.22 (m, 5 H), 3.60-3.31 (m, 6 H), 2.66-2.18 (m, 13 H), 1.32-1.20 (m, 2 H).

**B. 5-(1-Piperazinyl)-2-hexahydrocyclopenta[*c*]pyrrol-2(1H)-carbaldehyde**

The titled compound was prepared from 5-(4-benzyl-1-piperazinyl)-2-hexahydrocyclopenta[*c*]pyrrol-2(1H)-carbaldehyde according to the procedure described in example 1, H.
$^1$H NMR ($CDCl_3$) δ 8.16 (s, 1 H), 3.62-3.34 (m, 4 H), 2.92-2.20 (m, 13 H), 1.88 (br.s, 1 H), 1.32-1.21 (m, 2 H).

**C. Dimethyl 4-(2,6-dichlorophenyl)-2-(2-([4-(2-methyloctahydrocyclopenta[c]pyrrol-5-yl)-1-piperazinyl]-2-oxoethyl)-6-[2-(1,3-thiazol-2-yl)ethyl]-1,4-dihydro-3,5-pyridinedicarboxylate**

The titled compound was prepared from 2-[4-(2,6-dichlorophenyl)-3,5-bis(methoxycarbonyl)-6-[2-(1,3-thiazol-2-yl)ethyl]-1,4-dihydro-2-pyridinyl]acetic acid and 5-(1-piperazinyl)-2-hexahydrocyclopenta[*c*]pyrrol-2(1H)-carbaldehyde according to the procedure described in example 1, I.
$^1$H NMR ($CDCl_3$) δ 8.31 (s, 1 H), 8.17 (s, 1 H), 7.72 (d, *J* = 3.46 Hz, 1 H), 7.27-7.19 (m, 3 H), 7.03-6.97 (m, 1 H), 5.99 (s, 1 H), 4.12 (d, *J* = 15.0 Hz, 1 H), 3.77 (d, *J* = 15.0 Hz, 1 H), 3.63-3.25 (m, 11 H), 3.08-2.97 (m, 1 H), 2.64-2.42 (m, 8 H), 2.21-2.19 (m, 2 H), 1.3-1.21 (m, 2 H).

monohydrochloride form

$^1$H NMR (DMSO-$d_6$) δ 9.22 (br.s, 1 H), 8.01 (s, 1 H), 7. 58 (d, *J* = 3.3 Hz, 1 H), 7.48 (d, *J* = 3.3 Hz, 1 H), 7.45-7.21 (m, 2 H), 7.07-7.01 (m, 1 H), 5.74 (s, 1 H).
IR (KBr) ν: 3215, 2949, 1691, 1654, 1508, 1433, 1294, 1230, 1188, 1103, 1053, 767 $cm^{-1}$
MS (ESI) 716.01 $(M+H)^+$
m.p. 175-180 °C

**Example 29**

**(-)-Dimethyl 4-(2,6-dichlorophenyl)-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridine-3,5-dicarboxylate**

The NMR spectrum of the free base was identical to that of the racemic one.

monohydrochloride form

$[\alpha]^D$ = -33.8 (c 0.32, MeOH, 27 °C)
m.p. 212-220 °C (dec.)
IR (KBr); ν 2949, 2883, 1693, 1627, 1512, 1433, 1292, 1190, 1103 $cm^{-1}$.
MS (ESI) 717.27 $(M+H)^+$

The chemical structures of the compounds prepared in the Examples I to 29 are summarized in the following tables.

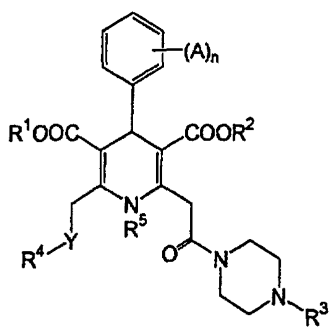

(wherein $(A)_n$ is 2,6-dichloro; $R^1$ and $R^2$ except Example 16 are methyl, $R^2$ of Example 16 is ethyl; $R^5$ is hydrogen; and Y is $-(CH_2)-$.)

TABLE

| Ex. # | $R^4$ | $R^3$ |
|---|---|---|
| 1 | 1,3-thiazol-2-yl | 8-methylbicyclo[3.2.1]oct-3-yl |
| 2 | 1,3-thiazol-2-yl | 8,8-ethylenedioxybicyclo[3.2.1]oct-3-yl |
| 3 | 1,3-thiazol-2-yl | 8-oxobicyclo[3.2.1]oct-3-yl |
| 4 | 1,3-thiazol-2-yl | 8-hydroxybicyclo[3.2.1]oct-3-yl |
| 5 | 1,3-thiazol-2-yl | *exo*-8-hydroxy-8-methylbicyclo[3.2.1]oct-3-yl |
| 6 | 1,3-thiazol-2-yl | 8-hydroxy-8-butylbicyclo[3.2.1]oct-3-yl |
| 7 | 1,3-thiazol-2-yl | 8-hydroxy-8-ipropylbicyclo[3.2.1]oct-3-yl |
| 8 | 1,3-thiazol-2-yl | 8-methoxybicyclo[3.2.1]oct-3-yl |
| 9 | 1,3-thiazol-2-yl | bicyclo[2.2.2]oct-2-yl |
| 10 | 1,3-thiazol-2-yl | 5-oxooctahydro-2-pentalenyl |
| 11 | 1,3-thiazol-2-yl | 1-methyl-3-piperidinyl |
| 12 | 1,3-thiazol-2-yl | 1-benzyl-3-piperidinyl |
| 13 | 1,3-thiazol-2-yl | *exo*-8-methyl-8-azabicyclo[3.2.1]oct-3-yl |
| 14 | (-)-isomer of Example 13 | |
| 15 | 1,3-thiazol-2-yl | *endo*-8-methyl-8-azabicyclo[3.2.1]oct-3-yl |
| 16 | 1,3-thiazol-2-yl | *exo*-8-methyl-8-azabicyclo[3.2.1]oct-3-yl |
| 17 | 1-methyl-1*H*-imidazol-2-yl | *exo*-8-methyl-8-azabicyclo[3.2.1]oct-3-yl |
| 18 | 1,3-oxazol-2-yl | *exo*-8-methyl-8-azabicyclo[3.2.1]oct-3-yl |
| 19 | 1,3-thiazol-2-yl | 8-ethyl-8-azabicyclo[3.2.1]oct-3-yl |
| 20 | 1,3-thiazol-2-yl | 8-$^i$propyl-8-azabicyclo[3.2.1]oct-3-yl |
| 21 | 1,3-thiazol-2-yl | 8-acetyl-8-azabicyclo[3.2.1]oct-3-yl |

TABLE (continued)

| Ex. # | $R^4$ | $R^3$ |
|---|---|---|
| 22 | 1,3-thiazol-2-yl | 8-formyl-8-azabicyclo[3.2.1]oct-3-yl |
| 23 | 1,3-thiazol-2-yl | (3*S*)-1-azabicyclo[2.2.2]oct-3-yl |
| 24 | 1,3-thiazol-2-yl | (3R)-1-azabicyclo[2.2.2]oct-3-yl |
| 25 | (-)-isomer of Example 23 | |
| 26 | 1,3-thiazol-2-yl | 2-methyloctahydrocyclopenta[*c*]pyrrol-5-yl |
| 27 | 1,3-thiazol-2-yl | 2-acetyloctahydrocyclopenta[*c*]pyrrol-5-yl |
| 28 | 1,3-thiazol-2-yl | 2-formyloctahydrocyclopenta[*c*]pyrrol-5-yl |
| 29 | (-)-isomer of Example 5 | |

## Claims

**1.** A compound of the formula (I):

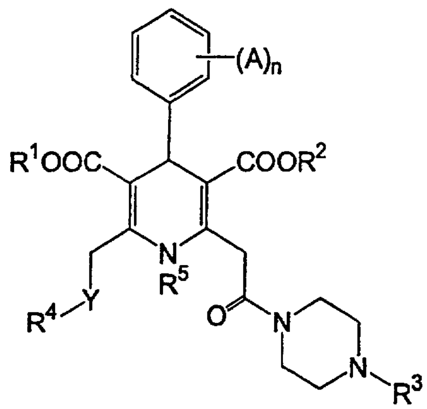

(I)

or the pharmaceutically acceptable salts thereof wherein
**A** is independently halo;
**Y** is $-(CH_2)_m-$, -C(O)- or -S(O)-;
$\mathbf{R^1}$ and $\mathbf{R^2}$ are independently $C_{1-4}$ alkyl;
$\mathbf{R^3}$ is $C_{7-9}$ bicycloalkyl, $C_{5-7}$ azacycloalkyl or $C_{7-9}$ azabicycloalkyl, the $C_{7-9}$ bicycloalkyl, $C_{5-7}$ azacycloalkyl or $C_{7-9}$ azabicycloalkyl being optionally substituted with one, two or three substituents independently selected from oxo, hydroxyl, $C_{1-4}$ alkyl, $C_{1-4}$ alkyloxy, $C_{1-4}$ alkyl-carbonyl, formyl, $C_{1-4}$ alkylenedioxy and phenyl-$C_{1-4}$ alkyl;
$\mathbf{R^4}$ is thiazolyl, imidazolyl or oxazolyl, the thiazolyl, imidazolyl or oxazolyl being optionally substituted with one or two substituents independently selected from $C_{1-4}$ alkyl and halo;
$\mathbf{R^5}$ is hydrogen or $C_{1-4}$ alkyl;
**m** is 0, 1 or 2; and
**n** is 0, 1, 2, 3, 4 or 5.

**2.** A compound according to Claim 1, wherein
$\mathbf{(A)_n}$ is 2,6-dichloro;
**Y** is $-CH_2-$
$\mathbf{R^4}$ is 1,3-thiazol-2-yl, 1*H*-imidazol-2-yl, 1-methyl-1*H*-imidazol-2-yl, 1-ethyl-1*H*-imidazol-2-yl or 1,3-oxazol-2-yl; and
$\mathbf{R^5}$ is hydrogen.

**3.** A compound according to Claim 2, wherein
$\mathbf{R^1}$ and $\mathbf{R^2}$ are independently methyl or ethyl;
$\mathbf{R^3}$ is bicyclo[3.2.1]octyl, bicyclo[2.2.2]octyl, octahydropentalenyl, piperidinyl, 8-azabicyclo[3.2.1]octyl, 1-azabicyclo[2.2.2]octyl ]octyl or octahydrocyclopenta[*c*]pyrrolyl, the bicyclo[3.2.1]octyl, bicyclo[2.2.2]octyl, octahydropentalenyl, piperidinyl, 8-azabicyclo[3.2.1]octyl, 1-azabicyclo[2.2.2]octyl or octahydrocyclopenta[*c*]pyrrolyl being optionally substituted with one, two or three substituents independently selected from oxo, hydroxyl, $C_{1-4}$ alkyl, $C_{1-4}$ alkyloxy, $C_{1-4}$ alkyl-carbonyl, formyl, $C_{1-4}$ alkylenedioxy and phenyl-$C_{1-4}$ alkyl;
**$R^4$** is 1,3-thiazol-2-yl, 1-methyl-1*H*-imidazol-2-yl or 1,3-oxazol-2-yl.

**4.** A compound according to Claim 3, wherein
**$R^3$** is 8-methylbicyclo[3.2.1]oct-3-yl, 8,8-ethylenedioxybicyclo[3.2.1]oct-3-yl, 8-oxobicyclo[3.2.1]oct-3-yl, 8-hydroxybicyclo[3.2.1]oct-3-yl, 8-hydroxy-8-methylbicyclo[3.2.1]oct-3-yl, 8-butyl-8-hydroxybicyclo[3.2.1]oct-3-yl, 8-hydroxy-8-isopropylbicyclo[3.2.1]oct-3-yl, 8-methoxybicyclo[3.2.1]oct-3-yl, bicyclo[2.2.2]oct-2-yl, 5-oxooctahydro-2-pentalenyl, 1-methyl-3-piperidinyl, 1-benzyl-3-piperidinyl, 8-methyl-8-azabicyclo[3.2.1]oct-3-yl, 8-ethyl-8-azabicyclo[3.2.1]oct-3-yl, 8-isopropyl-8-azabicyclo[3.2.1]oct-3-yl, 8-acetyl-8-azabicyclo[3.2.1]oct-3-yl, 1-azabicyclo[2.2.2]oct-3-yl, 2-methyloctahydrocyclopenta[*c*]pyrrol-5-yl, 2-acetyloctahydrocyclopenta[*c*]pyrrol-5-yl, 2-formyloctahydrocyclopenta[*c*]pyrrol-5-yl or 8-formyl-8-azabicyclo[3.2.1]oct-3-yl.

**5.** A compound according to claim 1 selected from
dimethyl 4-(2,6-dichlorophenyl)-2-[2-[4-(8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-oxobicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8,8-ethylenedioxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-hydroxy-8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-butyl-8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-hydroxy-8-isopropylbicyclo[3.2.1]oct-3-y1)-1-piperazinyl]-2-oxoethyl]-6-[2-(1,3-thiazol-2-yl)ethyl]-1,4-dihydro-3,5-pyridinedicarboxylate;
dimethyl4-(2,6-dichlorophenyl)-2-[2-[4-(8-methoxylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 2-[2-(4-bicyclo[2.2.2]oct-2-yl-1-piperazinyl)-2-oxoethyl]-4-(2,6-dichlorophenyl)-6-[2-(1,3-thiazol-2-yl)ethyl]-1,4-dihydro-3,5-pyridinedicarboxylate;
dimethyl 4-(2,6-dichlorophenyl)-2-[2-[4-(5-oxooctahydro-2-pentalenyl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(1-methyl-3-piperidinyl)-1-piperazinyl]-2-oxoethyl]-6-[2-(1,3-thiazol-2-yl)ethyl]-1,4-dihydro-3,5-pyridinedicarboxylate;
dimethyl 2-[2-[4-(1-benzyl-3-piperidinyl)-1-piperazinyl]-2-oxoethyl]-4-(2,6-dichlorophenyl)-6-[2-(1,3-thiazol-2-yl)ethyl]-1,4-dihydro-3,5-pyridmedicarboxylate;
dimethyl 4-(2,6-dichlorophenyl)-2-[2-[4-(8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
3-ethyl-5-methyl 4-(2,6-dichlorophenyl)-2-[2-[4-(8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-methyl-8-azabicyclo[3.2.1]oct-3-yl)-1-piperazinyl]-2-oxoethyl]-6-[2-(1-methyl-1*H*-imidazol-2-yl)ethyl]-1,4-dihydro-3,5-pyridinedicarboxylate;
dimethyl 4-(2,6-dichlorophenyl)-2-[2-[4-(8-methyl-8-azabicyclo[3.2.1]oct-3-yl)-1-piperazinyl]-2-oxoethyl]-6-[2-(1,3-oxazol-2-yl)ethyl]-1,4-dihydro-3,5-pyridinedicarboxylate;
dimethyl 4-(2,6-dichlorophenyl)-2-[2-[4-(8-ethyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-isoprppyl-8-azabicyc1o[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-acetyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-formyl-8-azabicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 2-(2-[4-[(1-azabicyclo[2.2.2]oct-3-yl)-1-piperazinyl]-2-oxoethyl)-4-(2,6-dichlorophenyl)-6-[2-(1,3-thiazol-2-yl)ethyl]-1,4-dihydro-3,5-pyridinedicarboxylate;
dimethyl 2-[2-[4-(2-methyloctahydrocyclopenta[*c*]pyrrol-5-yl)-1-piperazinyl]-2-oxoethyl]-4-(2,6-dichlorophenyl)-6-[2-(1,3-thiazol-2-yl)ethyl]-1,4-dihydro-3,5-pyridinedicarboxylate;
dimethyl 2-[2-[4-(2-acetyloctahydrocyclopenta[*c*]pyrrol-5-yl)-1-piperazinyl]-2-oxoethyl]-4-(2,6-dichlorophenyl)-6-[2-(1,3-thiazol-2-yl)ethyl]-1,4-dihydro-3,5-pyridinedicarboxylate; and
dimethyl 2-[2-[4-(2-formyloctahydrocyclopenta[*c*]pyrrol-5-yl)-1-piperazinyl]-2-oxoethyl]-4-(2,6-dichlorophenyl)-6-[2-(1,3-thiazol-2-yl)ethyl]-1,4-dihydro-3,5-pyridinedicarboxylate.

**6.** A compound according to claim 1 selected from
dimethyl 4-(2,6-dichlorophenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
dimethyl 4-(2,6-dichlorophenyl)-2-[2-[4-(8-methoxybicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate;
(-)-dimethyl 4-(2,6-dichlorophenyl)-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridine-3,5-dicarboxylate;
dimethyl 4-(2,6-dichlorophenyl)-2-(2-([4-(8-ethyl-8-azabicyclo[3.2.1]oct-3-yl)-1-piperazinyl]-2-oxoethy)-6-[2-(1,3-thiazol-2-yl)ethyl]-1,4-dihydro-3,5-pyridinedicarboxylate;
dimethyl 4-(2,6-dichlorophenyl)-2-(2-([4-(8-isopropyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinedicarboxylate;
dimethyl 2-[2-[4-(8-acetyl-8-azabicyclo[3.2.1]oct-3-yl-1-piperazinyl]2-oxoethyl]-4-(2,6-dichlorophenyl)-6-[2-(1,3-thiazol-2-yl)ethyl]-1,4-dihydro-3,5-pyridinedicarboxylate;
dimethyl 4-(2,6-dichlorophenyl)-2-(2-([4-(8-formyl-8-azabicyclo[3.2.1]oct-3-yl)-1-piperazinyl]-2-oxoethyl)-6-[2-(1,3-thiazol-2-yl)ethyl]-1,4-dihydro-3,5-pyridinedicarboxylate;
(-)-dimethyl 2-[2-[4-[(3S)-1-azabicyclo[2,2,2]oct-3-yl]-1-piperazinyl]-2-oxoethyl]-4-(2,6-dichlorophenyl)-6-[2-(1,3-thiazol-2-yl)ethyl]-1,4-dihydro-3,5-pyridinedicarboxylate;
dimethyl 4-(2,6-dichlorophenyl)-2-(2-([4-(2-acetyloctahydrocyclopenta[c]pyrrol-5-yl)-1-piperazinyl]-2-oxoethyl)-6-[2-(1,3-thiazol-2-yl)ethyl]-1,4-dihydro-3,5-pyridinedicarboxylate; and
(-)-dimethyl 4-(2,6-dichlorophenyl)-2-[2-[4-(*exo*-8-hydroxy-8-methylbicyclo[3.2.1]oct-3-yl)-1-piperazinyl]-2-oxoethyl]-6-[2-(1,3-thiazol-2-yl)ethyl]-1,4-dihydro-3,5-pyridinedicarboxylate.

**7.** A pharmaceutical composition for the treatment of disease conditions mediated by bradykinin, in a mammalian subject, which comprises a therapeutically effective amount of a compound of Claim 1 and a pharmaceutically acceptable carrier.

**8.** A pharmaceutical composition for the treatment of inflammation, rheumatoid arthritis, cystitis, post-traumatic and post ischemic cerebral edema, liver cirrhosis, Alzheimer's disease, cardiovascular disease, pain, common cold, allergies, asthma, burns, virus infection, head injury, multiple trauma, rhinitis, hepatorenal failure, diabetes, metastasis, pancreatitis, neovascularization, corneal haze, glaucoma, ocular pain or ocular hypertension, which comprises a therapeutically effective amount of a compound of Claim 1 and a pharmaceutically acceptable carrier.

**9.** A pharmaceutical composition for the treatment of Amyotrophic lateral sclerosis, Huntington's disease, Parkinson's disease, multiple sclerosis, stroke, head trauma, post-surgical brain edema, brain edema (general), cytotoxic brain edema, brain edema associated with metabolic diseases, rheumatoid arthritis, osteoarthritis, migraine, neuropathic pain, pruritis, brain tumor, pseudotumor cerebri, glaucoma, hydrocephalus, spinal cord trauma, spinal cord edema, neurodegenerative diseases, respiratory diseases, diuresis, natriuresis calciuresis, chronic obstructive pulmonary disease, post-traumatic brain injury, itching or sepsis, which comprises a therapeutically effective amount of a compound of Claim 1 or its pharmaceutically acceptable carrier.

**10.** Use of a therapeutically effective amount of a compound according to claim 1 for the manufacture of a medicament for the treatment of disease conditions mediated by bradykinin, in a mammalian subject.

**11.** Use of a therapeutically effective amount of a compound according to claim 1 for the manufacture of a medicament for the treatment of inflammation, rheumatoid arthritis, cystitis, post-traumatic and post ischemic cerebral edema, liver cirrhosis, Alzheimer's disease, cardiovascular disease, pain, common cold, allergies, asthma, pancreatitis, burns, virus infection, head injury, multiple trauma, rhinitis, hepatorenal failure, diabetes, metastasis, pancreatitis, neovascularization, corneal haze, glaucoma, ocular pain or ocular hypertension, in a mammalian subject.

**12.** A pharmaceutical formulation comprising a compound of claim 1, a pharmaceutically acceptable carrier and, optionally, one or more other pharmacologically active ingredients.

## Patentansprüche

**1.** Verbindung der Formel (I):

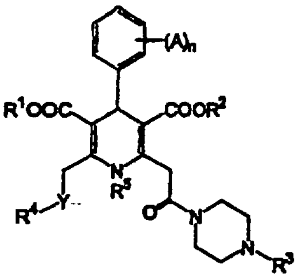

(I)

oder die pharmazeutisch verträglichen Salze davon, worin
A unabhängig Halogen darstellt;
Y $-(CH_2)_m-$, -C(O) - oder -S(O)- darstellt;
$R^1$ und $R^2$ unabhängig $C_{1-4}$-Alkyl darstellen;
$R^3$ $C_{7-9}$-Bicycloalkyl , $C_{5-7}$-Azacycloalkyl oder $C_{7-9}$-Azabicycloalkyl darstellt, wobei das $C_{7-9}$-Bicycloalkyl, $C_{5-7}$-Azacycloalkyl oder $C_{7-9}$-Azabicycloalkyl gegebenenfalls mit einem, zwei oder drei Substituenten, unabhängig ausgewählt aus Oxo, Hydroxyl, $C_{1-4}$-Alkyl, $C_{1-4}$-Alkyloxy, $C_{1-4}$-Alkylcarbonyl, Formyl, $C_{1-4}$-Alkylendioxy und Phenyl-$C_{1-4}$-alkyl, substituiert sind;
$R^4$ Thiazolyl, Imidazolyl oder Oxazolyl darstellt, wobei das Thiazolyl, Imidazolyl oder Oxazolyl gegebenenfalls mit einem oder zwei Substituenten, unabhängig ausgewählt aus $C_{1-4}$-Alkyl und Halogen, substituiert sind;
$R^5$ Wasserstoff oder $C_{1-4}$-Alkyl darstellt;
m 0, 1 oder 2 ist, und
n 0, 1, 2, 3, 4 oder 5 ist.

**2.** Verbindung nach Anspruch 1, worin
$(A)_n$ 2,6-Dichlor darstellt;
Y $-CH_2-$ darstellt;
$R^4$ 1,3-Thiazolyl-2-yl, 1*H*-Imidazol-2-yl, 1-Methyl-1*H*-imidazol-2-yl, 1-Ethyl-1*H*-imidazol-2-yl oder 1,3-Oxazol-2-yl darstellt und
$R^5$ Wasserstoff darstellt.

**3.** Verbindung nach Anspruch 2, worin $R^1$ und $R^2$ unabhängig Methyl oder Ethyl darstellen;
$R^3$ Bicyclo[3.2.1]octyl, Bicyclo[2.2.2]octyl, Octahydropentalenyl, Piperidinyl, 8-Azabicyclo[3.2.1]octyl, 1-Azabicyclo[2.2.2]octyl oder Octahydrocyclopenta[c]pyrrolyl darstellt, wobei die Bicyclo[3.2.1]octyl, Bicyclo[2.2.2]octyl, Octahydropentalenyl, Piperidinyl, 8-Azabicyclo[3.2.1]octyl, 1-Azabicyclo[2.2.2]octyl oder Octahydrocyclopenta[c]pyrrolyl gegebenenfalls mit einem, zwei oder drei Substituenten, unabhängig ausgewählt aus Oxo, Hydroxyl, $C_{1-4}$-Alkyl, $C_{1-4}$-Alkyloxy, $C_{1-4}$-Alkylcarbonyl, Formyl, $C_{1-4}$-Alkylendioxy und Phenyl-$C_{1-4}$-alkyl, substituiert sind;
$R^4$ 1,3-Thiazol-2-yl, 1-Methyl-1H-imidazol-2-yl oder 1,3-Oxazol-2-yl darstellt.

**4.** Verbindung nach Anspruch 3 worin
$R^3$ 8-Methylbicyclo[3.2.1]oct-3-yl, 8,8-Ethylendioxybicyclo[3.2.1]oct-3-yl, 8-Oxobicyclo[3.2.1]oct-3-yl, 8-Hydroxybicyclo[3.2.1]oct-3-yl, 8-Hydroxy-8-methylbicyclo[3.2.1]oct-3-yl, 8-Butyl-8-hydroxybicyclo[3.2.1]oct-3-yl, 8-Hydroxy-8-isopropylbicyclo[3.2.1]oct-3-yl, 8-Methoxybicyclo[3.2.1]oct-3-yl, Bicyclo[2.2.2]oct-2-yl, 5-Oxooctahydro-2-pentalenyl, 1-Methyl-3-piperidinyl, 1-Benzyl-3-piperidinyl, 8-Methyl-8-azabicyclo[3.2.1]oct-3-yl, 8-Ethyl-8-azabicyclo[3.2.1]oct-3-yl, 8-Isopropyl-8-azabicyclo[3.2.1]oct-3-yl, 8-Acetyl-8-azabicyclo(3.2.1]oct-3-yl, 1-Azabicyclo[2.2.2]oct-3-yl, 2-Methyloctahydrocyclopenta[c]pyrrol-5-yl, 2-Acetyloctahydrocyclopenta[c]pyrrol-5-yl, 2-Formyloctahydrocyclopenta[c]pyrrol-5-yl oder 8-Formyl-8-azabicyclo[3.2.1]oct-3-yl darstellt.

**5.** Verbindung nach Anspruch 1, ausgewählt aus
4-(2,6-Dichlorphenyl)-2-[2-[4-(8-methylbicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-oxobicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8,8-ethylendioxybicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-hydroxy-8-methylbicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-butyl-8-hydroxybicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-hydroxy-8-isopropylbicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-methoxylbicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

2-[2-(4-Bicyclo[2.2.2)oct-2-yl-1-piperazinyl)-2-oxoethyl]-4-(2,6-dichlorphenyl)-6-[2-(1,3-thiazol-2-yl)ethyl]-1,4-dihydro-3,5-pyridindicarbonsäuredimethylester;

4-(2,6-Dichlorphenyl)-2-[2-[4-(5-oxooctahydro-2-pentalenyl)-1-piperazinyl]-2-oxoethyl]-6-[2-(1,3-thiazol-2-yl)ethyl)-1,4-dihydro-3,5-pyridindicarbonsäuredimethylester;

4-(2,6-Dichlorphenyl)-2-[2-(4-(1-methyl-3-piperidinyl)-1-piperazinyl]-2-oxoethyl]-6-[2-(1,3-thiazol-2-yl)ethyl]-1,4-dihydro-3,5-pyridindicarbonsäuredimethylester;

2-[2-[4-(1-Benzyl-3-piperidinyl)-1-piperazinyl]-2-oxoethyl]-4-(2,6-dichlorphenyl)-6-[2-(1,3-thiazol-2-yl)ethyl]-1,4-dihydro-3,5-pyridindicarbonsäuredimethylester;

4-(2,6-Dichlorphenyl)-2-[2-[4-(8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-methyl-8-azabicyclo[3.2.1]oct-3-yl)-1-piperazinyl]-2-oxoethyl]-6-[2-(1,3-thiazol-2-yl)ethyl]-1,4-dihydro-3,5-pyridindicarbonsäure-3-ethyl-5-methylester;

4-(2,6-Dichlorphenyl)-2-[2-[4-(8-methyl-8-azabicyclo[3.2.1]oct-3-yl)-1-piperazinyl]-2-oxoethyl]-6-[2-(1-methyl-1*H*-imidazol-2-yl)ethyl]-1,4-dihydro-3,5-pyridindicarbonsäuredimethylester;

4-(2,6-Dichlorphenyl)-2-[2-[4-(8-methyl-8-azabicyclo[3.2.1]oct-3-yl)-1-piperazinyl]-2-oxoethyl]-6-[2-(1,3-oxazol-2-yl)ethyl]-1,4-dihydro-3,5-pyridindicarbonsäuredimethylester;

4-(2,6-Dichlorphenyl)-2-[2-[4-(8-ethyl-8-azabicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-isopropyl-8-azabicyclo[3.2.1)oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-acetyl-8-azabicyclo[3.2.l]oct-3-yl)-1-piperazinyl]-2-oxoethyl]-6-[2-(1,3-thiazol-2-yl)ethyl]-2,4-dihydro-3,5-pyridindicarbonsäuredimethylester;

4-(2,6-Dichlorphenyl)-2-[2-[4-(8-formyl-8-azabicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

2-(2-[4-[(1-Azabicyclo[2.2.2]oct-3-yl]-1-piperazinyl]-2-oxoethyl)-4-(2,6-dichlorphenyl)-6-[2-(1,3-thiazol-2-yl)ethyl]-1,4-dihydro-3,5-pyridindicarbonsäuredimethylester;

2-[2-[4-(2-Methyloctahydrocyclopenta[c]pyrrol-5-yl)-1-piperazinyl]-2-oxoethyl]-4-(2,6-dich1orphenyl)-6-[2-(1,3-thiazol-2-yl)ethyl]-1,4-dihydro-3,5-pyridindicarbonsäuredimethylester;

2-[2-[4-(2-Acetyloctahydrocyclopenta[c]pyrrol-5-yl)-1-piperazinyl]-2-oxoethyl]-4-(2,6-dichlorphenyl)-6-[2-(1,3-thiazol-2-yl)ethyl]-1,4-dihydro-3,5-pyridindicarbonsäuredimethylester und

2-[2-[4-(2-Formyloctahydrocyclopenta[c]pyrrol-5-yl)-1-piperazinyl]-2-oxoethyl]-4-(2,6-dichlorphenyl)-6-[2-(1,3-thiazol-2-yl)ethyl]-1,4-dihydro-3,5-pyridindicarbonsäuredimethylester.

**6.** Verbindung nach Anspruch 1, ausgewählt aus

4-(2,6-Dichlorphenyl)-2-[2-[4-(8-hydroxybicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

4-(2,6-Dichlorphenyl)-2-[2-[4-(8-methoxybicyclo[3.2.1]oct-3-yl)-1-piperazinyl]-2-oxoethyl]-6-[2-(1,3-thiazol-2-yl)ethyl]-1,4-dihydro-3,5-pyridindicarbonsäuredimethylester;

(-)-4-(2,6-Dichlorphenyl)-2-[4-(*exo*-8-methyl-8-azabicyclo[3.2.1]oct-3-yl)-1-piperazinyl]-2-oxoethyl-6-[2-(1,3-thiazol-2-yl)ethyl]-1,4-dihydropyridin-3,5-dicarbonsäuredimethylester;

4-(2,6-Dichlorphenyl)-2-[2-([4-(8-ethyl-8-azabicyclo[3.2.1)oct-3-yl)-1-piperazinyl]-2-oxoethyl)-6-[2-(1,3-thiazol-2-yl)ethyl]-1,4-dihydro-3,5-pyridindicarbonsäuredimethylester;

4-(2,6-Dichlorphenyl)-2-[2-([4-(8-isopropyl-8-azabicyclo[3.2.1]oct-3-yl)-1-piperazinyl]-2-oxoethyl)-6-[2-(1,3-thiazol-2-yl)ethyl]-1,4-dihydro-3,5-pyridindicarbonsäuredimethylester;

2-[2-[4-(8-Acetyl-8-azabicyclo[3.2.1]oct-3-yl-1-piperazinyl)-2-oxoethyl]-4-(2,6-dichlorphenyl)-6-[2-(1,3-thiazol-2-yl)ethyl)-1,4-dihydro-3,5-pyrididicarbonsäuredimethylester;
4-(2,6-Dichlorphenyl)-2-(2-([4-(8-formyl-8-azabicyclo[3.2.1]oct-3-yl)-1-piperazinyl]-2-oxoethyl)-6-[2-(1,3-thiazol-2-yl)ethyl)-1,4-dihydro-3,5-pyridindicarbonsäuredimethylester;
(-)-2-[2-[4-[(3S)-1-Azabicyclo[2.2.2]oct-3-yl]-1-piperazinyl]-2-oxoethyl]-4-(2,6-dichlorphenyl)-6-[2-(1,3-thiazol-2-yl)ethyl)-1,4-dihydro-3,5-pyridindicarbonsäuredimethylester;
4-(2,6-Dichlorphenyl)-2-[2-([4-(2-acetyloctahydrocyclopenta[c]pyrrol-5-yl)-1-piperazinyl]-2-oxoethyl)-6-[2-(1,3-thiazol-2-yl)ethyl]-1,4-dihydro-3,5-pyridindicarbonsäuredimethylester und
(-)-4-(2,6-Dichlorphenyl)-2-[2-[4-(*exo*-8-hydroxy-8-methylbicyclo[3.2.1]oct-3-yl)-1-piperazinyl)-2-oxoethyl]-6-[2-(1,3-thiazol-2-yl)ethyl]-1,4-dihydro-3,5-pyridindicarbonsäuredimethylester.

**7.** Pharmazeutische Zusammensetzung für die Behandlung von Bradykinin vermittelten Krankheitszuständen bei einem Säuger, welche eine therapeutisch wirksame Menge einer Verbindung nach Anspruch 1 und eine pharmazeutisch verträglichen Träger umfasst.

**8.** Pharmazeutische Zusammensetzung für die Behandlung von Entzündung, rheumatischer Arthritis, Cystitis, posttraumatischem und postischämischem cerebralem Ödem, Leberzirrhose, Alzheimer Krankheit, cardiovaskuläre Erkrankung, Schmerz, üblicher Erkältung, Allergien, Asthma, Verbrennungen, Virusinfektion, Kopfschmerz, multiples Trauma, Schnupfen, Leber-Nieren-Insuffizienz, Diabetes, Metastasis, Pankreatitis, Neovascularisierung, Linsentrübung, Glaukom, Okularschmerz oder okulare Hypertension, welche eine therapeutisch wirksame Menge einer Verbindung nach Anspruch 1 und einen pharmazeutisch verträglichen Träger umfasst.

**9.** Pharmazeutische Zusammensetzung für die Behandlung von amyotropher lateraler Sklerose, Huntington-Krankheit, Parkinson-Krankheit, multipler Sklerose, Schlaganfall, Kopftrauma, postchirurgischem Hirnödem, Hirnödem (allgemein), cytotoxischem Hirnödem, Hirnödem, bedingt durch metabolische Erkrankungen, rheumatischer Arthritis, Osteoarthritis, Migräne, neuropatischem Schmerz, Pruritis, Hirntumor, Pseudotumor cerebri, Glaucom, Hydrocephalus, Wirbelsäulentrauma, Wirbelsäulenödem, neurodegenerativer Erkrankungen, respiratorischer Erkrankungen, Diurese, Natriurese, Calciurese, chronischer obstruktiver pulmonaler Erkrankung, posttraumatischer Hirnschädigung, Juckreiz oder Sepsis, welche eine therapeutisch wirksame Menge einer Verbindung nach Anspruch 1 oder ihres pharmazeutisch verträglichen Trägers umfasst.

**10.** Verwendung einer therapeutisch wirksame Menge einer Verbindung nach Anspruch 1 zur Herstellung eines Arzneimittels zur Behandlung von Bradykinin vermittelten Krankheitszuständen bei einem Säuger.

**11.** Verwendung einer therapeutisch wirksamen Menge einer Verbindung nach Anspruch 1 zur Herstellung eines Arzneimittels zur Behandlung von Entzündung, rheumatischer Arthritis, Cystitis, posttraumatischem und postischämischem cerebralem Ödem, Leberzirrhose, Alzheimer-Krankheit, cardiovaskulärer Erkrankung, Schmerz, üblicher Erkältung, Allergien, Asthma, Pankreatitis, Verbrennungen, Virusinfektion, Kopfverletzung, multiplem Trauma, Schnupfen, Leber-Nieren-Insuffizienz, Diabetes, Metastasis, Pankreatitis, Neovascularisierung, Linsentrübung, Glaukom, Okularschmerz und okularer Hypertension bei einem Säuger.

**12.** Pharmazeutische Formulierung, umfassend eine Verbindung nach Anspruch 1, einen pharmazeutisch verträglichen Träger und gegebenenfalls einen oder mehrere andere pharmakologische Wirkstoffe.

## Revendications

**1.** Un composé de formule (I):

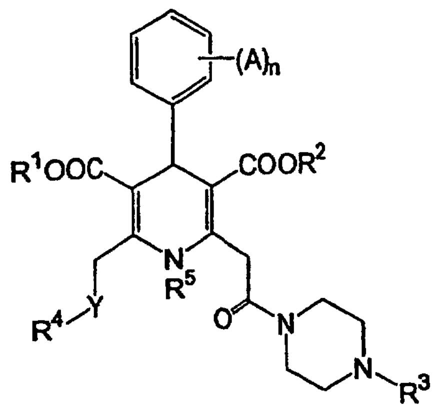

(I)

ou un de ses sels pharmaceutiquement acceptables, où

A est indépendamment un halo;

Y est $-(CH_2)_m$ , -C(O)- ou -S(O)-;

$R^1$ et $R^2$ sont indépendamment un alkyle en $C_{1-4}$;

$R^3$ est un bicycloalkyle en $C_{7-9}$, azacycloalkyle en $C_{5-7}$ ou azabicycloalkyle en $C_{7-9}$, le bicycloalkyle en $C_{7-9}$, l'azacycloalkyle en $C_{5-7}$ ou l'azabicycloalkyle en $C_{7-9}$ étant éventuellement substitué par un, deux ou trois substituants choisis indépendamment parmi oxo, hydroxyle, alkyle en $C_{1-4}$, alcoxy en $C_{1-4}$, alkyle en $C_{1-4}$-carbonyle, formyle, alkylènedioxy en $C_{1-4}$ et phényl-alkyle en $C_{1-4}$;

$R^4$ est thiazolyle, imidazolyle ou oxazolyle, le thiazolyle, l'imidazolyle ou l'oxazolyle étant éventuellement substitués par un ou deux substituants choisis indépendamment parmi alkyle en $C_{1-4}$ et halo;

$R^5$ est un hydrogène ou un alkyle en $C_{1-4}$;

M vaut 0, 1 ou 2; et

N vaut 0, 1, 2, 3, 4 ou 5.

**2.** Un composé selon la revendication 1, dans lequel:

$(A)_n$ est 2,6-dichloro;

Y est $-CH_2-$;

$R^4$ est 1,3-thiazol-2-yle, 1*H*-imidazol-2-yle, 1-méthyl-1*H*-imidazol-2-yle, 1-éthyl-1*H*-imidazol-2-yle ou 1,3-oxazol-2-yle; et

$R^5$ est l'hydrogène.

**3.** Un composé selon la revendication 2, où

$R^1$ et $R^2$ sont indépendamment méthyle ou éthyle;

$R^3$ est bicyclo[3.2.1]octyle, bicyclo[2.2.2]octyle, octahydropentalényle, pipéridinyle, 8-azabicyclo[3.2.1]octyle, 1-azabicyclo[2.2.2]octyle ou octahydrocyclopenta[*c*]pyrrolyle, les bicyclo[3.2.1]octyle, bicyclo[2.2.2]octyle, octahydropentalényle, pipéridinyle, 8-azabicyclo[3.2.1]octyle, 1-azabicyclo[2.2.2]octyle ou octahydrocyclopenta[*c*]pyrrolyle étant éventuellement substitués par un, deux ou trois substituants choisis indépendamment parmi les oxo, hydroxyle, alkyle en $C_{1-4}$, alkyloxy en $C_{1-4}$, alkyle en $C_{1-4}$-carbonyle, formyle, alkylènedioxy en $C_{1-4}$ et phényl-alkyle en $C_{1-4}$;

$R^4$ est 1,3-thiazol-2-yle, 1-méthyl-1*H*-imidazol-2-yle ou 1,3-oxazol-2-yle.

**4.** Un composé selon la revendication 3 où:

$R^3$ est 8-méthylbicyclo[3.2.1]oct-3-yle, 8,8-éthylènedioxybicyclo[3.2.1]oct-3-yle, 8-oxobicyclo[3.2.1]oct-3-yle, 8-hydroxybicyclo[3.2.1]oct-3-yle, 8-hydro-xy-8-méthylbicyclo[3.2.1]oct-3-yle, 8-butyl-8-hydroxybicyclo[3.2.1]oct-3-yle, 8-hydroxy-8-isopropylbicyclo[3.2.1]oct-3-yle, 8-méthoxybicyclo[3.2.1]oct-3-yle, bicyclo[2.2.2]oct-2-yle, 5-oxooctahydro-2-pentalényle, 1-méthyl-3-pipéridinyle, 1-benzyl-3-pipéridinyle, 8-méthyl-8-azabicyclo[3.2.1]oct-3-yle, 8-éthyl-8-azabicyclo[3.2.1]oct-3-yle, 8-isopropyl-8-azabicyclo[3.2.1]oct-3-yle, 8-acétyl-8-azabicyclo[3.2.1]oct-3-yle, 1-azabicyclo[2.2.2]oct-3-yle, 2-méthyloctahydrocyclopenta[*c*]pyrrol-5-yle, 2-acétyloctahydrocyclopenta[*c*]pyrrol-S-yle, 2-formyloctahydrocyclopenta[*c*]pyrrol-5-yle ou 8-formyl-8-azabicyclo[3.2.1]oct-3-yle.

5. Un composé selon la revendication 1 choisi parmi les:

diméthyl 4-(2,6-dichorophényl)-2-[2-[4-(8-méthylbicyclo[3.2.1]oct-3-yl)-1-pipérazinyl]-2-oxoéthyl]6-[2-(1,3-thiazol-2-yl)éthyl]-1,4-dihydro-3,5-piridinedicarboxylate;
diméthyl 4-2,6-dichlorophényl-2-[2-[4-(8-oxobicyclo[3.2.1]oct-3-yl)-1-pipérazinyl-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8,8-éthylènedioxybicyclo[3.2.1]oct3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-hydroxybicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-hydroxy-8-méthylbicyclo[3.2.1] oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-butyl-8-hydroxybicyclo[3.2.1 ]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-hydroxy-8-isopropylbicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-méthoxybicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 2-[2-(4-bicyclo[2.2.2]oct-2-yl)-1-pipérazinyl]-2-oxoéthyl]-4-(2,6-dichlorophényl)-6-[2-(1,3-thiazol-2y1)éthyl]-1,4-dihydro-3,5-pyridinedicarboxylate;
diméthyl 4-(2,6-dichlorophényl)-2-[2-[4-(5-oxooctahydro-2-pentalényl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(1-méthyl-3-pipéridinyl)-1-pipérazinyl]- 2-oxoéthyl]-6-[2-(1,3-thiazol-2-yl)éthyl]-1,4-dihydro-3,5-pyridinedicarboxylate;
diméthyl 2-[2-[4-(1-benzyl-3-pipéridinyl)-1-pipérazinyl]-2-oxoéthyl]-4-(2,6-dichlorophényl)-6-[2-(1,3-thiazol-2-yl)éthyl]-1,4-dihydro-3,5-pyridinedicarboxylate;
diméthyl 4-(2,6-dichlorophényl)-2-[2-[4-(8-méthyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
3-éthyl-5-méthyl 4-(2,6-dichlorophényl)-(2-[4-(8-méthyl-8-azabicyclo[3.2.1] oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-méthyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-méthyl-8-azabicyclo[3.2.1]oct-3-yl)-1-pipérazinyl]-2-oxoéthyl]-6-[2-(1,3-oxazol-2-yl)éthyl]-1,4-dihydro-3,5-pyridinedicarboxylate;
diméthyl 4-(2,6-dichlorophényl)-2-[2-[4-(8-éthyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-isopropyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-acétyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-formyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 2-(2-[4-[1-azabicyclo[2.2.2]oct-3-yl)-1-pipérazinyl]-2-oxoéthyl]-4-(2,6-dichlorophényl)-6-[2-(1,3-thiazol-2-yl)éthyl]-1,4-dihydro-3,5-pyridinedicarboxylate;
diméthyl 2-(2-[4-[2-méthyloctahydrocyclopenta[c]pyrrol-5-yl)-1-pipérazinyl]-2-oxoéthyl]-4-(2,6-dichlorophényl)-6-[2-(1,3-thiazol-2-yl)éthyl]-1,4-dihydro-3,5-pyridinedicarboxylate;
diméthyl 2-(2-[4-[2-acétyloctahydrocyclopenta[c]pyrrol-5-yl)-1-pipérazinyl]-2-oxoéthyl]-4-(2,6-dichlorophényl)-6-[2-(1,3-thiazol-2-yl)éthyl]-1,4-dihydro-3,5-pyridinedicarboxylate; et
diméthyl 2-(2-[4-[2-formyloctahydrocyclopenta[*c*]pyrrol-5-yl)-1-pipérazinyl)-2-oxoéthyl]-4-(2,6-dichlorophényl)-6-[2-(1,3-thiazol-2-yl)éthyl]-1,4-dihydro-3,5-pyridinedicarboxylate.

6. Un composé selon la revendication 1 choisi parmi les:

diméthyl 4-(2,6-dichlorophényl)-2-[2-[4-(8-hydroxybicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-[4-(8-méthoxybicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
(-)-diméthyl 4-(2,6-dichlorophényl)-2-[2-[4-(exo-8-méthyl--8-azabicyclo[3.2.1] oct-3-yl)-1-pipérazinyl]-2-oxoéthyl]-6-[2-(1,3-thiazol-2-yl)éthyl]-1,4-dihydro-3,5-pyridinedicarboxylate;
diméthyl 4-(2,6-dichlorophényl)-2-[2-([4-(8-éthyl-8-azabicyclo[3.2.1]oct-3-yl)-1-pipérazinyl)-2-oxoéthyl]-6-[2-(1,3-thiazol-2-yl)éthyl)-1,4-dihydro-3,5-pyridinedicarboxylate;
diméthyl 4-(2,6-dichlorophényl)-2-[2-([4-(8-isopropyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
diméthyl 2-[2-[4-(8-acétyl-8-azabicyclo[3.2.1]oct-3-yl)-1-pipérazinyl]-2-oxoéthyl]-4-(2,6-dichlorphényl)-6-[2-(1,3-thiazol-2-yl)éthyl]-1,4-dihydro-3,5-pyridinedicarboxylate;
diméthyl 4-(2,6-dichlorophényl)-2-[2-([4-(8-formyl-8-azabicyclo[3.2.1]oct-3-yl)-1-pipérazinyl]-2-oxoéthyl]-6-[2-(1,3-thiazol-2-yl)éthyl]-1,4-dihydro-3,5-pyridinedicarboxylate;
(-)-diméthyl 2-[2-[4-[3S)-1-azabicyclo[2.2.2]oct-3-yl)-1-pipérazinyl]-2-oxoéthyl]-4-(2,6-dichlorophényl)-6-[2-(1,3-thiazol-2-yl)éthyl]-1,4-dihydro-3,5-pyridinedicarboxylate;
diméthyl 4-(2,6-dichlorophényl)-2-[2-([4-(2-acétyloctahydrocyclopenta[*c*]pyrrol-5-yl)-1-pipérazinyl]-2-oxoéthyl]-6-[2-(1,3-thia-zol-2-yl)éthyl]-1,4-dihydro-3,5-pyridinedicarboxylate; et
(-)-diméthyl 4-(2,6-dichlorophényl)-2-[2-[4-(*exo*-8-hydroxy-8-azabicyclo [3.2.1]oct-3-yl)-1-pipérazinyl]-2-oxoéthyl]-6-[2-(1,3-thiazol-2-yl)éthyl]-1,4-dihydro-3,5-pyridinedicarboxylate.

**7.** Une composition pharmaceutique pour le traitement d'états pathologiques médiés par la bradykinine, chez un mammifère, qui comprend une quantité thérapeutiquement efficace d'un composé selon la revendication 1 et un support pharmaceutiquement acceptable.

**8.** Une composition pharmaceutiquement pour le traitement de 1'inflammation, de l'arthrite rhumatoïde, de la cystite, de l'oedème cérébral post-traumatique et post-ischémique, de la cirrhose du foie, de la maladie d'Alzheimer, des maladies cardiovasculaires, de la douleur, des rhumes, des allergies, de l'asthme, des brûlures, de l'infection virale, des blessures à la tête, des traumatismes multiples, de la rhinite, de l'insuffisance hépatorénale, du diabète, des métastases, de la pancréatite, de la néovascularisation, du voile cornéen, du glaucome, de la douleur oculaire ou de l'hypertension oculaire qui comprend une quantité thérapeutiquement efficace d'un composé selon la revendication 1 et un support pharmaceutiquement acceptable.

**9.** Une composition pharmaceutique pour le traitement de la sclérose amyotrophique latérale, de la maladie de Huntington, de la maladie de Parkinson, de la sclérose multiple, des attaques, des traumatismes crâniens, de l'oedème crânien post-chirurgical, de l'oedème cérébral (général), de l'oedème cérébral cytotoxique, de l'oedème cérébral associé aux troubles métaboliques, de l'arthrite rhumatoïde, de l'ostéoarthrite, de la migraine, de la douleur néopathique, du prurite, des tumeurs du cerveau, des pseudotumeurs cerebri, du glaucome, de l'hydrocéphalus, des traumatismes de la moelle épinière, de l'oedème de la moelle épinière, des troubles neurodégénératifs, des troubles respiratoires, la diurée, de la natriurée, de la calciurée, des maladies pulmonaires obstructrices chroniques, des lésions cérébrales post-traumatiques, des prurites, de la septicémie, qui comprend une quantité thérapeutiquement d'un composé selon la revendication 1 ou un de ses supports pharmaceutiquement acceptables.

**10.** Utilisation d'une quantité thérapeutiquement efficace d'un composé selon la revendication 1, pour la fabrication d'un médicament pour le traitement d'états maladifs médiés par la bradykinine chez un mammifère.

**11.** Utilisation d'une quantité thérapeutiquement efficace d'un composé selon la revendication 1, pour la fabrication d'un médicament pour le traitement de l'inflammation, de l'arthrite rhumatoïde, de la cystite, de l'oedème cérébral post-traumatique et post-ischémique, de la cirrhose du foie, de la maladie d'Alzheimer, des maladies cardiovasculaires, de la douleur, des rhumes, des allergies, de l'asthme, de la pancréatite, des brûlures, de l'infection virale, des blessures à la tête, des traumatismes multiples, de la rhinite, de l'insuffisance hépatorénale, du diabète, des métastases, de la pancréatite, de la néovascularisation, du voile cornéen, du glaucome, de la douleur oculaire ou de l'hypertension oculaire chez un mammifère.

**12.** Une formulation pharmaceutique comprenant un composé selon la revendication 1, un support pharmaceutiquement acceptable et le cas échéant, un ou plusieurs autres ingrédients pharmacologiquement actifs.